# EUROPEAN PATENT APPLICATION

(11) **EP 3 733 688 A1**
(43) Date of publication of application: **04.11.2020**
(21) Application number: 20176389.3
(22) Date of filing: 29.06.2017
(51) Int. Cl.: C07K 14/18

(54) **FISH VIRUS**

(30) Priority: 30.06.2016 GB 201611512
(62) Divisional of application: 17001115.9
(71) Applicant: Pharmaq AS, 7863 Overhalla (NO)
(72) Inventor: BRUDAL, Espen, 7863 Overhalla (NO); HAUGLAND, Oyvind, 7863 Overhalla (NO); JOHANSEN, Renate, 7863 Overhalla (NO); VESTVIK, Nils Frederik, 7863 Overhalla (NO)
(74) Representative: Mannion, Sally Kim

(57) **Abstract**

The present invention relates to a novel fish virus, nucleic acid sequences originating from the virus, and uses thereof.

## Description

### CROSS REFERENCE TO RELATED APPLICATIONS

This application claims priority from GB1611512.3 filed June 30, 2016, which is incorporated by reference in its entirety.

### FIELD OF THE INVENTION

The present invention relates to a newly discovered virus (Lumpfish flavivirus), which causes disease in fish, and the use of the isolated virus within veterinary immunology and diagnosis. More specifically, the invention provides nucleic acid sequences coding proteins and the use thereof in inter alia recombinant vaccines, DNA vaccines, live recombinant vaccines, and vaccines comprising attenuated or inactivated Lumpfish flavivirus. The protein coding sequences are also useful in respect of developing methods providing for immunological determination of virus infection. The invention thus also relates to antibodies, and the use thereof in immunoassays. The present invention also relates to primers useful in methods for detection of virus in a test sample. The present invention also provides diagnostic kits comprising recombinant proteins, antibodies and primers, respectively.

### BACKGROUND

The lumpfish (Cyclopterus lumpus L.) has become an important aquaculture species due to its efficacy as a cleaner fish for Atlantic salmon. This species has shown a high appetite for the ectoparasitic copepod, the sea louse *Lepeophtherius salmonis* Krøyer, representing a non-pharmaceutical and therefore an environmentally friendly delousing method when compared to chemical treatments. It is therefore important to ensure a stable supply of lumpfish for use as cleaner fish in the salmon industry.

Lumpfish, a common species along the Norwegian coast, has a broad geographical distribution, from Greenland in the north to the coast of Portugal in the east and Cape Cod in the south-west, indicating a broad temperature tolerance. This species in addition seems to be more robust and with a less complicated intensive production of larvae and juveniles compared to other cleaner fish species such as the ballan wrasse *Labrus bergylta,* goldsinny wrasse *Ctenolabrus rupestris* L. and corkwing wrasse *Symphodus melops* L.

Since the intensive production of this species is relatively new (around 2012), very little is known about the disease problems potentially faced by this species when being produced at high densities. At present, the main causes of death in cleaner fish are related to bacterial diseases. A number of bacteria with pathogenic potential have been isolated from farmed and wild caught cleaner fish including such species as *Vibrio anguillarum, Vibrio ordalii* and *Pseudomonas anguilliseptica,* and there are indications that primary pathogens of cleaner fish are also *Aeromonas salmonicida, Vibrio splendidus* and *Vibrio tapetis.* A few vaccines have been available for vaccinating lumpfish against bacterial diseases.

No viral diseases have been characterized in lumpfish until the description given in this patent application.

Elevated levels of mortalities were observed in a rearing facility for lumpfish in Norway. Histology samples were collected, and these showed mild to severe necrosis in the liver from all sampled lumpfish. The genome of a novel virus, most closely related to the virus family *Flaviviridae* was characterized. This novel virus is referred to herein as Lumpfish flavivirus.

It is therefore an object of the present invention to provide nucleic acid sequences originating from Lumpfish flavivirus, and various useful applications thereof.

### SUMMARY OF THE INVENTION

In a first aspect, the present invention provides an isolated virus comprising a ribonucleic acid genome having at least 70% sequence identity to SEQ ID NO. 20, and variants and fragments thereof. In an embodiment, the virus of the invention comprises a ribonucleic acid genome sequence having at least 80%, at least 85%, at least 90%, at least 91%, at least 92%, at least 93%, at least 94%, at least 95%, at least 96%, at least 97%, at least 98%, at least 99%, at least 99.5% or at least 99.9% sequence identity to SEQ ID NO. 20. In some embodiments, the virus of the invention belongs to the family flaviviridae. In some embodiments, the virus of the invention is isolated from lumpfish. In some embodiments, the virus of the invention is a flavivirus isolated from a lumpfish. In some embodiment, the virus of the invention is a lumpfish flavivirus (LFV).

In a second aspect, the present invention provides an isolated DNA nucleic acid sequence originating from the virus of the invention having a sequence selected from the group consisting of: SEQ ID NO. 1, SEQ ID NO. 2, SEQ ID NO. 4, SEQ ID NO. 6, and variants or fragments thereof having at least 70 % sequence identity to any of the sequences SEQ ID NO. 1, SEQ ID NO. 2, SEQ ID NO. 4 or SEQ ID NO. 6.

In some embodiments, the isolated DNA nucleic acid sequence originating from the virus of the invention may have at least 80%, at least 85%,at least 90%, at least 91%, at least 92%, at least 93%, at least 94%, at least 95%, at least 96%, at least 97%, at least 98% or at least 99% sequence identity to at least 99.5 %, or at least 99.9% sequence identity to a nucleic acid selected from the group consisting of: SEQ ID NO. 1, SEQ ID NO. 2, SEQ ID NO. 4, and SEQ ID NO. 6.

A third aspect of the invention relates to a vector comprising a nucleic acid sequence according to the second aspect of the present invention.

In some embodiments, a vector is provided comprising a nucleic acid sequence selected from the group consisting of: SEQ ID NO. 2, SEQ ID NO. 4, SEQ ID NO. 6, and variants or fragments thereof which have at least 70 % sequence identity to any of the sequences SEQ ID NO. 2, SEQ ID NO. 4 or SEQ ID NO. 6, wherein the nucleic acid sequence is operably or operatively linked to control sequences directing the expression of said nucleic acid sequences. In some embodiments, the variants or fragments may have at least 80%, preferably at least 90 %, at least 92 %, at least 95 %, at least 97 %, at least 99 %, at least 99.5 %, or at least 99.9% sequence identity to a nucleic acid selected from the group consisting of: SEQ ID NO. 2, SEQ ID NO. 4, and SEQ ID NO. 6.

In a fourth aspect of the invention, a host cell is provided comprising a vector of the third aspect.

According to a fifth aspect of the invention, DNA vaccines are provided comprising a nucleic acid sequence selected from the group consisting of: SEQ ID NO. 2, SEQ ID NO. 4, SEQ ID NO. 6, and variants or fragments thereof which have at least 70 % sequence identity to any of the sequences SEQ ID NO. 2, SEQ ID NO. 4, or SEQ ID NO. 6. In some embodiments, the variants or fragments may have at least 80%, preferably at least 90 %, at least 92 %, at least 95 %, at least 97 %, at least 99 %, at least 99.5 %, or at least 99.9% sequence identity to a nucleic acid selected from the group consisting of: SEQ ID NO. 2, SEQ ID NO. 4, and SEQ ID NO. 6.

A sixth aspect of the invention relates to recombinant proteins encoded by a nucleic acid sequence selected from the group consisting of: SEQ ID NO. 2, SEQ ID NO. 4, SEQ ID NO. 6, and variants or fragments thereof which have at least 70 % sequence identity to any of the sequences SEQ ID NO. 2, SEQ ID NO. 4, or SEQ ID NO. 6. In some embodiments, the variants or fragments may have at least 80%, preferably at least 90 %, at least 92 %, at least 95 %, at least 97 %, at least 99 %, at least 99.5 %, or at least 99.9% sequence identity to a nucleic acid selected from the group consisting of: SEQ ID NO. 2, SEQ ID NO. 4, and SEQ ID NO. 6.

In some embodiments, recombinant proteins according to the present invention have an amino acid sequence selected from the group consisting of: SEQ ID NO. 3, SEQ ID NO. 5, SEQ ID NO. 7, and variants or fragments thereof which have at least 70 % sequence identity to any of the sequences SEQ ID NO. 3, SEQ ID NO. 5, or SEQ ID NO. 7. In some embodiments, the variants or fragments may have at least 80%, preferably at least 90 %, at least 92 %, at least 95 %, at least 97 %, at least 99 %, at least 99.5 %, or at least 99.9% sequence identity to a nucleic acid selected from the group consisting of: SEQ ID NO. 3, SEQ ID NO. 5, and SEQ ID NO. 7.

In a seventh aspect, the present invention provides a recombinant vaccine comprising at least one of the recombinant proteins of the sixth aspect.

In an eighth aspect of the invention, an antibody is provided that specifically recognises and binds to a recombinant protein of the sixth aspect. The antibody may be a monoclonal antibody or a polyclonal antibody.

According to a ninth aspect of the invention, an immunoassay is provided for the detection of antibodies specific for the virus of the invention in a biological sample. The immunoassay comprises the following steps:
a) contacting a biological sample suspected to comprise antibodies raised against the virus of the invention with at least one of the recombinant proteins according to the sixth aspect of the present invention; and
(b) determining whether the at least one recombinant protein binds to antibodies present in the biological sample wherein said binding represents an indication that the sample contains antibodies specific for the virus of the invention.

In some embodiments, the virus of the invention is isolated from lumpfish. In some embodiments, the virus of the invention is from the family flaviviridae, In some embodiments the virus of the invention is a Lumpfish flavivirus.

According to a tenth aspect of the invention an immunoassay is provided for detection of the virus of the invention in a biological sample. The immunoassay comprises the following steps:
(a) contacting a biological sample suspected to comprise the virus of the invention with one or more antibodies that specifically recognise and bind to a recombinant protein according to the sixth aspect of the invention (such as an antibody of the eighth aspect); and
(b) determining whether the one or more antibody binds to the virus of the invention present in the biological sample wherein said binding represents an indication that the sample contains the virus of the invention.

In some embodiments, the one or more antibody may be a monoclonal antibody.

In some embodiments, the virus of the invention is isolated from lumpfish. In some embodiments, the virus of the invention is from the family flaviviridae, In some embodiments the virus of the invention is a Lumpfish flavivirus (LFV).

In an eleventh aspect, the present invention provides a primer comprising a sequence of at least about 10 nucleotides, wherein said primer hybridizes to a nucleic acid sequence originating from the virus of the invention having a sequence selected from the group consisting of: SEQ ID NO. 1, SEQ ID NO. 2, SEQ ID NO. 4, SEQ ID NO. 6, and variants or fragments thereof which have at least 70 % sequence identity to any of the sequences SEQ ID NO. 1, SEQ ID NO. 2, SEQ ID NO. 4, or SEQ ID NO. 6. In some embodiments the primer is suitable for use in PCR and hybridizes to the nucleic acid sequence at a temperature of at least 50 °C. In some embodiments, the variants or fragments may have at least 80%, preferably at least 90 %, at least 92 %, at least 95 %, at least 97 %, at least 99 %, at least 99.5 %, or at least 99.9% sequence identity to a nucleic acid selected from the group consisting of: SEQ ID NO. 1, SEQ ID NO. 2, SEQ ID NO. 4, and SEQ ID NO. 6.

In some embodiments, the primer may consist of or comprise at least 15 nucleotides, such as at least 20 nucleotides.

In some embodiments, the virus of the invention is isolated from lumpfish. In some embodiments, the virus of the invention is from the family flaviviridae, In some embodiments the virus of the invention is a Lumpfish flavivirus (LFV).

In twelfth aspect of the invention, a method for the detection of the virus of the invention in a biological sample is provided. The method comprises the following steps:
a) isolating nucleic acids from a biological sample suspected to comprise the virus of the invention;
b) contacting the nucleic acids with at least one primer in accordance with the eleventh aspect of the invention;
c) subjecting the mixture of b) to stringent hybridisation conditions; and,
d) determining whether hybridisation between the primer and the nucleic acids has occurred, wherein hybridisation indicates the presence of the virus of the invention in the biological sample.

In some embodiments, the mixture of b) is also subjected to reverse transcription and amplification conditions, preferably suitable PCR conditions.

In some embodiments, the stringent hybridisation conditions comprise hybridization in a buffer consisting of 6 X SSC, 50 mM Tris-HCL (pH=7.5), 1 mM EDTA, 0.02% PVP, 0.02% Ficoll, 0.02% BSA and 100 µg/ml denatured salmon sperm DNA, for 48 hours at 65°C, washing in a buffer consisting of 2 X SSC, 25 0.01 % PVP, 0.01% Ficoll, 0.01% BSA for 45 minutes at 37°C, and washing in a buffer consisting of 0.1 X SSC, for 45 minutes at 50°C.
In some embodiments, the virus of the invention is isolated from lumpfish. In some embodiments, the virus of the invention is from the family flaviviridae, In some embodiments the virus of the invention is a Lumpfish flavivirus (LFV).
In a thirteenth aspect, the present invention relates to a diagnostic kit comprising at least one primer sequence in accordance with the eleventh aspect of the invention. In a fourteenth aspect, the present invention relates to the use of the nucleic acid sequences of the second aspect of the invention for the preparation of a DNA vaccine, a recombinant vaccine, or a live recombinant microorganism.

In some embodiments, the isolated virus of the invention may be used in medicine. For example, the isolated virus may be used in inactivated or attenuated form as a vaccine.
In a fifteenth aspect, the isolated virus of the invention may be used as part of a vaccine formulation in a method of preventing an infection in a lumpfish. In some embodiments, the isolated virus may be attenutated or killed. In some embodiments the attenuated or killed virus of the invention may be formulated with an adjuvant.

In a sixteenth aspect, the invention provides a vaccine comprising the virus of the fourteenth or fifteenth aspect in inactivated or attenuated form.

In some embodiments, the virus of the invention is isolated from lumpfish. In some embodiments, the virus of the invention is from the family flaviviridae, In some embodiments the virus of the invention is a Lumpfish flavivirus (LFV).

In some embodiments, the vaccine may be used in the treatment or prevention of liver necrosis in lumpfish.

### BRIEF DESCRIPTION OF THE FIGURES

Figures 1A-1D discloses the DNA sequence (SEQ ID NO. 1) corresponding to the genomic sequence of the Lumpfish flavivirus, which is 11336 nucleotides in length.
Figure 2A and 2B discloses the DNA sequence (SEQ ID NO. 2) corresponding to the open reading frame 1 of the Lumpfish flavivirus. The open reading frame 1 comprises the sequence starting at the nucleotide in position 277 and ending at the nucleotide in position 4323 of SEQ ID NO. 1.
Figure 3 discloses the amino acid sequence coded by the sequence depicted in figure 2, i.e. the amino acid sequence corresponding to SEQ ID NO. 3.
Figure 4A-4C discloses the DNA sequence (SEQ ID NO. 4) corresponding to the open reading frame 2 of the Lumpfish flavivirus. The open reading frame 2 comprises the sequence starting at the nucleotide in position 4320 and ending at the nucleotide in position 10727 of SEQ ID NO. 1.
Figure 5 discloses the amino acid sequence coded by the sequence depicted in figure 4, i.e. the amino acid sequence corresponding to SEQ ID NO. 5.
Figure 6 discloses the DNA sequence (SEQ ID NO. 6) corresponding to the putative envelope protein of the Lumpfish flavivirus. This sequence starts at the nucleotide in position 1297 and ends at the nucleotide in position 2853 of SEQ ID NO. 1.
Figure 7 discloses the amino acid sequence coded by the sequence depicted in figure 6, i.e. the amino acid sequence corresponding to the putative envelope protein, SEQ ID NO. 7.

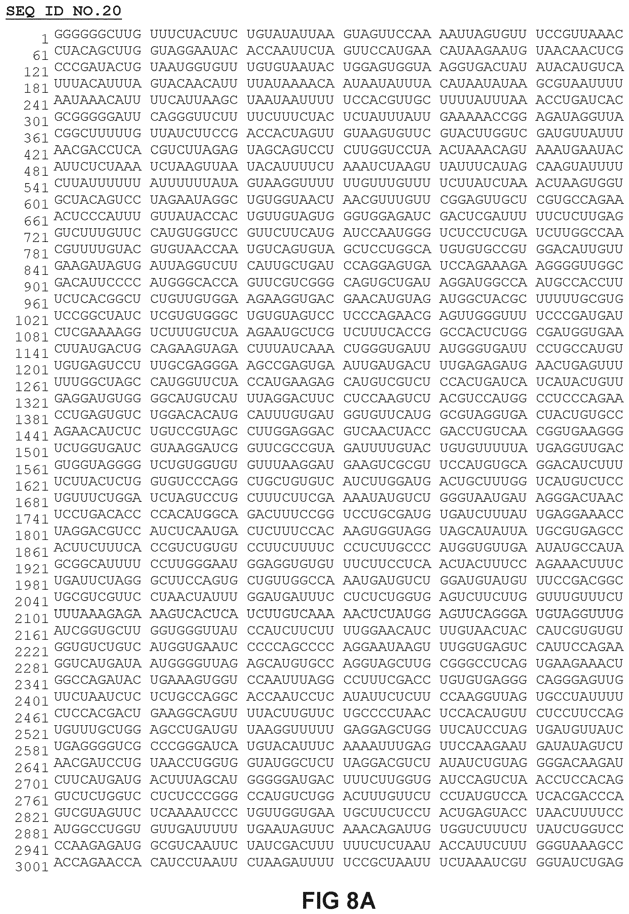

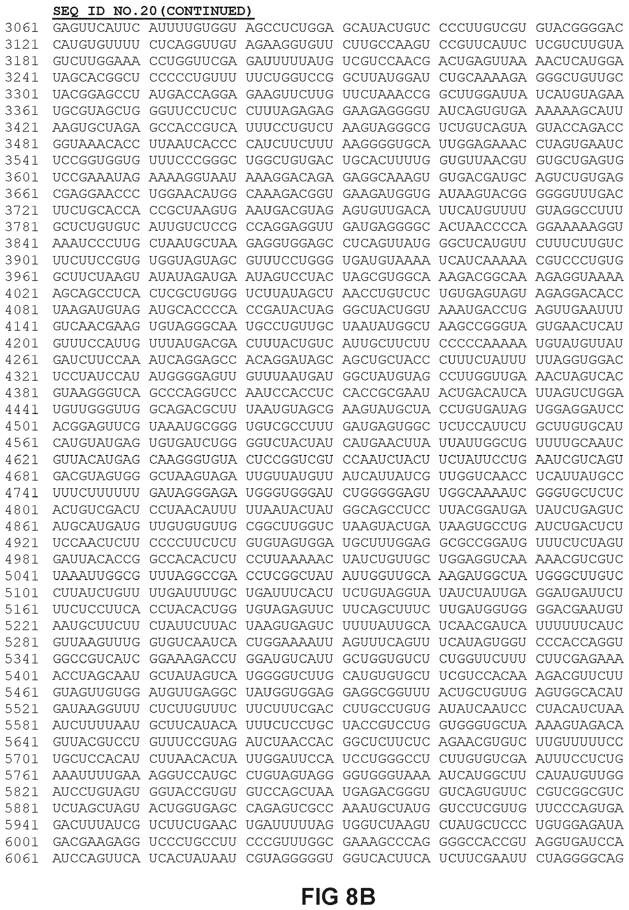

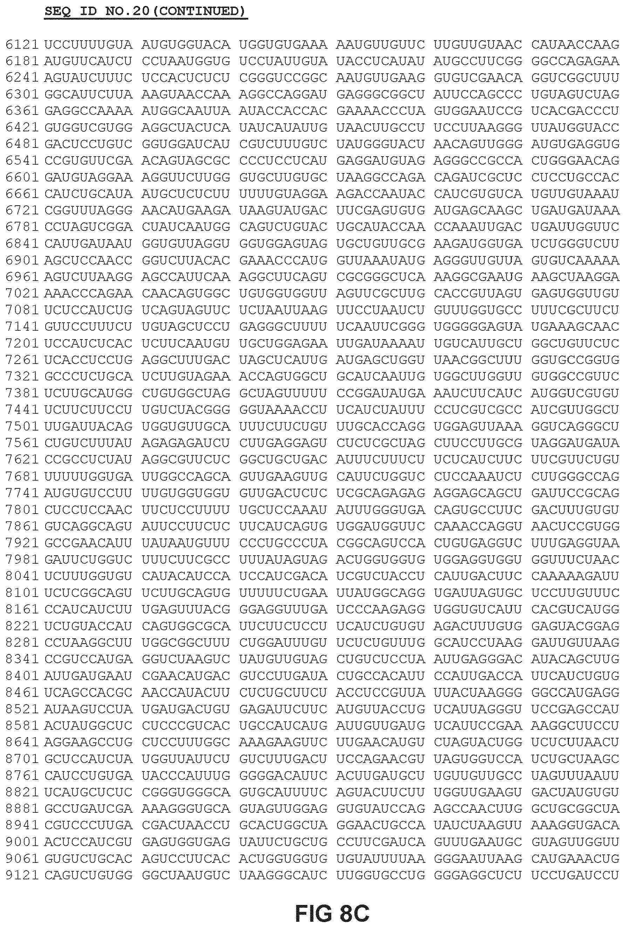

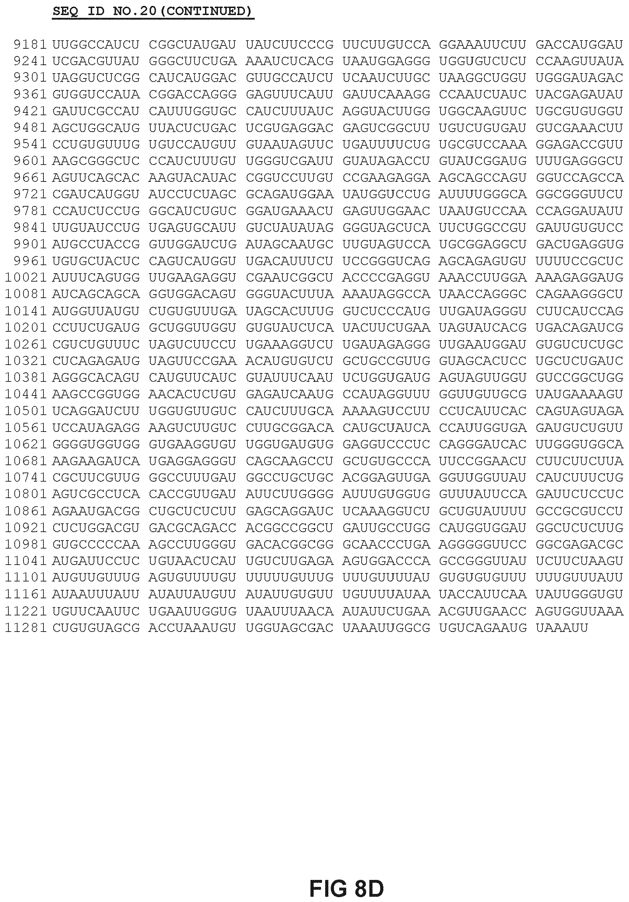

Figure 8A-8D discloses the RNA sequence (SEQ ID NO.8) corresponding to the RNA genome of the Lumpfish flavivirus, which is 11336 nucleotides in length.
Figure 9 discloses the DNA sequence (SEQ ID NO. 21) corresponding assembled sequence of the sequence of the LFV strain 1 isolate of Lumpfish flavivirus.
Figure 10 discloses the DNA sequence (SEQ ID NO. 22) corresponding assembled sequence of the sequence of the LFV strain 2 isolate of Lumpfish flavivirus.
Figure 11 discloses the DNA sequence (SEQ ID NO. 23) corresponding assembled sequence of the sequence of the LFV strain 3 isolate of Lumpfish flavivirus.
Figure 12 discloses the DNA sequence (SEQ ID NO. 24) corresponding assembled sequence of the sequence of the LFV strain 4 isolate of Lumpfish flavivirus.
Figure 13 discloses the DNA sequence (SEQ ID NO. 25) corresponding assembled sequence of the sequence of the LFV strain 5 isolate of Lumpfish flavivirus.

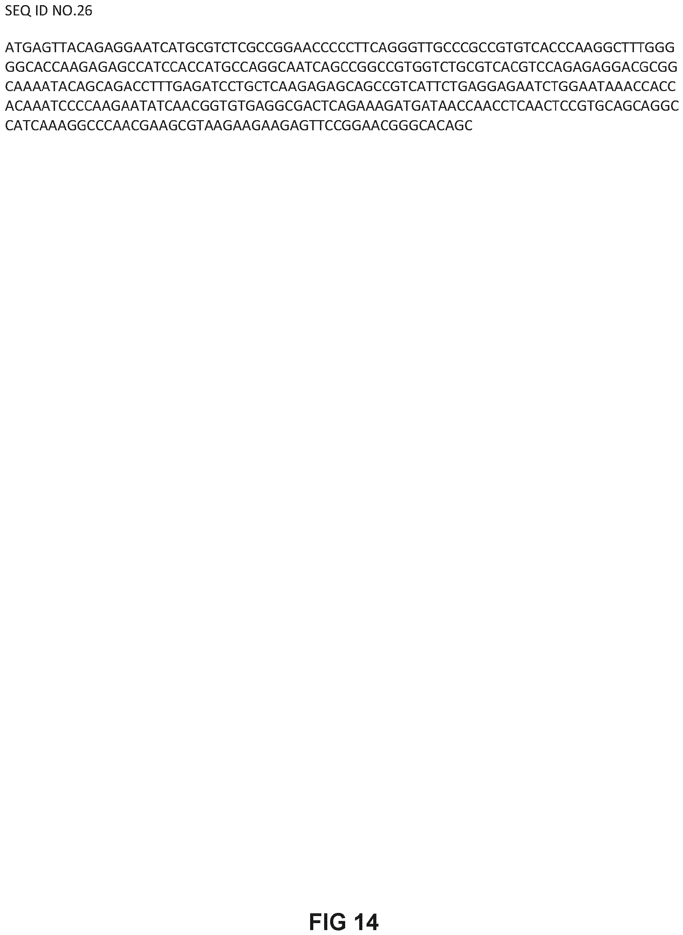

Figure 14 discloses the DNA sequence (SEQ ID NO. 26) corresponding assembled sequence of the sequence of the LFV strain reference of Lumpfish flavivirus.

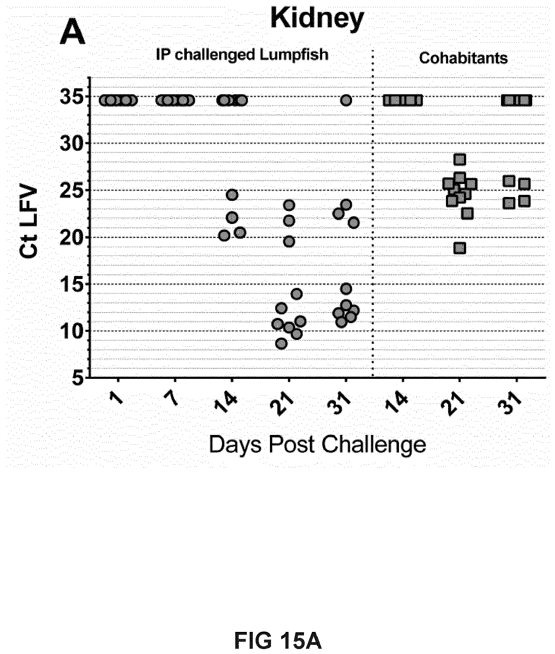

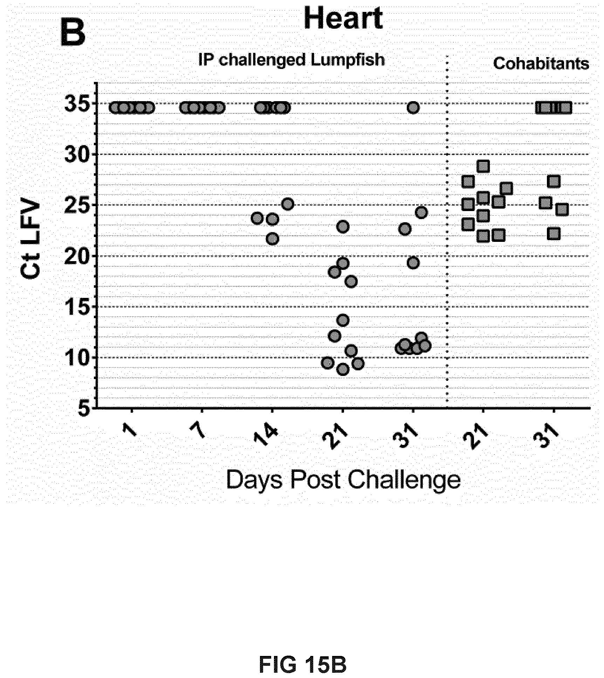

Figure 15A and B graphically discloses the LFV Ct values of Lumpfish tissue samples from (A) Kidney and (B) Heart tissue sampled at 1, 7, 14, 21 and 31 days post challenge. Each icon represents one fish. Ten fish are sampled per time point.

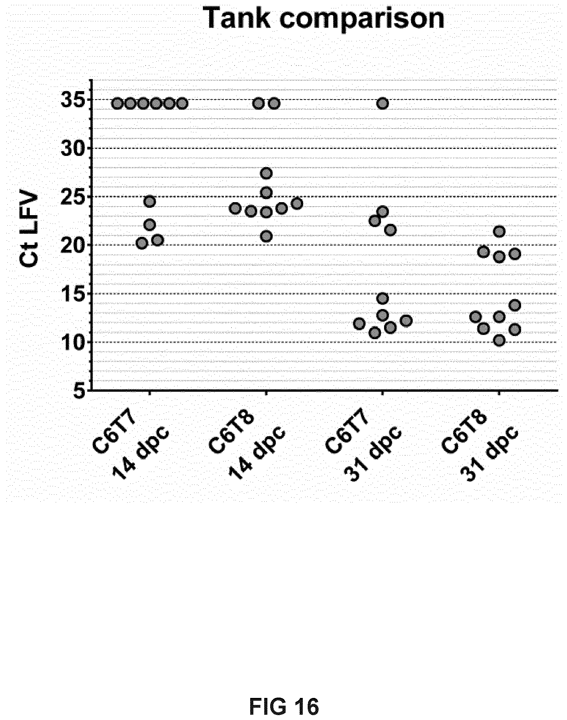

Figure 16 graphically discloses the LFV Ct values of Lumpfish kidney tissue samples sampled at 14 and 31 days post challenge. Each icon represents one fish. Ten fish are sampled per tank per time point.
Figure 17 discloses a photographic image of a histological section of a normal lumpfish liver.
Figure 18 discloses a photographic image of a histological section of a lumpfish liver with extensive tissue damage sampled at 21 days post IP challenge with LFV containing tissue homogenate.
Figure 19 discloses a photographic image of a histological section of a lumpfish liver with extensive tissue damage sampled at 31 days post IP challenge with LFV containing tissue homogenate.
Figure 20 discloses a photographic image of an ethidium bromide stained agarose gel showing the PCR product using the LFV1 forward and reverse primers, the products of which were sequenced and aligned to show variant LFV.
Figure 21 discloses a photographic image of an ethidium bromide stained agarose gel showing the PCR product using the LFV2 forward and reverse primers, the products of which were sequenced and aligned to show variant LFV.
Figure 22 discloses a photographic image of an ethidium bromide stained agarose gel showing the PCR product using the LFV3 forward and reverse primers, the products of which were sequenced and aligned to show variant LFV.
Figure 23 discloses a photographic image of an ethidium bromide stained agarose gel showing the PCR product using the LFV4 forward and reverse primers, the products of which were sequenced and aligned to show variant LFV.
Figure 24 represents a phylogenetic tree constructed from the nucleotide alignments of the five variant LFV strains isolated.
Figure 25 represents individual sequence alignment and consensus sequence of the putative structural proteins of the five variant LFV isolated

### DETAILED DESCRIPTION OF THE INVENTION

The present invention and the various embodiments thereof will now be described in detail in the following.

### Definitions

The present invention relates to isolated nucleic acid sequences and variants or fragments thereof which have at least 70% sequence identity to the isolated nucleic acid sequences. The terms "% sequence identity", "% identical", "% identity", "% homology", and similar terms, are to be understood to refer to the percentage of nucleotides that two or more sequences or fragments thereof contain that are the same. A specified percentage of nucleotides can be referred to as having, for example, 70%, 80%, 85%, 90%, 95%, 99% sequence identity or homology over a specified region when compared and aligned for maximum correspondence. Unless otherwise stated, the determination of sequence identity/homology is based on a comparison of the entire sequences, and not on selected portions. The skilled person will acknowledge that various means for comparing sequences are available. For example, one non-limiting example of a useful computer sequence alignment program useful for determining the percent sequence identity/ homology between sequences includes the Basic Local Alignment Search Tool (BLAST) (Altschul et al., 1990, J. of Molec. Biol., 215:403-410, "The BLAST Algorithm; Altschul et al., 1997, Nuc. Acids Res. 25:3389-3402, , Karlin and Altschul 1990, Proc. Nat'l Acad. Sci. USA, 87:2264-68; 1993, Proc. Nat'l Acad. Sci. USA 90:5873-77).

The term "fragments or variants thereof" used in respect of the nucleic acid sequences and recombinant proteins according to the present invention is to be understood to encompass nucleic acid sequences and recombinant proteins that only differ from the isolated sequences SEQ ID NO. 1-26 by way of some amino acid or nucleotide additions, deletions or alteration that have little or no effect on the functional activity of the claimed sequences. The skilled person will acknowledge that modifications of a protein coding nucleotide sequence may be introduced which do not alter the amino acid sequence, e.g. the substitution of a nucleotide resulting in that the triplet affected by the substitution still codes for the same amino acid. Such alterations may, for example, be introduced to adapt the nucleic acid sequence to the codons preferably used by a host cell and thus to enhance the expression of a desired recombinant protein. Furthermore, the addition of nucleic acid sequences coding polypeptides which facilitate purification may be added without affecting the activity of the resulting recombinant protein.

The skilled person will further acknowledge that alterations of the nucleic acid sequence resulting in modifications of the amino acid sequence of the corresponding recombinant protein may have little, if any, effect on e.g. the protein's ability to induce protection against Lumpfish flavivirus infection, if the alteration does not have any impact on the resulting three dimensional structure of the recombinant protein. For example, a codon for the amino acid alanine, a hydrophobic amino acid, may be substituted by a codon encoding another less hydrophobic residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, can also be expected to produce a recombinant protein with substantially the same functional activity as the protein having the amino acid sequences as depicted in SEQ ID NO.s 3, 5, and 7, and thus to be expected to constitute a biologically equivalent product. Nucleotide changes which result in alteration of the N-terminal or C-terminal portions of the protein molecule would also not be expected to alter the functional activity of the protein. Each of the proposed modifications is well within the routine skill in the art, as is determination of retention of biological activity of the encoded products. Therefore, where the terms "nucleic acid sequences of the invention" or "recombinant protein of the invention" are used in either the specification or the claims each will be understood to encompass all such modifications and variations which result in the production of a biologically equivalent protein.

Thus, the present invention therefore encompasses recombinant proteins and fragments thereof which differ in respect of amino acid substitutions, addition or deletions, compared with the recombinant proteins having the sequence of SEQ ID NO. 3, SEQ ID NO. 5, and SEQ ID NO. 7, respectively, but which exert substantially the same functional activity as the proteins having the sequence of SEQ ID NO. 3, SEQ ID NO. 5, and SEQ ID NO. 7.

Terms such as "antigen", "immunogenic protein or peptide", "antigenic determinant", and "epitope", when used in connection with the present invention, are to be understood to refer to a recombinant protein or fragment thereof according to the invention which is able to induce protection against Lumpfish flavivirus infection in fish or which is able to bind to an antibody which recognises and binds to the Lumpfish flavivirus. A fragment of a recombinant protein having a sequence as depicted in SEQ ID NO. 3, SEQ ID NO. 5, and SEQ ID NO. 7, respectively may constitute such an antigen or epitope. A non-limiting example of an antigenic fragment is a fragment constituting, for example, 8-11 amino acids of the sequence SEQ ID NO. 3, SEQ ID NO. 5, or SEQ ID NO. 7.

The term "originating from", as used herein, refers to a DNA sequence that was originally isolated from an RNA virus, reverse transcribed and made into double stranded DNA molecules. The DNA sequences of the present invention originated from the RNA genome of the virus of the invention.

The term "vaccine" as used herein refers to a material that can produce an immune response that blocks the infectivity, either partially or fully, of an infectious agent, which in respect of the present invention is the Lumpfish flavivirus affecting fish such as e.g. Lumpfish. Thus, when the vaccines of the invention are administered to a fish, the fish is immunised against the disease caused by Lumpfish flavivirus. The immunising component of the vaccine may be e.g. DNA as in a DNA vaccine, a recombinant protein or fragment thereof according to the present invention, a live recombinant microorganism, or an inactivated or attenuated virus.

When used in relation to the virus of the present invention, the term "isolated" refers to the virus when separated from its natural environment, such as the cells and tissues from its natural host. In particular, the term "isolated" refers to a culture, such as a pure culture of the virus derived from a tissue sample from an infected host. As viruses cannot reproduce on their own and use host cell machinery to make both the viral genome and capsids, the term "isolated" may in particular refer to a culture of cells infected with the virus. The term "isolated" may further refer to a virus which is substantially free of other viral or microbial material. The term "substantially free of other viral or microbial material" as used herein refers to a preparation of a virus in which other viral or microbial material cannot be detected using conventional techniques like seeding on TSA or cysteine heart agar spread plates, seeding in cell cultures known to support the growth of fish viruses like Pancreas Disease Virus, Infectious Salmon Anemia virus and Infectious pancreatic Necrosis virus and PCR with primers designed against known sequences from fish pathogens. Further, it is to be understood that when "substantially free of other viral or microbial material" the virus of the invention is in a form wherein it may be used for therapeutic purposes. This definition does not exclude applications in which a pure culture of the virus according to the invention is combined with other vaccine antigens, e.g. other bacterial or viral antigens in compositions, e.g. compositions intended for vaccination purposes.

As outlined below, the present invention also provides primers (i.e. oligonucleotide sequences) useful as diagnostic tools. The term "primer" as used herein refers to an oligonucleotide produced from a longer sequence (e.g. as a restriction fragment) or produced synthetically, which is significantly complementary to a Lumpfish flavivirus target sequence and thus capable of hybridizing to a nucleic acid sequence of the present invention. The primer may be labelled to facilitate detection, e.g. using fluorescent labels or other label means known to the skilled person.

### Detailed description of the embodiments of the invention

It is to be understood that content of prior art documents referred to herein is incorporated herein by reference in its entirety.

### Lumpfish flavivirus

The present inventors have identified and isolated a new virus, referred to herein as "Lumpfish flavivirus" (LFV). According to specific embodiments of the invention, the virus comprises in its genome a ribonucleic acid sequence, SEQ ID NO. 20 and which, by reverse transcription and two-strand synthesis provides the DNA sequence set forth in SEQ ID NO: 1, or a closely related or variant sequence. *Nucleic acid sequences*

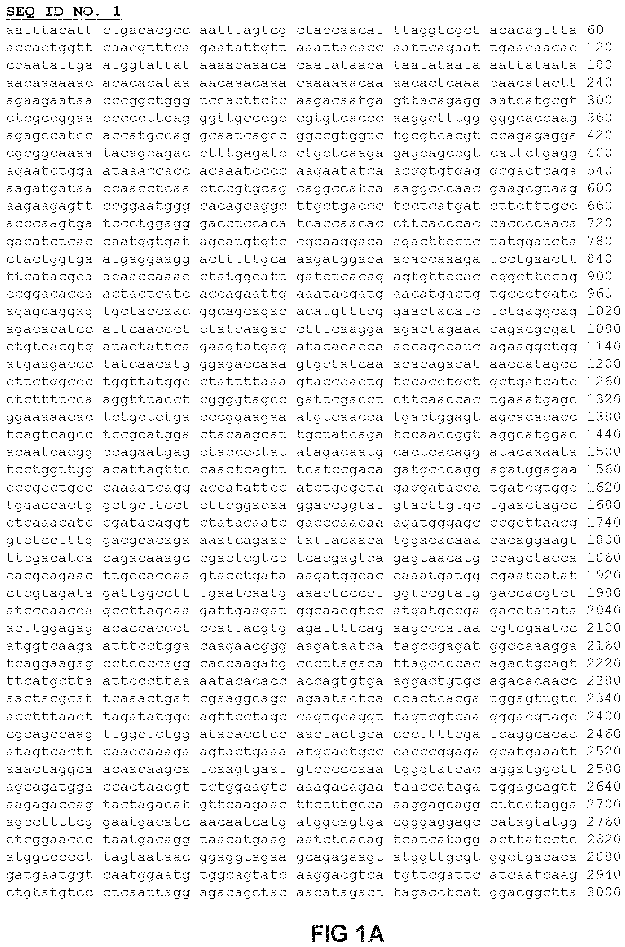

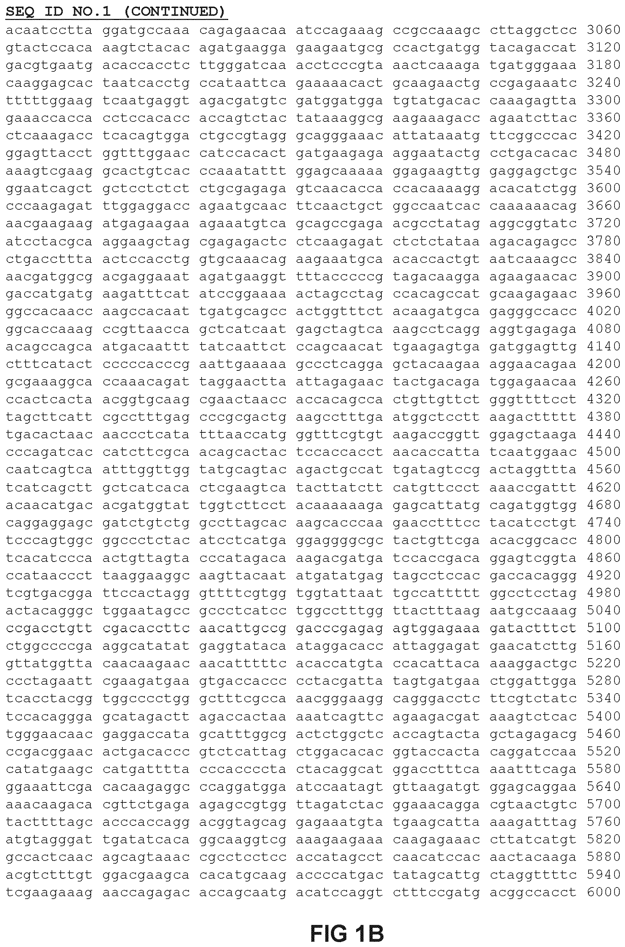

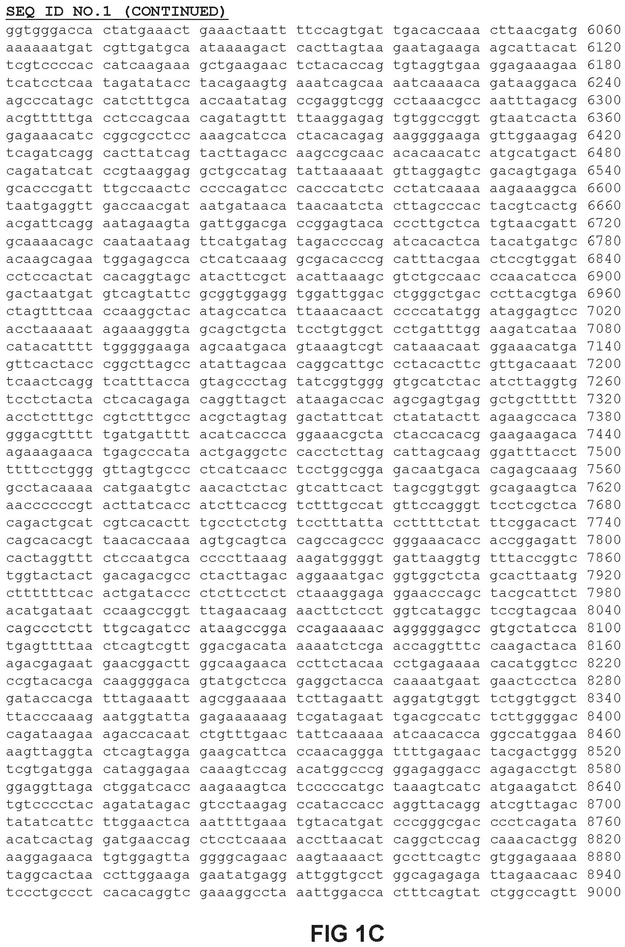

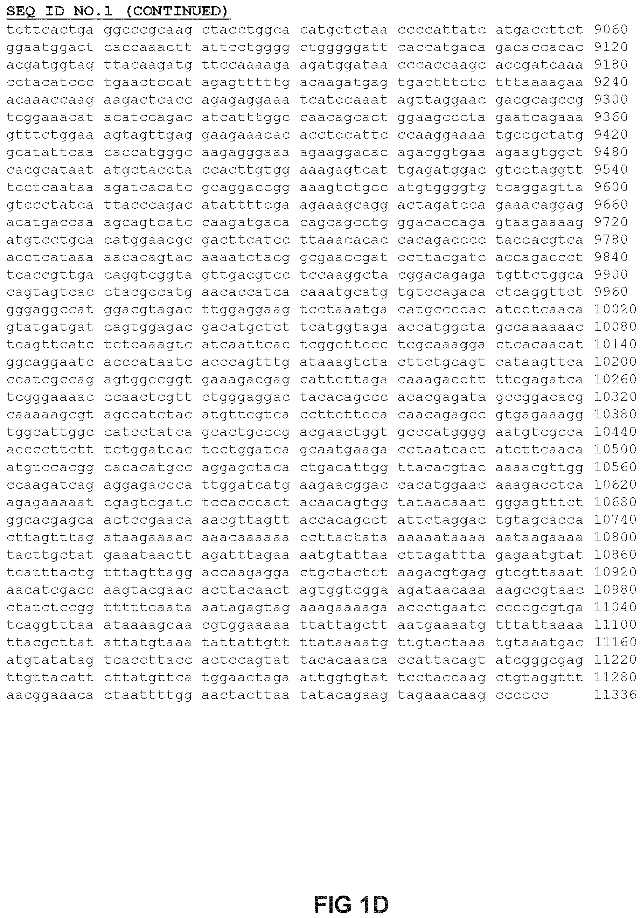

The present invention describes the genomic sequence of the new Lumpfish flavivirus (LFV). Tissue was collected from lumpfish showing clinical signs of disease, with extensive liver necrosis. Tissue was homogenized, pelleted by centrifugation, and the supernatant subjected to DNase and RNase treatment. RNA was then isolated and cDNA synthesised using random primers. Thereafter, double stranded DNA was provided and amplified by PCR using random primers, and the obtained products were sequenced and analysed to obtain part of the genomic sequence. The sequence of the rest of the genome was obtained by PCR with specific primers and 3' and 5' RACE (Rapid Amplification of cDNA Ends), and the DNA sequence corresponding to the full genomic sequence of the virus is given in figure 1 and SEQ ID NO. 1 and further represented as the ribonucleic acid sequence of the RNA genome as set forth in SEQ ID NO.20..

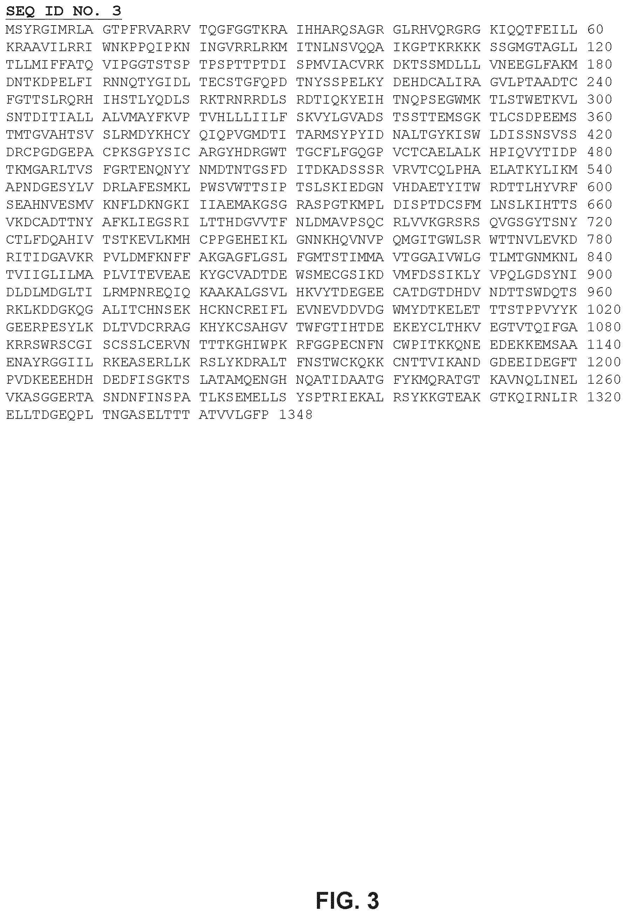

The further analysis of SEQ ID NO. 1 by the present inventors revealed two open reading frames which are believed to include the coding region for several viral proteins. Sequence comparison with other known viral sequences has revealed that the open reading frame 1 (SEQ ID NO. 2) according to the present invention shows very weak homology to other viral sequences. However, certain putative protein motives can be identified, and an organisation corresponding to the common outline of Flaviviridae is suggested. Furthermore, the analysis of the open reading frame 1 (SEQ ID NO. 2) indicates that it comprises a nucleic acid sequence coding an envelope protein (SEQ ID NO. 6). The amino acid sequence of the open reading frame 1 is shown in figure 3 and SEQ ID NO. 3. Said sequence shows only very weak homology with other known proteins.

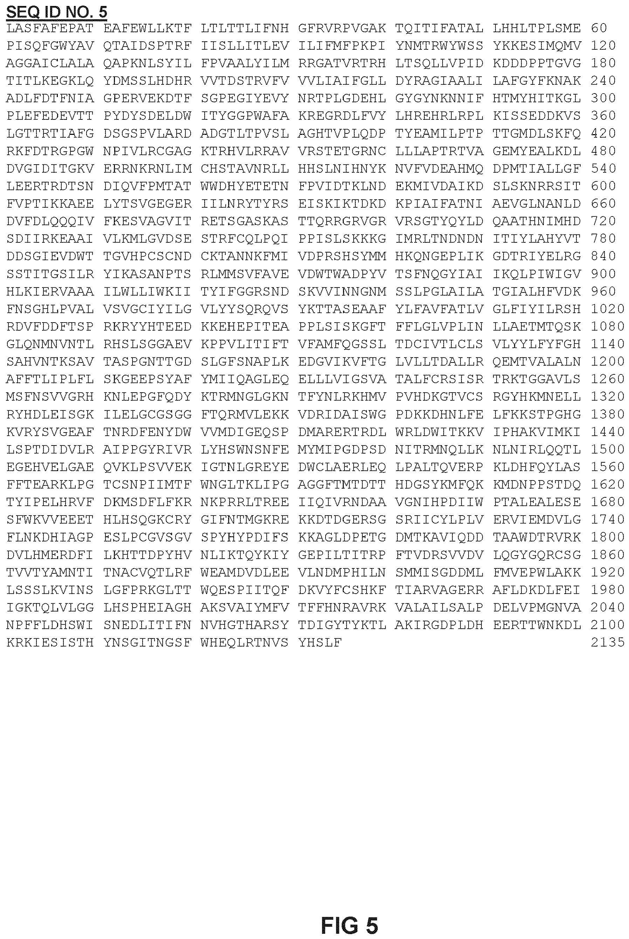

A further isolated nucleic acid sequence of the invention (open reading frame 2, SEQ ID NO. 4) comprises a sequence coding putative proteins and the amino acid sequence thereof is depicted in figure 5 and SEQ ID NO. 5

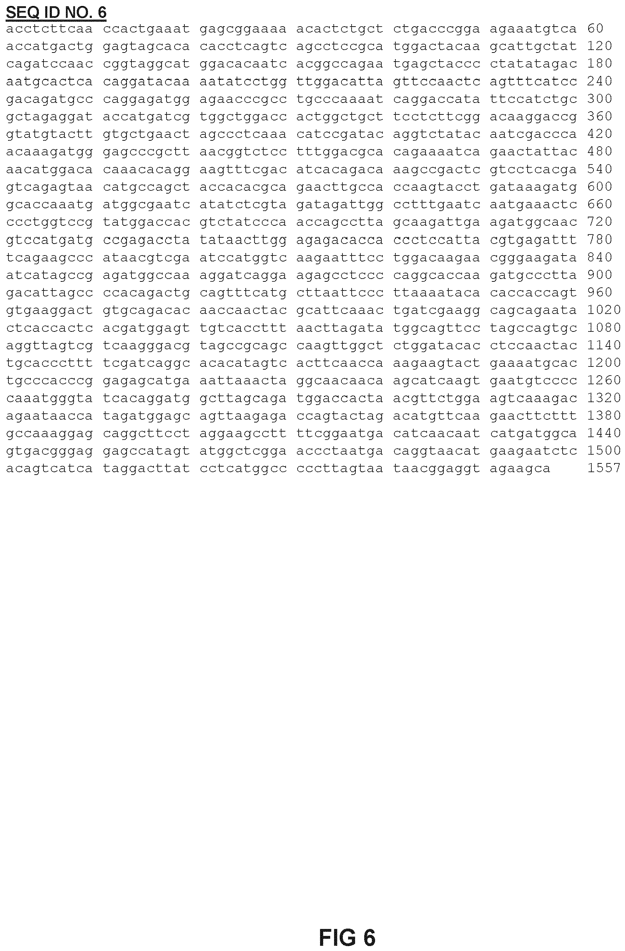

The SEQ ID NO. 6 of the invention comprises a sequence which is also believed to include the coding sequence of a putative envelope protein, the amino acid sequence thereof being depicted in figure 6/SEQ ID NO. 6. Surprisingly, said sequence shows only very weak homology with other viral envelope proteins, and no other members of the Flaviviridae family comprise this particular open reading frame.

The discovery of the Lumpfish flavivirus and the determination and disclosure of the viral nucleotide sequences advantageously facilitates substantial progress in respect of controlling and preventing liver necrosis in fish. For example, the sequences of the open reading frames 1, 2, or the putative envelope protein (SEQ ID NO. 2, SEQ ID NO.4, or SEQ ID NO. 6), may be used in the development of recombinant proteins to be used in vaccines, or the development of DNA vaccines or a live recombinant microorganism.

Recombinant proteins which are coded for by sequences based on the open reading frames 1, 2, or the putative envelope protein (SEQ ID NO. 2, SEQ ID NO.4, or SEQ ID NO. 6), may also be used in the development of diagnostic tools, for example, through the production of antibodies and the development of immunoassays for the detection of the presence of Lumpfish flavivirus in biological samples.

In addition, the sequences provided may be used in the development of diagnostic tools. For example, the nucleic acid sequences or fragments thereof may be used to detect the presence of Lumpfish flavivirus in fish or other biological samples.

Various aspects and embodiments of the invention resulting from the disclosed viral nucleic acid sequences are outlined further below. The skilled person will acknowledge that said embodiments should not be understood to represent a limit on the scope of the uses of the claimed nucleic acid sequences. Other uses of the claimed nucleic acid sequences are also understood to be covered by the scope of the present invention.

### Recombinant proteins

According to one aspect of the invention, the invention provides recombinant Lumpfish flavivirus proteins.

The skilled person is well familiar with the various available biotechnological techniques providing for the expression of isolated nucleic acid sequences for the preparation of recombinant proteins by heterologous expression in various host cell systems using commonly available genetic engineering techniques and recombinant DNA expression systems. Various protocols for the expression of recombinant protein are known, cf. e.g. "Recombinant Gene Expression Protocols, in Methods in Molecular Biology, 1997, Ed. Rocky S Tuan, Human Press (ISSN 1064-3745) or Sambrook et al., Molecular Cloning: A laboratory Manual (third edition), 2001, CSHL Press, (ISBN 978-087969577-4). For example, the nucleic acid sequences of the open reading frames 1 and 2 or fragments or variants thereof may be inserted in suitable expression vectors comprising all the necessary transcriptional and translational regulatory sequences specifically adapted for directing the expression of the desired protein coding nucleic acid sequence in a suitable host cell. Suitable expression vectors are e.g. plasmids, cosmids, viruses or artificial yeast chromosomes (YAC's). A non-limiting list of examples of suitable expression vector systems is e.g. pET-type of vectors (available from Invitrogen), pcDNA3.1 vectors (available from Invitrogen), or the pBR-type, pUC-type or pGEM-type of expression vectors. The obtained expression vector including the open reading frames (SEQ ID NO. 2, SEQ ID NO. 4 or SEQ ID NO. 6) or a fragment thereof is then introduced in suitable host cells for the production of the desired recombinant protein.

According to one embodiment of the invention, a vector is provided comprising a sequence selected from the group consisting of SEQ ID NO. 2, SEQ ID NO. 4 or SEQ ID NO. 6, and variants or fragments thereof which have at least 70 % sequence identity to any of the sequences SEQ ID NO. 2, SEQ ID NO. 4, or SEQ ID NO. 6, operatively linked to control sequences directing the expression of said sequences. The term "operatively linked", means that the desired sequence coding for a recombinant protein is linked to all the necessary transcriptional and translational regulatory sequences specifically adapted for directing the expression of the desired protein coding nucleic acid sequence in a suitable host cell.

It should be understood that various modifications may be introduced in the nucleic sequences of the present invention utilising techniques well known to the skilled person for example to facilitate expression. By the use of site directed mutagenesis, modification may be introduced to adapt the coding sequence to the desired host used to express the sequence and thus produce the recombinant protein. The skilled person is well aware of the fact of the presence of host specific codons, and that the adaption of a heterologous nucleic acid sequence to the host specific codons increase the expression efficiency. Other modification may also be introduced, e.g. to facilitate isolation and purification, i.e. by adding a sequence coding for a peptide or protein useful for such purposes. Also, nucleic acid sequences coding signal peptide providing for secretion of the desired recombinant protein from the host cell may also be linked to the nucleic acid sequences of the present invention. In case the recombinant protein is expressed in a live recombinant microorganism (see below), the nucleic acid sequence may be modified to enable the anchorage of the recombinant protein in the membrane or cell wall of the microorganism resulting in the presentation of the recombinant protein on the surface of the live recombinant microorganism. According to such an embodiment of the invention, the nucleic acid sequence of the present invention is modified by adding a nucleic acid sequence coding a protein or peptide resulting in the anchorage of the resulting recombinant protein in the membrane or cell wall of the live recombinant microorganism.

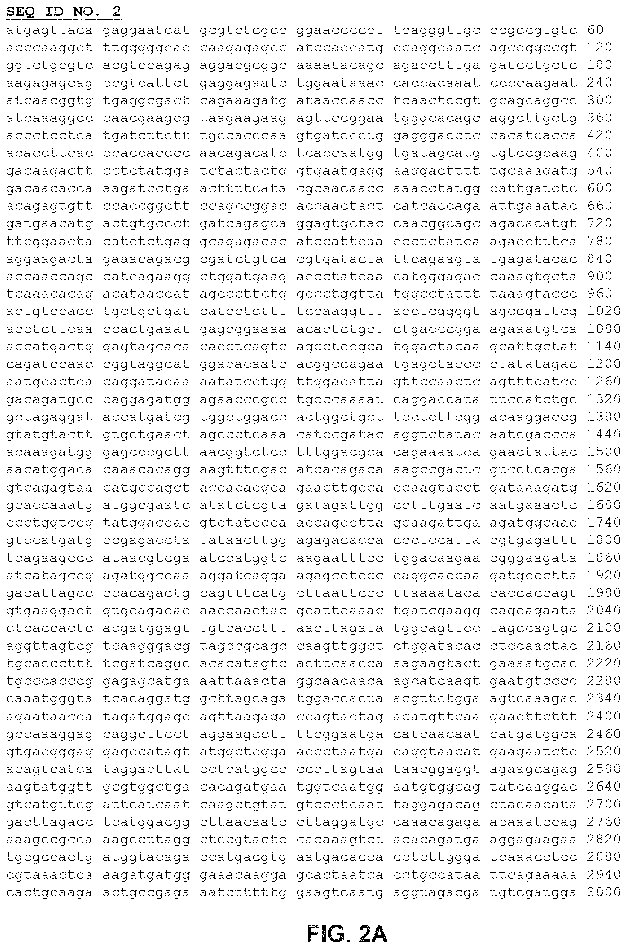

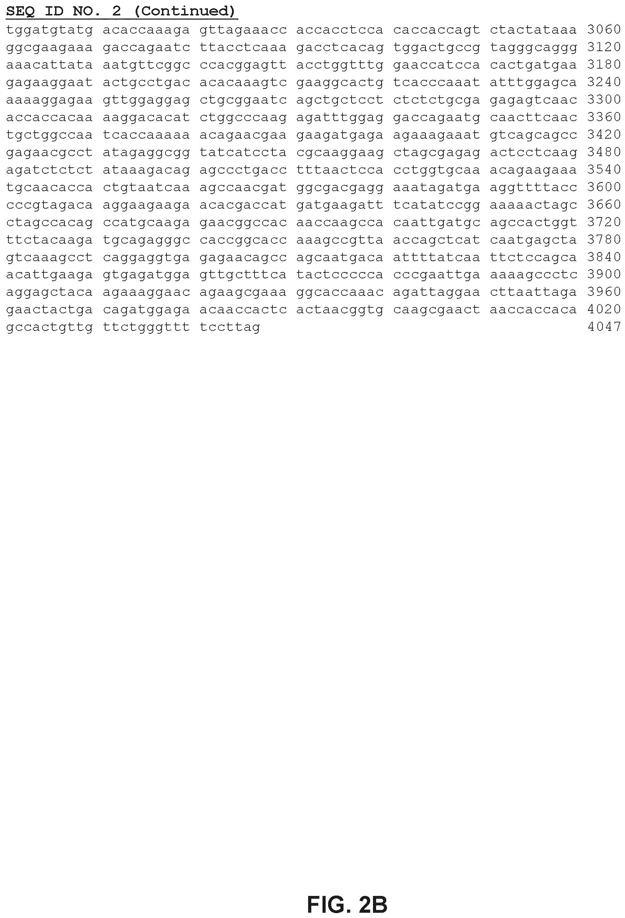

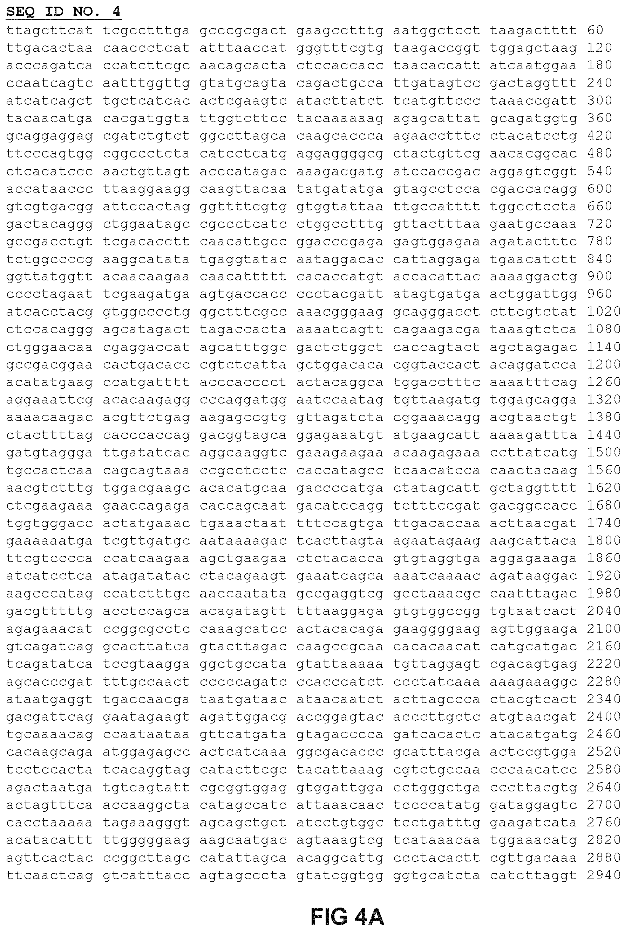

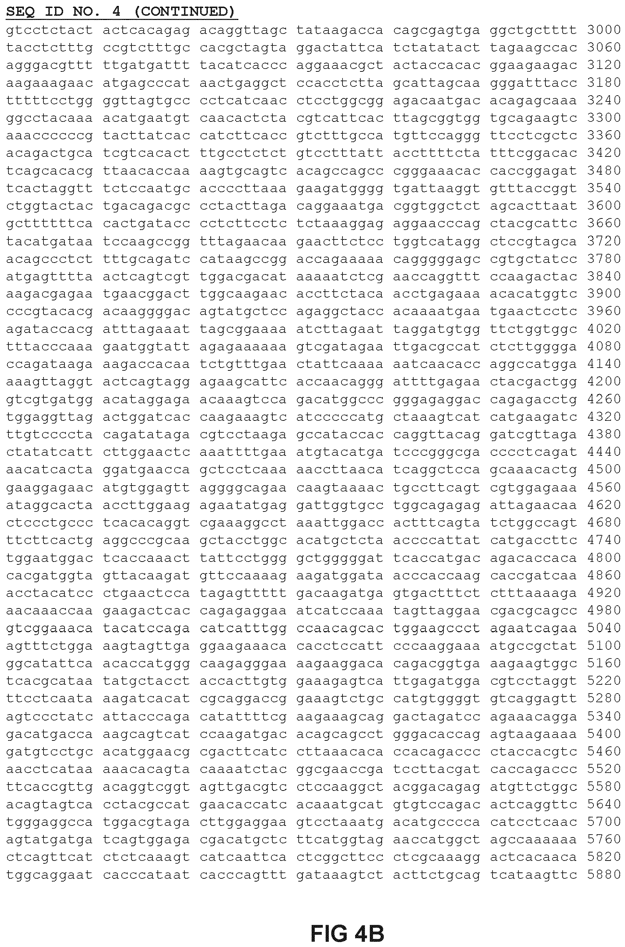

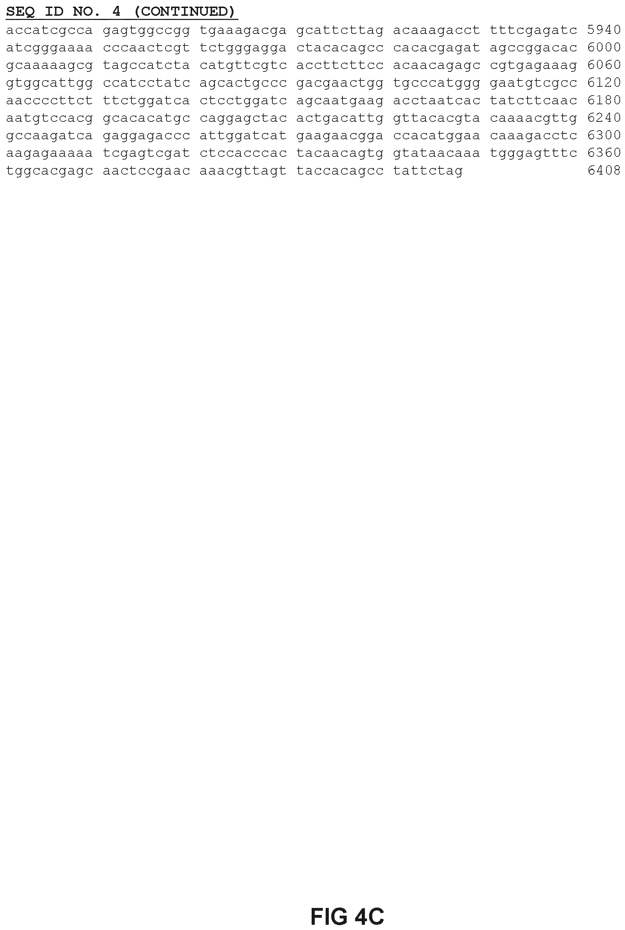

The present invention thus provides vectors comprising the nucleic sequence as depicted in figure 2 and/or 4 and/or 6 (SEQ ID NO. 2, SEQ ID NO. 4 or SEQ ID NO. 6) or variants or fragments thereof. The present invention also provides host cells comprising the vectors of the invention.

Various commercially available host cells specifically adapted for the production of recombinant proteins may be used, including both prokaryotic host cells and eukaryotic host cells. Non-limiting examples of suitable prokaryote host cells are *Escherichia Coli, Bacillus substilis, Lactobacilus sp, Caulobacter cresentus, Yersinia ruckeri, or Vibrio anguillarum.* Non-limiting examples of suitable eukaryote host cells are yeast such as *Saccharomyces cerevisiae,* or *Chinese hamster ovary cells.* Viruses may also be used for the recombinant expression of a desired protein, cf. e.g. alphavirus, adenovirus or baculovirus vector systems (Bac-to-Bac® Baculovirus Expression Systems, Invitrogen, PichiaPink™ Yeast Expression System, Invitrogen).

According to one embodiment of the invention, a pET vector available from Invitrogen and specifically adapted for the expression of recombinant proteins in *Escherichia Coli* is used. The pET vector is an example of a bacterial expression vector and the invention is not limited to one vector system. One of skill in the art will recognize that other bacterial expression vectors may be used in place of a pET vector and yield positive results.

Recombinant protein may also be provided by using cell free expression systems. Recombinant proteins being the results of expression of the open reading frames 1 or 2 or putative envelope protein (SEQ ID NO. 2, 4 or 6) or fragments, variants, or combinations thereof may be used in a vaccine to obtain protection against further Lumpfish flavivirus infections.

### Recombinant vaccine composition

Recombinant proteins prepared by heterologous expression of the nucleic acid sequences according to the present invention may be used as immunogenic component(s) of a recombinant vaccine composition.

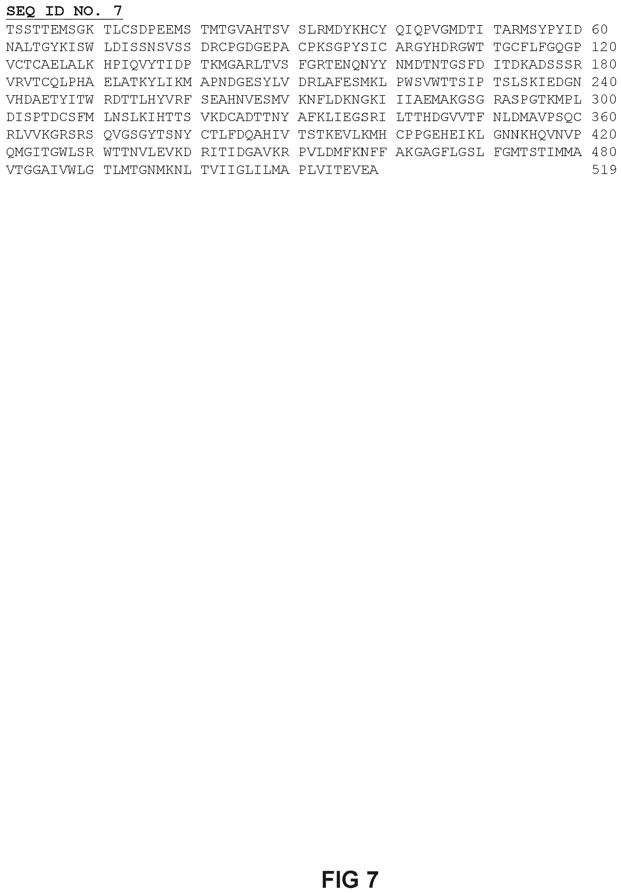

The present invention therefore provides a vaccine composition comprising at least one recombinant protein having an amino acid sequence as depicted in figure 3, 5 or 7 (SEQ ID NO. 3, SEQ ID NO. 5 or SEQ ID NO. 7), or fragments or variants thereof.

According to one aspect of the invention, the at least one recombinant protein used in a vaccine consists of or comprises a fragment of the envelope protein coded for by the sequences of the SEQ ID NO. 6 that induce an immune response in the individual being administered the recombinant vaccine of the invention. Such fragments may constitute an epitope of the envelope protein coded for by SEQ ID NO. 6, is also known as an *antigenic determinant.* The skilled person is well familiar with the fact that the envelope proteins of a virus may comprise one or more epitopes that constitutes the part of the envelope protein that is recognized by the immune system and thus result in the development of protection against further infection. The epitopes may be found as linear, continuous sequence of amino acids, such as e.g. 8-11 amino acids, within an envelope protein or another surface structure of a pathogen organism. Such an epitope is commonly named a linear epitope. An epitope of an envelope protein may also be the results the three dimensional structure of the envelope protein, e.g. formed by the assembling of amino acids of the sequence that are not sequential. Such epitopes are commonly named discontinuous epitope, and are often larger than a linear epitope.

Linear and discontinuous epitopes comprised in the envelope protein of the invention may be determined by the use of epitope mapping technology available to the skilled person, c.f. Methods in Molecular Biology: Epitope Mapping Protocols, 1996, vol. 66, Ed. Glenn E Morris, Human Press (ISBN 978-0-89603-375-7). It is to be understood that vaccines comprising recombinant proteins according to the present invention which constitute protecting epitopes of the envelope protein of Lumpfish flavivirus, prepared by the expression of fragments of the putative envelope protein open reading frame (SEQ ID NO. 6), or variants or fragments thereof, are covered by scope of the present invention. More specifically, epitopes which are recognized by neutralizing antibodies will be of particular interest.

Epitopes that are found in those parts of the recombinant proteins according to the present invention that are particularly specific for the Lumpfish flavivirus are preferred.

The recombinant vaccine composition according to the invention comprising recombinant proteins or fragments thereof as outlined above may further comprise an adjuvant. Examples of adjuvants frequently used in fish and shellfish farming are muramyldipeptides, lipopolysaccharides, several glucans and glycans, mineral oil, Montanide™ and Carbopol®. An extensive overview of adjuvants suitable for fish and shellfish vaccines is given in the review paper by Jan Raa, 1996, Reviews in Fisheries Science 4(3): 229-228.

The vaccine of the invention may further comprise a suitable pharmaceutical carrier. In a currently preferred embodiment the vaccine is formulated as an emulsion of water in oil. The vaccine may also comprise a so-called "vehicle". A vehicle is a device to which the antigen adheres, without being covalently bound to it. Such vehicles include, for example, biodegradable nano/micro-particles or - capsules of PLGA (poly-lactide-co-glycolic acid), alginate or chitosan, liposomes, niosomes, micelles, multiple emulsions and macrosols, all of which are known in the art. A special form of such a vehicle, in which the antigen is partially embedded in the vehicle, is the so-called ISCOM (European patents EP0109942, EP0180564 and EP0242380.

In addition, the vaccine may comprise one or more suitable surface-active compounds or emulsifiers, e.g. Cremophore®, Tween® and Span®. Adjuvants such as interleukin, CpG and glycoproteins may also be used.

It is to be understood that the vaccine may further be in a formulation comprising an antigen from a bacterial source, an antigenic material obtained from a viral source other than the fish virus as described herein, an antigenic material obtained from a parasitical source, and/or an antigenic material obtained from a fungal source. Polyvalent vaccines containing antigens from typical fish pathogens other than Lumpfish flavivirus are well known in the art and are already commercially available. In addition, representative isolates of relevant fish pathogens are available from various sources.

In particular embodiments of the invention said antigen from a bacterial source is selected from the group consisting of: live, attenuated or killed bacteria of the species *Piscirickettsias sp. Aeromonas sp., Vibrio sp., Listonella sp., Moritella viscosa, Photobacterium damsela, Flavobacterium sp., Yersinia sp., Renibacterium sp., Streptococcus sp., Lactococcus sp., Leuconostoc sp., Bifidobacterium sp., Pediococcus sp., Brevibacterium sp., Edwarsiella sp., Francisella sp., Pseudomonas sp., Cytophaga sp., Nocardia sp., Mycobacerium sp.,* parts or subunits of these bacteria, and any combination hereof.

Isolates of such bacteria are available, e.g. from LGC Promochem/American Type Culture Collection ATCC repository and distribution center (ATCC) including strains of *A. salmonicida* (ATCC 33658), *V. salmonicida* (ATCC 43839), *V. anguillarum* serotype O1(ATCC 43305) and O2(ATCC 19264), and *Moritella viscosa* (ATCC BAA-105). In addition, cultures of *Piscirickettsias salmonis* have been deposited in the European Collection of Cell Culture (ECACC), Health Protection Agency, Porton Down, Salisbury, Wiltshire (UK), SP4 0JG UK on the 9 June 2006 under the following accession numbers: 06050901, 06050902, 06050903 and 07032110.

Other specific embodiments pertain to a vaccine, wherein said antigenic material obtained from a viral source other than the Lumpfish flavivirus described herein is from a virus selected from the group consisting of: Viral Hemorrhagic Septicemia Virus (VHSV), Viral Hemorrhagic Septicemia Virus (VHSV), Infectious Hematopoietic Necrosis virus (IHNV), Infectious Pancreatic Necrosis Virus (IPNV), Spring Viremia of Carp (SVC), Channel Catfish Virus (CCV), Infectious Salmon Anaemia virus (ISAV), pancreatic disease virus (SPDV), Iridovirus, and heart and skeletal muscle inflammation virus (HSMIV), parts or subunits of any one of these viruses, and combinations hereof. Representative species of such viruses are available to the skilled artisan, for instance from the following deposits: infectious pancreatic necrosis virus (IPNV, ATCC VR-1318, country of origin: unknown), Viral Hemorrhagic Septicemia Virus (VHSV, ATCC VR_1389, country of origin: Denmark); Infectious Hematopoietic Necrosis virus (IHNV, ATCC VR-1392, country of origin: USA)); Pancreatic Necrosis Virus; Spring Viremia of Carp (SVC, ATCC VR-1390, country of origin: Denmark); Channel Catfish Virus (CCV) (ATCC VR-665, country of origin: USA); Infectious Salmon Anaemia (ISA) virus (ATCC VR-1554, country of origin: Canada).

Patent deposits have previously been made by the present applicant of the following viral species: Heart and Skeletal Muscle Infection Virus (HSMIV, patent deposit nr ECACC 04050401, country of origin: Norway).

In more specific embodiments, said antigenic material obtained from a viral source other than the Lumpfish flavivirus as described herein is from the group consisting of: Glycoprotein of Viral Hemorrhagic Septicemia Virus (VHSV), nucleoprotein of Viral Hemorrhagic Septicemia Virus (VHSV), glycoprotein of Infectious Hematopoietic Necrosis virus (IHNV), nucleoprotein structural proteins of Infectious Pancreatic Necrosis Virus (IPNV), G protein of Spring Viremia of Carp (SVC), and a membrane-associated protein, tegumin or capsid protein or glycoprotein, of Channel Catfish Virus (CCV), antigenic fragments of any one of these proteins and combinations thereof.

In other embodiments said antigenic material from a parasitic source is from a source selected from the group consisting of *Lepeophtheirus Sp., Caligus Sp.,* and *Ichthyophthirius Sp,* parts of any one of these parasites, and combinations thereof.

In yet other embodiments said antigenic material is from a fungal source selected from the group consisting of *Saprolegnia Sp., Branchiomyces sanguinis, Branchiomyces demigrans* and *Icthyophonus hoferi.*

The vaccine according to the invention may in particular be formulated for administration to a fin fish. More specifically the vaccine may be (formulated) for administration to a telostei. The teleostei include, but are not limited to salmonids, lumpfish, wrasse, basses, breams, cods, snappers, flatfish, catfish, yellowtails and tilapias.

In a presently preferred embodiment the vaccine is formulated for administration to lumpfish (*Cyclopterus lumpus*)

In further embodiments of the invention the vaccine is formulated for administration by a route selected from the group consisting of: Bath, immersion, intraperitoneal injection, intramuscular injection and oral administration.

### Live recombinant microorganism

According to another embodiment of the invention, the sequences of SEQ ID NO. 6, or variants or fragments thereof, may also be used to develop a live recombinant microorganism to be used in a vaccine against Lumpfish flavivirus infection. A live recombinant organism functions as a carrier for genetic information according to the present invention by the insertion of genetic material of the SEQ ID NO. 6, or variants or fragments thereof. Said sequence or variants or fragments thereof may be inserted in the microorganism in such a way that the microorganism is capable of expressing said nucleic acid sequences. The nucleic acid sequences or variants or fragments thereof according to the invention may be inserted in the genome of the microorganism or by the introduction of a vector comprising the desired nucleic acid sequences. The live recombinant microorganism may thus be a carrier of nucleic acid sequences coding for the recombinant proteins or variants or fragments thereof according to the present invention. As mentioned above, the live recombinant organism may e.g. carry one or more recombinant proteins according to the present invention on the surface, e.g. wherein said recombinant proteins are anchored in the membrane or the cell wall of the live recombinant organism.

The live recombinant microorganism may be e.g. a virus, a bacteria or a parasite. The live recombinant microorganism may function in the same way as a live fish vaccine, i.e. the microorganism may replicate in the target fish and result in an immunological response against the recombinant proteins coded by the nucleic acids of the invention.

The live recombinant microorganism may have the advantage of being efficiently cultured and thus being optimised for desired fermentation processes and high yield production of vaccine.

The skilled person will be able, by the use of common biotechnological techniques for cloning and constructions of recombinant organisms, to construct a live recombinant microorganism according to the present invention.

The live recombinant microorganism may also be the carrier of nucleic acid coding for other fish pathogens thus providing for protection against a variety of pathogens. The live recombinant organism may thus include nucleic acids coding e.g. coating proteins or fragments thereof of other fish viruses, such as e.g. SAV, IPNV, IHNV or HSMIV.

According to one embodiment of the invention, the live recombinant microorganism is a virus able to replicate in fish, preferably lumpfish. For example, IHNV may be used as live recombinant microorganism, c.f. WO 03/097090 reporting the use of a modified novirhabdovirus to obtain vaccines. The use of live recombinant microorganism as carrier for nucleic acids coding antigens is also suggested for other fish pathogens, e.g. in WO 2007/031572.

A vaccine composition comprising a live recombinant microorganism may further comprise adjuvants, carrier, surface-active compounds or emulsifiers, including as those agents described above in relation to recombinant vaccines. Furthermore, the advantage of combining different vaccine components is equally applicable to the live recombinant vaccine according to the present invention as to recombinant vaccine composition as disclosed above.

### DNA vaccine

According to one aspect of the invention, a DNA vaccine is provided comprising nucleic acids according to the present invention.

DNA vaccines are based on the administration of nucleic acids sequence(s) coding an antigen to a patient, wherein the expression of said nucleic acid(s) results in an immune response in said patient. For the purpose of the present invention, the patient is a fish. A DNA vaccine may consist of a naked plasmid DNA comprising a nucleic acid sequence coding the desired antigen of a pathogen operably linked to suitable promoter sequences and other suitable control sequences enabling the expression of said nucleic acid sequence by the cells of the patient after administration of the vaccine. A DNA vaccine may also be carried in a live host microorganism which is involved in delivering the genetic material to the patient.

Various DNA vaccines to combat fish pathogens are known. Heppell et al. reports of the protection against live virus after injection with a DNA vaccine comprising viral haemorrhagic septicaemia virus G and N genes cloned into an expression plasmid and injected into rainbow trout (Fish and Shellfish Immunology, 1998, vol 8, issue 4, pp 271-286). Lorenzen and LaPatra describes a DNA vaccine against a fish rabdhovirus in "DNA vaccines for aquacultured fish", Rev. sci.tech. Off int. Epiz., 2005 (1), 201-213. A DNA vaccine resulting in protective effects is further more reported by Mikalsen et al., 2005, "Protective effects of a DNA vaccine expressing the infectious salmon anaemia virus hemagglutinin esterase in Atlantic salmon", Vaccine, 23:30, pp 4895-4905.

In EP 1 818 406 A1, a DNA expression system that may be used to produce a DNA vaccine that prevents infectious disease in aquatic animals is described, wherein the expression is directed by a cytomegalovirus promoter.

WO 2007/31572 discloses inter alia a DNA vaccine useful to combat SAV infections in salmonid fish.

WO 2009/002376 discloses a DNA vaccine useful to combat viral infection in carp (Spring viremia of carp virus).

EP 2 011 876 regards the development of a DNA fish vaccine useful to combat KOI Herpes disease that affects carps.

Based on the prior art and the common general knowledge referred to above, the skilled person is able to construct a DNA vaccine comprising the SEQ ID NO. 6 of the present invention or fragments or variants thereof utilizing methods commonly used in the field of genetic engineering.

DNA vaccines consisting of naked plasmid comprising the SEQ ID NO. 6 or fragments or variant thereof may be administered to fish by intramuscular injection or particle mediated delivery by gene gun. The latter involves the coating of small gold particles with the DNA vaccine, and the administration is then effected by air-pressured mediated intradermal delivery. Intramuscular injection of a DNA vaccine according to the present invention is preferred as it is more cost effective than the gene gun method, cf. Lorenz and LaPatra, *supra.* Methods for the administration of DNA vaccines to fish is furthermore disclosed in "Intramuscular Injection of DNA vaccines in Fish" by Heppel and Davis in "DNA Vaccines: Methods and Protocols", Methods of Molecular Medicine, 2000, vol. 29 (ISBN 978-0-89603-508-5).

### Inactivated or attenuated vaccine

The isolated Lumpfish flavivirus of the invention may be used in the preparation of a vaccine, for example, in which the virus is in attenuated or inactivated form.

Various methods are known, including chemical or physical means for inactivation of viruses. Chemical inactivation may for instance be carried out by treatment of the virus with enzymes, with formaldehyde, β-propiolactone or ethyleneimine or a derivative thereof, with organic solvent (e.g. 30 halogenated hydrocarbon) and/or detergent, e.g. Triton® or Tween®. Physical inactivation can advantageously be carried out by subjecting the viruses to energy-rich radiation, such as UV light, gamma irradiation or X-rays.

### Antibodies and immunoassays

The recombinant proteins or isolated virus according to the present invention may be used to obtain antibodies capable of binding a Lumpfish flavivirus.

Another aspect of the invention thus relates to an antiserum or an isolated antibody or antibodies (monoclonal or polyclonal) which selectively binds to a Lumpfish flavivirus or to a component or part of said virus. Antiserum is conventionally obtained for instance by immunising a laboratory animal with the appropriate antigenic determinant. Once the concentration of antibodies in serum from the animal reaches a desired level, the animal is bled. The serum thus obtained should contain antibodies produced in response to the immunogenic stimulus.

Likewise, techniques for the preparation of antibodies are known to the skilled person. The techniques include the traditional hybridoma technology and alternative techniques such as mRNA display, ribosome display, phage display and covalent display.

In some embodiments of the invention the isolated antibody comprises a marker, e.g. a radiolabel, a fluorescent tag, a chemiluminescent label or an enzyme. The antibody may be a polyclonal or monoclonal antibody. Protocols for the labelling of antibodies by coupling of the various markers mentioned above are well known to the skilled person.

For sufficient protection and control of the disease caused by Lumpfish flavivirus, means for efficient diagnosis and detection of Lumpfish flavivirus is needed. The present invention therefore provides immunoassays, useful for the diagnosis and detection of Lumpfish flavivirus in fish. An immunoassay within the meaning of the present invention is a biochemical test that measures the presence and/or concentration of the Lumpfish flavivirus in a biological sample, such as e.g. serum. An immunoassay thus utilises the specific binding of an antibody to its antigen. Both the presence of antigens (e.g. present on the Lumpfish flavivirus) and the presence of antibodies against the Lumpfish flavivirus may be measured in the biological sample.

The present invention further provides an immunoassay for the detection of antibodies specific for Lumpfish flavivirus in a biological sample, comprising (a) contacting the recombinant proteins according to the present invention and a biological sample suspected to comprise antibodies against a Lumpfish flavivirus; and (b) determining whether the one or more antibodies binds to a Lumpfish flavivirus present in the biological sample wherein said binding represents an indication that the sample contains antibodies specific for Lumpfish flavivirus.

According to another aspect of the invention, an immunoassay for the detection of Lumpfish flavivirus in a biological sample is provided, comprising (a) contacting one or more antibodies that specifically recognise and bind to a recombinant protein according to the invention and a biological sample suspected to comprise Lumpfish flavivirus; and (b) determining whether the antibody binds to Lumpfish flavivirus present in the biological sample wherein said binding represent an indication that the sample contains a Lumpfish flavivirus.

The complex formed in the immunoassays according to the present invention, e.g. the binding of an antibody to a Lumpfish flavivirus present in a biological sample or the binding of an antibody present in a biological sample to a recombinant protein or fragment thereof according to the present invention, may be determined by various means known to the skilled person such as, for example, densitometry, fluorimetry, or colorimetry.

The immunoassays of the invention as described above may be a standard Enzyme-linked immunosorbent assay (ELISA). The skilled person will be able to provide ELISA protocols using antibodies developed against the recombinant proteins according to the present invention, or the recombinant proteins. Reference is in this respect made to the ELISA Guidebook, Methods in Molecular biology, vol. 149, ed. By John R Crowther, Humana Press, 2001.

The present invention also provides a diagnostic kit for carrying out the immunoassays according to the present invention. According to one embodiment, the kit according to the invention comprises antibodies recognizing a recombinant protein according to the invention and usual means for carrying out an immunoassay, such as e.g. an ELISA plate, and a reagent capable of producing a detectable colour change upon the binding of the antibodies to a Lumpfish flavivirus in a biological sample to be tested. According to another embodiment, a kit is provided comprising one or more recombinant proteins according to the invention and usual means for carrying out an immunoassay, such as e.g. an ELISA plate, and a reagent capable of producing a detectable colour change upon the binding of said recombinant proteins to antibodies in a biological sample to be tested.

### Primer sequences and method of detection of Lumpfish flavivirus infection

The nucleic acid sequences of the present invention may be used to detect the presence of Lumpfish flavivirus in fish. The present invention therefore also relates to a diagnostic method wherein primers specifically hybridize to a nucleic acid in a test sample that is similar to the nucleic acid sequences of the invention. Such tests may take the form of a PCR test or a hybridization test without amplification.

The detection of nucleic acids present in a biological sample is widely applied in both human and veterinary diagnosis, wherein nucleic acids from e.g. pathogens present in biological samples are isolated and hybridized to one or more primers. The one or more primers to be used in hybridization based detection methods is constructed so that they are able to specifically bind to a nucleic acid sequence if present in the sample to be tested. The primers may be linked to a signalling unit or probe, such as e.g. a radiolabel, a luminescent molecule, or a fluorescent molecule, enabling the visualisation of the binding of the primers to a target sequence.

A primer as used herein refers to a nucleic acid sequence, which can be used as a probe in a method of detection of the presence of Lumpfish flavivirus in fish. The primers according to the present invention consist of at least 10, preferably at least 15, and more preferably at least 20 nucleotides that are able to hybridize to nucleic acid sequences isolated from a Lumpfish flavivirus.

The primer according to the present invention is able to hybridize to another nucleic acid molecule, such as genomic RNA originating from Lumpfish flavivirus or cDNA prepared from said genomic RNA, under appropriate conditions of temperature and solution ionic strength, cf. e.g. Sambrook et al., Molecular Cloning: A laboratory Manual (third edition), 2001, CSHL Press, (ISBN 978-087969577-4). The condition of temperature and ionic strength determine what the skilled person will recognise as the "stringency" of the hybridization. The suitable stringency for hybridisation of a primer to target nucleic acids depends on inter alia the length of the primer and the degree of complementation, variables well known to the skilled person. For example, high stringency conditions may comprise hybridization in a buffer consisting of or comprising 6 X SSC, 50 mM Tris-HCL (pH=7.5), 1 mM EDTA, 0.02% PVP, 0.02% Ficoll, 0.02% BSA and 100 µg/ml denatured salmon sperm DNA, for 48 hours at 65°C, washing in a buffer consisting of 2 X SSC, 25 0.01% PVP, 0.01% Ficoll, 0.01% BSA for 45 minutes at 37°C, and washing in a buffer consisting of 0.1 X SSC, for 45 minutes at 50°C. A minimum length of a hybridisable primer is usually at least about 10 nucleotides, preferably about 15 nucleotides and more preferably about 20 nucleotides.

Various protocols for the detection of nucleic acid sequences by PCR are available to the skilled person; cf. e.g. Methods in Molecular Biology, vol. 226, PCR protocols, second ed., Ed. Barlett and Stirling, Humana Press, 2003. The skilled person will furthermore, with the knowledge of the genomic Lumpfish flavivirus sequence depicted in figure 1 (SEQ ID NO. 1) be able to provide suitable primers for the purpose of using said primers in a method for detection of the presence of Lumpfish flavivirus according to the present invention. Reference is in this respect made to the textbook PCR Primer Design, vol. 402 of the series Methods in Molecular Biology, Ed. A. Yuryev, 2007, Humana Press.

According to one embodiment of the invention, the following primers are provided:

**Table 1**

| |
|---|
| 5'- CCTCCATTCCCAAGGAAAAT-'3 (SEQ ID NO. 8) |
| 5'- GGTCCTGCGATGTGATCTTT -'3 (SEQ ID NO. 9) |
| 5'- GAGAACAGCCAGCAATGACA -'3 (SEQ ID NO. 10) |
| 5'- TTCGAGTGTGATGAGCAAGC -'3 (SEQ ID NO. 11) |

### EXAMPLES

### Example 1

### Identification of viral sequences.

Lumpfish in a culture facility were showing signs of disease and dying. Samples were collected for histopathological analysis. Several organs were collected, and the specific histopathological change that was observed was necrosis in the liver.

Livers from the diseased fish were homogenized, centrifuged at 3000xg and passed through a 0.22 µm filter. The homogenate was digested with approximately 250U DNase l/ml and 5µg RNase A/ml in RDD-buffer (included with Qiagen DNase I) for 1h at 37°C.

RNA was prepared from the sample using QIAamp viral kit (Qiagen) according to the manufacturer's instructions. Single stranded cDNA was synthesized using SuperScript® III reverse transcriptase kit (Invitrogen) and random primers with a fixed 5' overhang (FR26RVN primer 5' GCC GGA GCT CTG CAG ATA TCN NNN NN 3'; Djikeng et al., BMC Genomics 2008, 9:5). The RNA was incubated at 95°C prior to cDNA synthesis to avoid problems caused by a possible secondary structure of the RNA. After synthesis the RNA strand was digested using RNase H before double stranded DNA was made using a combination of the 2^{nd} strand buffer components of the Universal RiboClone® cDNA Synthesis System (Promega) and Exo-Klenow polymerase and including additional supply of the random primers. A single primer PCR reaction using primer against the random primer fixed overhang (FR20RV 5' GCC GGA GCT CTG CAG ATA TC 3'; Djikeng et al., BMC Genomics 2008, 9:5) and standard *Taq* polymerase (Invitrogen) was performed (initial denature of cDNA 95°C for 5 minutes, 40 cycles of 94°C for 30 seconds, primer annealing 60°C for 15 seconds and synthesis 72°C for 1 minute and then final synthesis 72°C for 10 minutes), and the resulting products were separated by 1% agarose gel electrophoresis. PCR products in the size range 500 - 1000 bp were excised from the gel in one piece, purified and cloned using the TOPO-TA cloning system (Invitrogen). Approximately 300 clones were sequenced and subjected to analysis.

Sequences were subjected to analysis in the ContigExpress program of Vector NTI software, resulting in identification of different sequences. The complete genome sequence was obtained by PCR amplification of genome sequence located between the initially obtained sequences, as well as 3' and 5' RACE using standard procedures.

Only weak homology with known virus sequences was detected. Finally, a genome of 10779 bp was assembled (as set forth in SEQ ID NO.1), containing two open reading frames. This is the first time a putative member of the *Flaviviridae* is identified in a teleost.

The analysis of SEQ ID NO. 1, as described, revealed two open reading frames which are believed to include the coding region for several viral proteins. Sequence comparison with other known viral sequences revealed that the open reading frame 1 (SEQ ID NO. 2) according to the present invention shows very weak homology to other viral sequences. However, certain putative protein motives can be identified, and an organisation corresponding to the common outline of Flaviviridae is suggested. Furthermore, the analysis of the open reading frame 1 (SEQ ID NO. 2) indicates that it comprises a nucleic acid sequence coding an envelope protein (SEQ ID NO. 6).

### Example 2

### Expression and use of recombinant proteins

Gene sequences from SEQ ID NO. 6 were amplified by PCR and cloned into a pET vector system using standard methods readily known by one of skill in the art. An *E.coli* host cell was transformed, and recombinant protein expressed. Recombinant protein was purified using standard techniques. Additionally, SEQ ID NO. 6 was amplified by PCR and cloned into a eukaryotic vector. A eukaryotic host cell was transformed and recombinant protein expressed.

Recombinant proteins, as described herein, were formulated into a suitable formulation, preferably but not limited to, a water-in-oil emulsions and will be used as a formulation to produce an immune response in a lumpfish. Any variants related to SEQ ID 6. that can be construed as having conservative amino acid substitutions are included in this description. Additionally, any open reading frames expressing proteins from a lumpfish flavivirus, or any variant of a lumpfish flavirus having about 70% sequence identity to the open reading frames described herein, may be used as part of a formulation to produce and immune response.

Additionally, rabbits and mice will be immunized with recombinant proteins cloned and expressed, as described herein. The antibody responses against the proteins will be assessed at various time points. Serum from the immunized animals will be tested in virus neutralization assays and used for additional *in vitro* assays.

### Example 3

### Vaccination and challenge of lumpfish with a recombinant lumpfish flavivirus protein

The effect of a vaccine based on the recombinant putative envelope protein (SEQ ID NO.6) from Lumpfish flavivirus will be assessed.

Lumpfish will be vaccinated by intraperitoneal injection with a vaccine comprising recombinant putative Lumpfish flavivirus envelope protein, SEQ ID NO.6, or variants thereof. After an immunization period, vaccine efficacy will be tested through i.p. challenge of vaccinated and unvaccinated lumpfish with Lumpfish flavivirus (LFV). Tissue samples from vaccinated and unvaccinated fish will be collected for histopathological evaluation. Samples will also analyzed for the presence of virus by PCR-screening and other methods.

Mortality will be registered daily and tissue samples will be collected at different time points from the vaccinated and unvaccinated groups for histopathological evaluation, measurement of antibody response and PCR-screening for the presence of virus.

### Example 4

### DNA vaccine protecting against infection with Lumpfish flavivirus

SEQ ID NO. 6 and parts, fragments, and variants thereof, will be cloned into a vaccine vector suitable to be used as part of a DNA vaccine. The resulting vector will be injected intramuscularly into lumpfish. After an immunization period, vaccine efficacy will be tested through i.p. challenge of vaccinated and unvaccinated lumpfish with Lumpfish flavivirus. Tissue samples from vaccinated and unvaccinated fish will be collected for histopathological evaluation. Samples will also be analyzed for the presence of virus by PCR-screening and other methods.

### Example 4

### Testing the infectivity of LFV

The aim of this study was to reproduce disease in Lumpfish by experimental infection to investigate if Lumpfish virus (LFV) could be transmitted horizontally by infected Lumpfish to naïve Lumpfish by cohabitation, and to investigate whether the virus could be transmitted to Atlantic salmon by cohabiting with infected Lumpfish.

### Study design

This was a comparative, randomized, double tank laboratory study. The study period lasted 4 weeks. The fish were acclimatized to the conditions to be used in the study prior to start. Below is a table of characteristics of the fish (Table 2).

**Table 2**

| **Species** | **Lumpfish (*Cyclopterus lumpus)*** | **Atlantic salmon (*Salmo sa*/*ar*)** |
|---|---|---|
| Strain | Fjordforsk AS | Stofnfiskur Iceland |
| Origin | Fjordforsk AS | Egghouse Vogavik, Iceland |
| Batch no. | - | ILAB_15_500 |
| Number of fish | 160 (incl. 10 fish for baseline | 30 (incl. 10 fish for baseline |
| Size | 16.8 grams in average at challenge | 94.3 grams in average at challenge |
| Vaccination status | Unvaccinated | Unvaccinated |
| Disease history | None | None |

The study fish were unvaccinated and were free from disease. The Atlantic salmon were already smoltified by ILAB, in order to be ready for cohabiting with infected Lumpfish. Head kidney samples from 15 Lumpfish were screened for the presence of atypical *A. salmonicida, Pasteurella* sp. and Lumpfish Flavivirus upon introduction to the facility. All samples were negative for all the investigated disease agents.

The fish were acclimatized for minimum 1 week to the conditions to be used in the study period (12 °C, 34 % salinity sea water, 12:12 light:darkness). The fish were kept in two 150 L tanks during the challenge period. Environmental parameters during the study period are described in Table 3

**Table 3**

| **Week** | **Salinity** | **Temp. °C** | **O₂ (%)** | **Tank size / density** | **Photoperiod (L:D)** |
|---|---|---|---|---|---|
| 41-45 | 34 ‰ | 12±1°C | 70-100 | 150 L | 12:12 |

### Challenge

### Challenge isolate

The challenge material ALV 1224 consisted of tissue homogenate from Lumpfish originating from a field outbreak of LFV. Histopathological examination revealed changes dominated by acute and chronic inflammation in the liver, necrosis and loss of normal tissue structure of the kidney, loss of normal tissue structure in the spleen, and changes in the heart. The fish were strongly positive for LFV in all investigated organs (gills, brain, heart, skin, liver, spleen, kidney, intestines) with Ct values averaging 10-14. The liver, spleen and kidney were aseptically extracted from the infected fish. Liver from nine fish were pooled, and spleen plus kidney from the same nine fish were pooled, homogenized in L-15 medium with gentamicin, cell debris removed by centrifugation, and the supernatant filtered through a 0.45 µm filter. The resulting challenge material was strongly positive for LFV, with Ct-values of 17 and 18 from liver and head-kidney/spleen respectively. However, when RNAse treating the samples, the Ct values increased to 30 and 23 respectively, indicating that the majority of intact (and therefore probably infectious) viral particles can be found in the kidney/spleen rather than the liver.

### Challenge procedure

The Lumpfish were marked by VIE-tagging at the time of marking, according to Table 4. 3x1 ml ampulles of spleen/kidney and liver homogenate each was thawed on ice, and pooled prior to challenge. Disposable syringes were used to infect 50 Lumpfish by IP injection of 0.05 ml challenge material to each tank. To C6T7, 50 uninfected Lumpfish were added to be infected by cohabitation, while 20 uninfected Atlantic salmon were added to C6T8. These were to be infected by cohabitation.

**Table 4**

| Challenge study design | | | | | |
|---|---|---|---|---|---|
| **Species** | **Marking** | **Challenge mode** | **Cell** | **Tank** | **No. of fish** |
| Lumpfish | 2x Orange | IP | C6 | 7 | 50 |
| Lumpfish | 2x Yellow* | Cohabitation | C6 | 7 | 50 |
| Lumpfish | 2x Red | IP | C6 | 8 | 50 |
| Atlantic salmon | - | Cohabitation | C6 | 8 | 20 |
| | | | | **Total** | **170** |

### Collection of tissue samples for Reverse Transcriptase quantitative PCR

The fish were euthanized prior to collection of tissue samples. Kidney tissue was collected on RNAlater from minimum ten (10) unvaccinated fish prior to challenge. The collected kidney tissue was screened by Reverse Transcriptase quantitative-PCR (RT-qPCR) to confirm absence of Lumpfish Flavivirus in the test population prior to vaccination. Liver, heart, gill and kidney tissue was collected on RNAlater from 10 Lumpfish from C6T8 the day after challenge, in order to determine the quantity of LFV in the respective tissues as a consequence of injection of the challenge material. Also, liver, heart, gill and kidney tissue samples was collected on RNAlater from 10 fish per group at one, two, three and four weeks post challenge for C6T7, and at two and four weeks post challenge for C6T8. The samples were analysed.

### Collection of tissue samples for histopathology

Tissue samples (liver, kidney, spleen and heart) were collected from ten (10) Lumpfish and Atlantic salmon on formalin for histopathological evaluation prior to challenge. Also, tissue samples were collected from ten (10) fish per group at one, two, three and four weeks post challenge for C6T7, and at two and four weeks post challenge for C6T8. Histopathological changes developing after challenge were investigated for a selection of groups and time points after evaluating the results obtained by RT-qPCR:
- Baseline samples of ten Lumpfish prior to challenge
- ten IP injected Lumpfish from C6T7 at 21 dpc
- ten IP injected Lumpfish from C6T7 at 31 dpc

The scoring was performed blind by a third-party assessor in order to evaluate the degree of histopathological changes developing in the respective tissue as a consequence of infection with LFV.

### Results- Prevalence of LFV in Fish Post Challenge

### IP injected Lumpfish

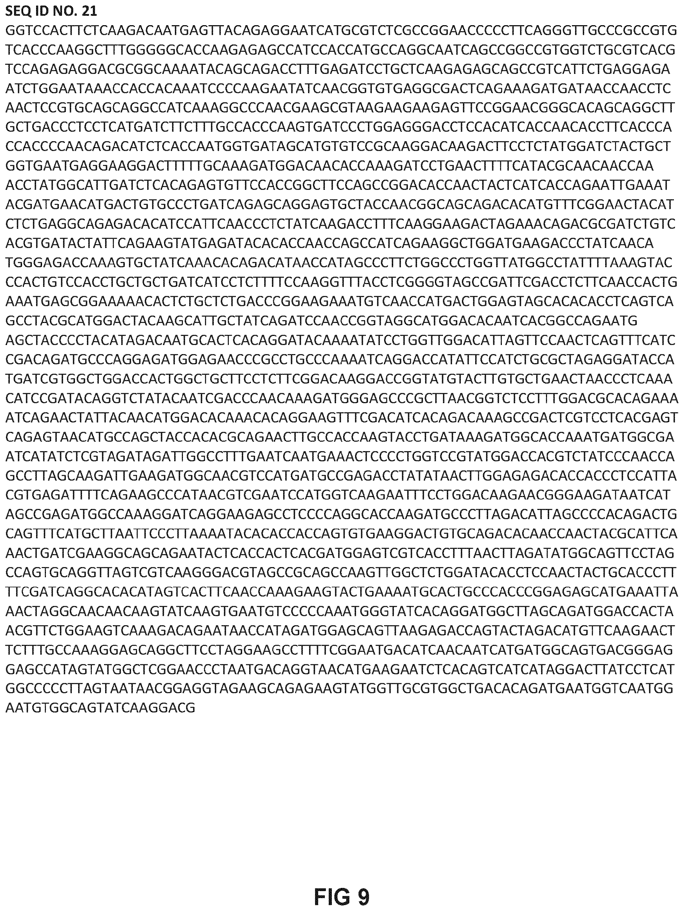

The prevalence of LFV in IP injected Lumpfish increased throughout the study. The results are shown in Figure 9A and 9B. No fish were positive for LFV at introduction to the study facility, one day post challenge (dpc) or 7 dpc. However, at 14 dpc, 4/10 fish were positive for LFV in kidney, heart and gills, and 5/10 fish were positive for LFV in liver. At 21 dpc 10/10 fish were positive for LFV in kidney and heart. At 31 dpc, 9/10 fish were positive for LFV in kidney, heart and liver.

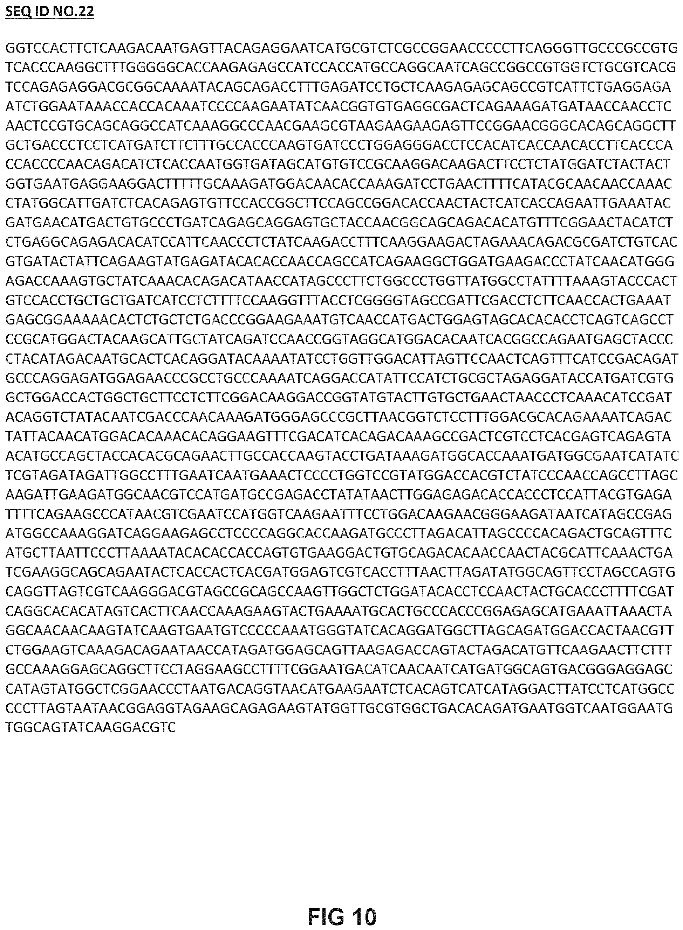

The quantity of LFV in IP-infected Lumpfish increased from 14 dpc to 21 dpc. However, there was no apparent change in the quantity of LFV from 21 to 31 dpc. The quantity of LFV in kidney samples was investigated from 10 Lumpfish from C6T8 at 14 dpc and at termination at 31 dpc, in order to investigate if there were any relevant differences between the two tanks. As can be seen in Figure 10, there is a slightly higher prevalence of LFV-positive Lumpfish at 14 dpc in C6T8 compared to C6T7 (8/10 compared to 4/10). The Lumpfish in C6T8 have been cohabiting with Atlantic salmon, with expected higher stress levels for these fish compared to C6T7. This might be the reason for a higher prevalence. At 31 dpc, there were no relevant differences between the two challenge tanks with regards to the prevalence of LFV-positive Lumpfish (9/10 vs 10/10).

### Cohabiting Lumpfish

None of the Lumpfish cohabitants were positive for LFV (kidney) when investigated at 14 dpc, Figure 9. However, 10/10 Lumpfish were positive for LFV (kidney and heart) when investigated at 21 dpc showing that LFV can be horizontally transmitted from IP injected Lumpfish to naïve Lumpfish. At 31 dpc, the prevalence of LFV positive fish had decreased to 4/10 fish (kidney and heart).

### Cohabiting Atlantic salmon

Atlantic salmon were investigated for the presence of LFV in kidney, in order to investigate whether LFV could be horizontally transmitted from IP infected Lumpfish. At 14 dpi, 0/10 Atlantic salmon were positive for LFV by PCR. 1/9 Atlantic salmon sampled at 31 dpc was positive for LFV in the kidney. However, as this sample was negative when re-analyzed, and gill, liver and heart samples from the same fish were all negative, it is apparent that this was a false positive sample.

### Histopathological changes developing post challenge with LFV

The histopathological changes developing after IP infection with LFV-containing tissue homogenate was investigated at 21 and 31 dpc (Table 5 below). Kidney, heart, liver and spleen were investigated. The most interesting findings were in the liver. 4/10 fish exhibited severe histopathological changes of the liver, dominated by diffuse necrosis at 21 dpc. This is characteristic for what is observed in field outbreaks of LFV. In addition, 2/10 fish exhibited mild changes, while 4/10 were negative. Only minor histpathological changes were detected in the kidney and heart at 21 dpc. While the spleen showed some histopathology, this might
not be related to the disease, as the spleen is a difficult organ to evaluate. Investigation of 10 Lumpfish prior to challenge showed the absence of necrotic or inflammatory changes in the liver prior to infection with LFV. However, minor changes were observed in the spleen and kidney, which might be related to the sampling procedure or other factors not related to LFV.

At 31 dpc, similar histopathological changes were observed as at 21 dpc. Severe necrosis of the liver was observed in 6/10 fish IP injected Lumpfish. Also, inflammatory changes were observed, and infiltration of connective tissue. No significant changes were detected for the Lumpfish infected by cohabitation sampled at 31 dpc.

The relationship between the Ct value of LFV in kidney tissue and the histopathological changes observed in the different organs was investigated by non-parametric Spearman correlation. A strong correlation was observed between histopathological changes in liver and the LFV tissue burden in kidney at 21 dpc (Spearman r -0.9342, p<0.0001) and 31 dpc (-0.8528, p-value <0.0001). For the other tissues, no significant correlation was observed between LFV kidney burden and the histopathological changes in the respective organs.

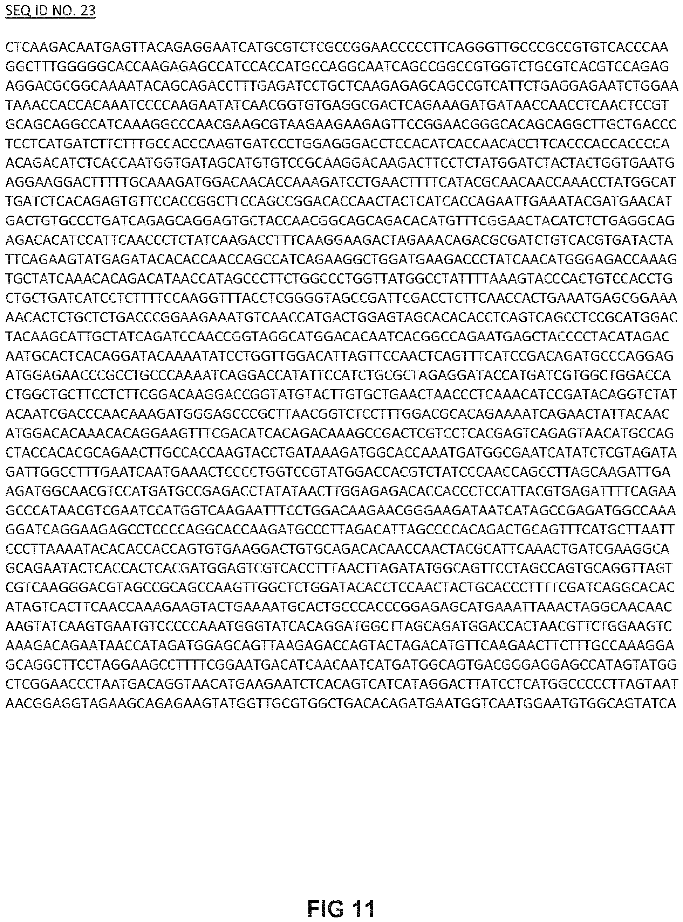

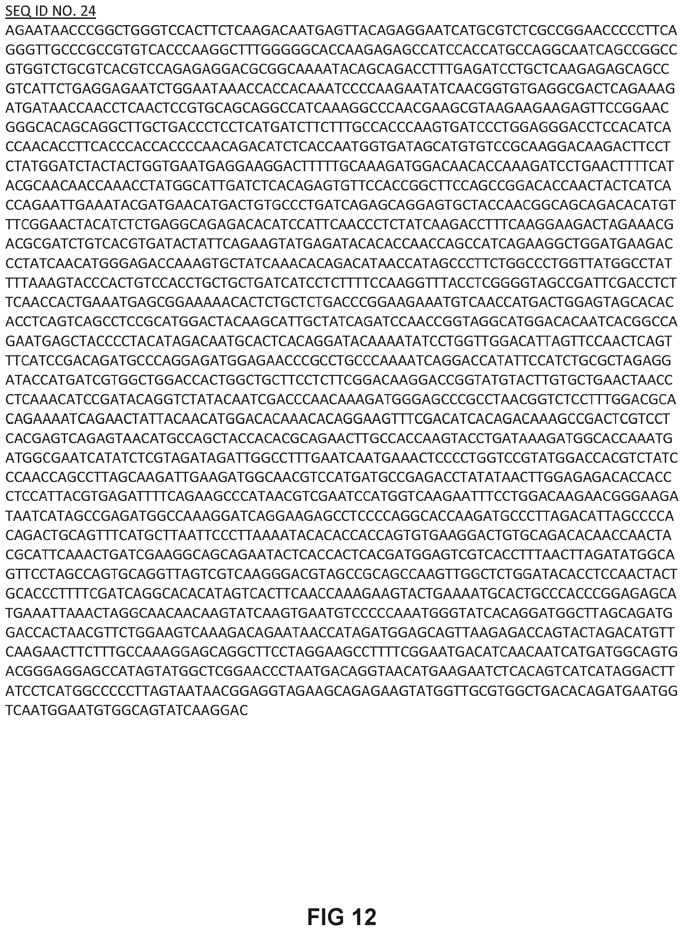

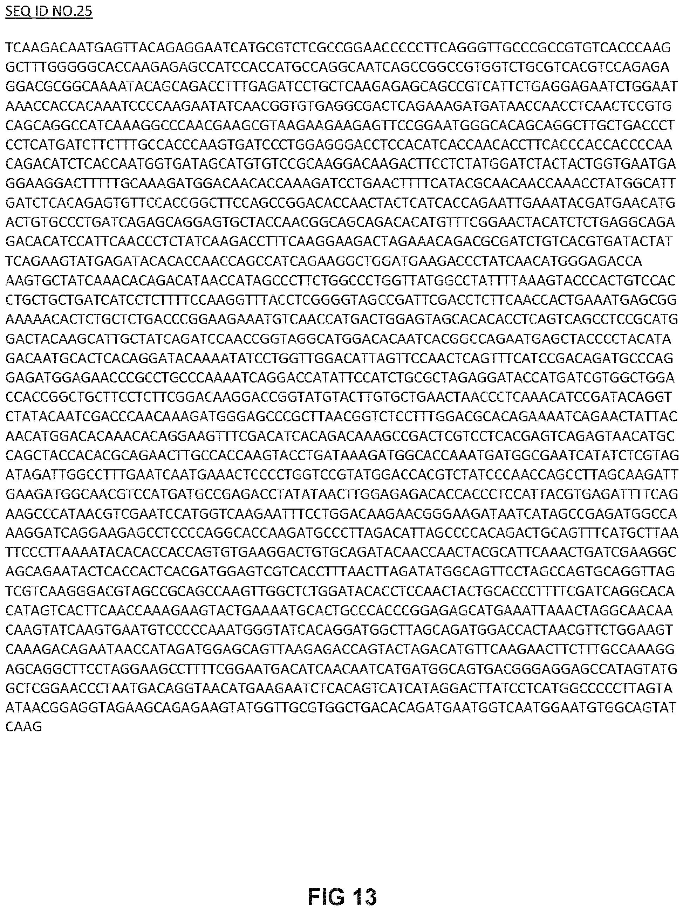

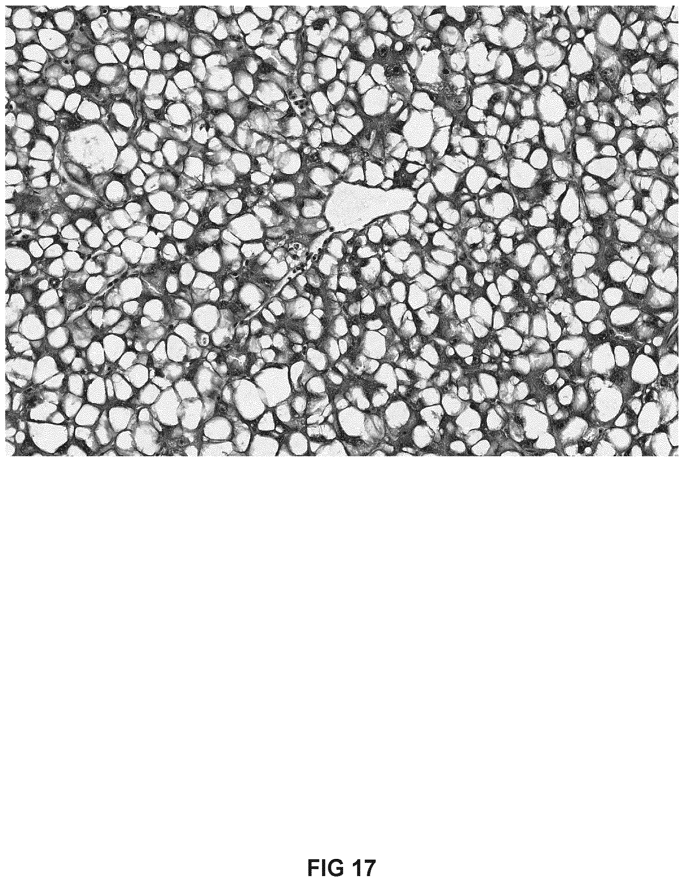

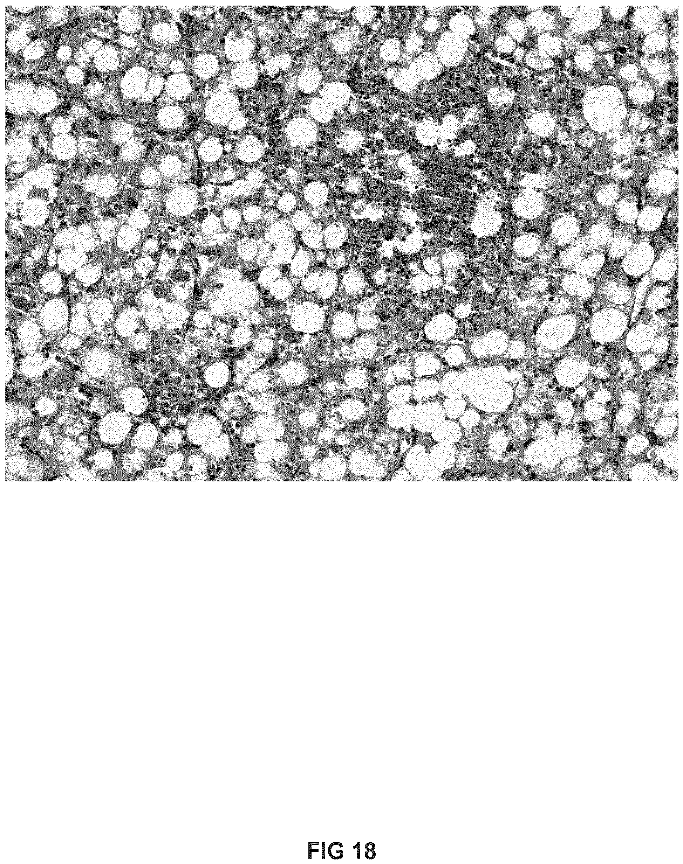

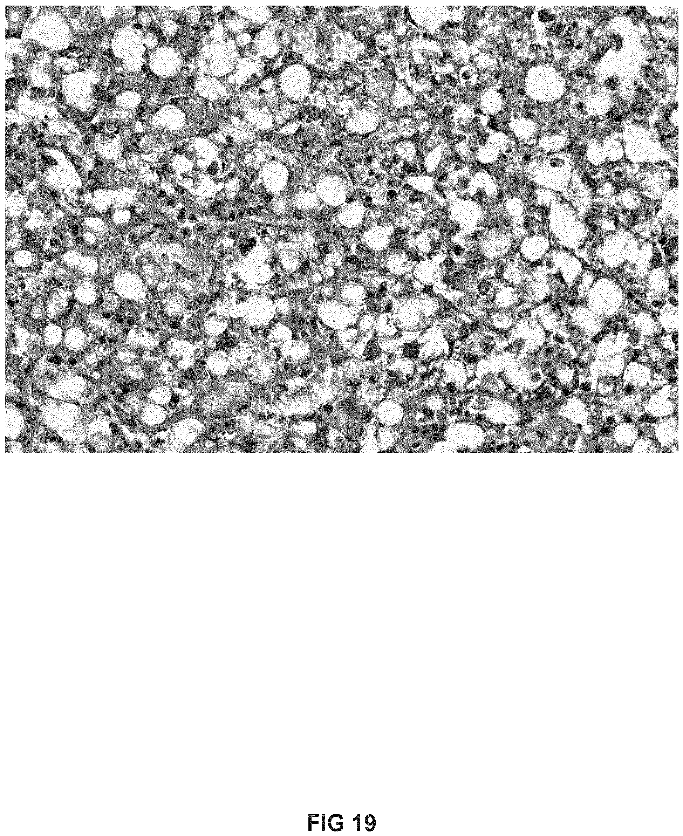

As can be seen in Figures 11-13, there is significant histological differences between the normal liver from a control fish (Fig 17), the liver having extensive tissue damage sampled at 21 days post IP challenge with LFV containing tissue homogenate (Fig 18), and a liver with extensive tissue damage sampled at 31 days post IP challenge with LFV containing tissue homogenate (Fig 19)

**Table 5**

| Histopathology of Lumpfish IP injected with LFV-containing tissue homogenate (C6T7) | | | | | | | | | | |
|---|---|---|---|---|---|---|---|---|---|---|
| Fish **no.** | **21 dpc** | | | | | **31 dpc** | | | | |
| | **Ct kidney** | **Kidney** | **Heart** | **Liver** | **Spleen** | **Ct kidney** | **Kidney** | **Heart** | **Liver** | **Spleen** |
| **1** | 11.03 | 0.5 | 0.0 | 1.0 | 0.0 | 34.60 | 0.0 | 0.0 | 0.0 | 1.0 |
| **2** | 9.70 | 0.5 | 0.5 | 3.0 | 0.0 | 14.51 | 0.0 | 0.0 | 3.0 | 1.0 |
| **3** | 12.44 | 0.0 | 0.0 | 1.0 | 0.0 | 11.90 | 1.0 | 0.0 | 3.0 | 1.0 |
| **4** | 10.75 | 1.0 | 0.0 | 3.0 | 2.0 | 12.19 | 1.0 | 0.0 | 3.0 | 3.0 |
| **5** | 23.41 | 0.5 | 0.5 | 0.0 | 2.0 | 23.45 | 1.0 | 0.0 | 0.0 | 1.0 |
| **6** | 21.77 | 0.0 | 0.0 | 0.0 | 2.0 | 21.56 | 2.0 | 1.0 | 0.0 | 1.0 |
| **7** | 10.38 | 0.5 | 0.0 | 3.0 | 3.0 | 12.76 | 1.0 | 0.0 | 3.0 | 3.0 |
| **8** | 19.55 | 0.0 | 0.0 | 0.0 | 2.0 | 22.51 | 0.0 | 0.0 | 0.0 | 3.0 |
| **9** | 13.96 | 0.5 | 0.0 | 0.0 | 2.0 | 10.97 | 1.5 | 0.0 | 3.0 | 3.0 |
| **10** | 8.66 | 0.0 | 0.0 | 3.0 | 2.0 | 11.50 | 1.0 | 0.0 | 3.0 | 2.0 |
| **Mean** | | 0.35 | 0.1 | 1.4 | 1.5 | | 0.85 | 0.1 | 1.8 | 1.9 |
| **Spearman r** | | - | 0.08 | - | 0.097 | | - | 0.17 | - | - |
| ***p*-value** | | 0.4397 | 0.40 | <0.0 | 0.797 | | 0.0917 | 0.20 | <0.0 | 0.114 |

### Weight at challenge

The mean weight at challenge was 17.8 and 94.3 grams for Lumpfish and Atlantic salmon respectively. The results are given in Table 6.

**Table 6**

| Weight at challenge | | | |
|---|---|---|---|
| **Species** | **n** | **Mean weight (± 1 SD)** | **95 % CI of mean** |
| Lumpfish | 30 | 17.8 ± 5.3 g | 16.8 - 18.8 g |
| Atlantic salmon | 30 | 94.3 ± 12.6 g | 92.0 - 96.6 g |

The described study showed that Lumpfish injected IP with tissue-homogenate containing LFV reproducibly developed characteristic signs of disease caused by LFV. Strong replication of LFV in IP injected Lumpfish was verified, as a function of time post challenge. Also, a clear correlation between the amount of LFV and the degree of histopathological damage to the liver was shown, verifying that the tissue damage observed was caused by LFV. To further support this proof, ten Lumpfish were shown to be negative for potential differential diagnoses (*Nucleospora cyclopteri*).

When non-challenged Lumpfish were cohabited with Lumpfish IP injected with LFV-containing tissue-homogenate, the virus was efficiently transmitted to the naíive Lumpfish, verifying that the virus can be transmitted horizontally to Lumpfish by natural exposure. No evidence of transmission of LFV to Atlantic salmon was observed throughout the study, indicating that LFV is unlikely to be able to be transmitted to Atlantic salmon by cohabiting with infected Lumpfish.

### Conclusion

LFV has been demonstrated that it can cause disease in Lumpfish upon IP challenge, and can further be transmitted horizontally to naïve Lumpfish by natural exposure through cohabitation. Additionally, there are no indications that LFV can be transmitted to Atlantic salmon by cohabiting with infected Lumpfish.

### Example 5

### PCR Diagnostics and Analysis of Lumpfish flavirus (LFV) Structural Region Diversity Between Isolated Strains

The following primers described in Table 7, below, have been tested and found suitable for PCR diagnostics and identification of the Lumpfish flavivirus (LFV).

**Table 7**

| **PRIMER** | **FORWARD (5' - 3')** | **REVERSE (5' -** 3') |
|---|---|---|
| POL | 5'-CCTCCATTCCCAAGGAAAAT-'3 (SEQ ID NO.8) | 5'- GGTCCTGCGATGTGATCTTT-'3 (SEQ ID NO.9) |
| FRAME | 5'- GAGAACAGCCAGCAATGACA-'3 (SEQ ID NO.10) | 5'- TTCGAGTGTGATGAGCAAGC-'3 (SEQ ID NO.11) |
| LFV 1 | 5'-ACATACTTAGAAGAATAACCCGG-'3 (SEQ ID NO12) | 5'-TGCCGTTGGTAGCACTCCT-'3 (SEQ ID NO.13) |
| LFV 2 | 5'-TACGCAACAACCAAACCTATGG-'3 (SEQ ID NO.14) | 5'-GCAGATGGAATATGGTCCTG-'3 (SEQ ID NO.15) |
| LFV 3 | 5'-GACAATGCACTCACAGGATAC-'3 (SEQ ID NO.16) | 5'-GCATGAAACTGCAGTCTGTG-'3 (SEQ ID NO.17) |
| LFV 4 | 5'-TCAGAAGCCCATAACGTCGA-'3 (SEQ ID NO.18) | 5'-GATGAATCGAACATGACGTCC-'3 (SEQ ID NO.19) |

The study, as described herein, examined the diversity in field isolates of LFV. Variations of nucleotide sequences of the region coding for the structural proteins of five different LFV strains isolated is described. Fourteen samples of LFV were isolated from five different sites, as outlined in Table 8, below.

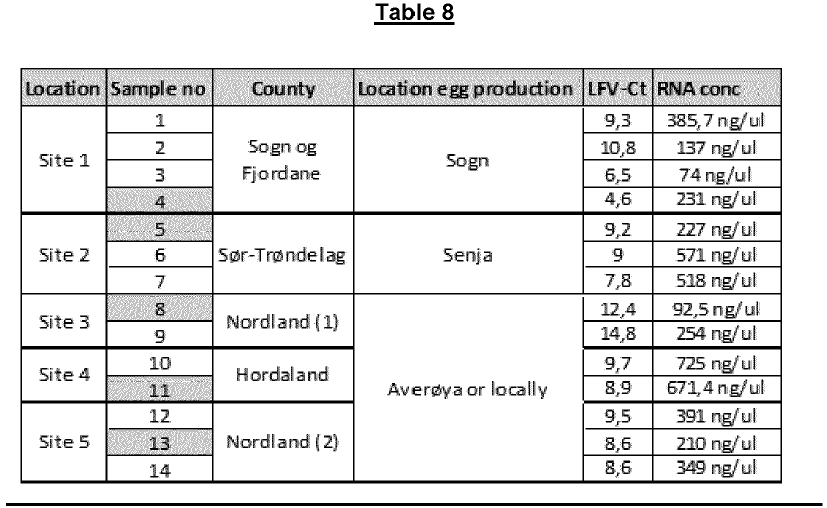

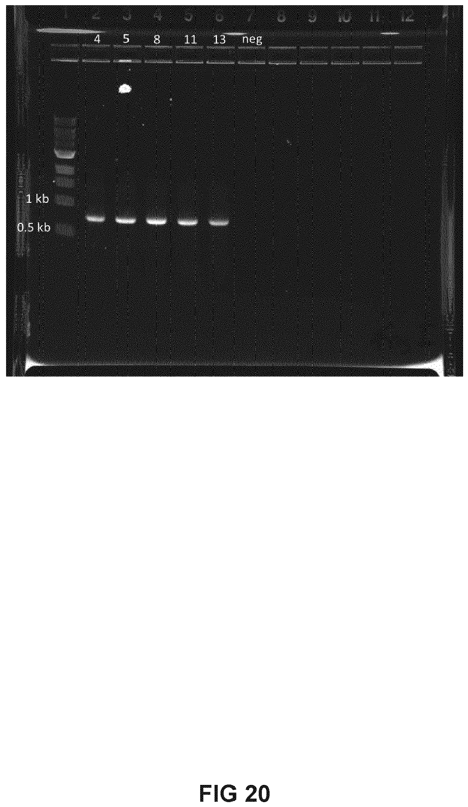

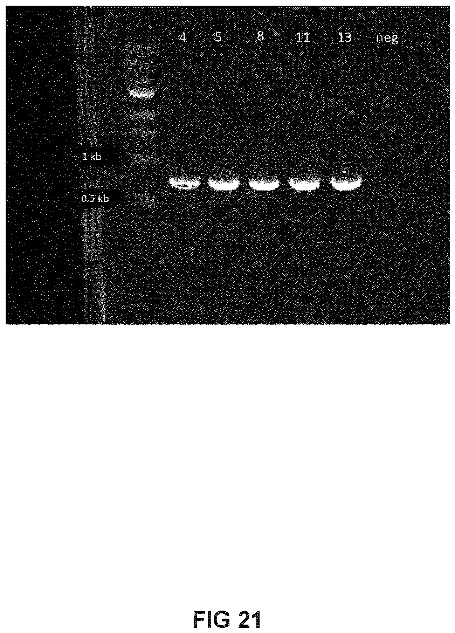

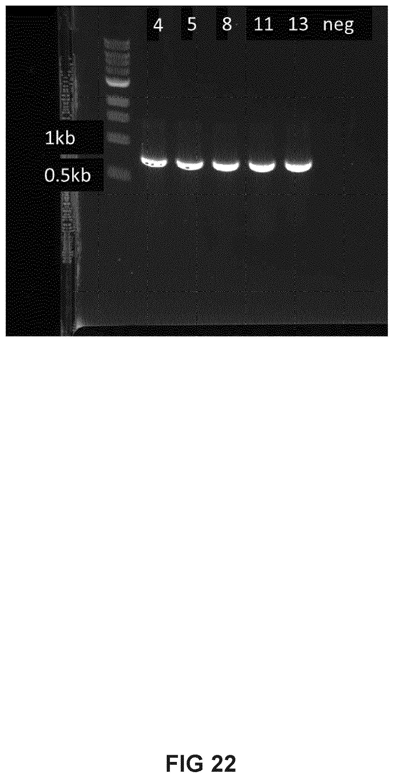

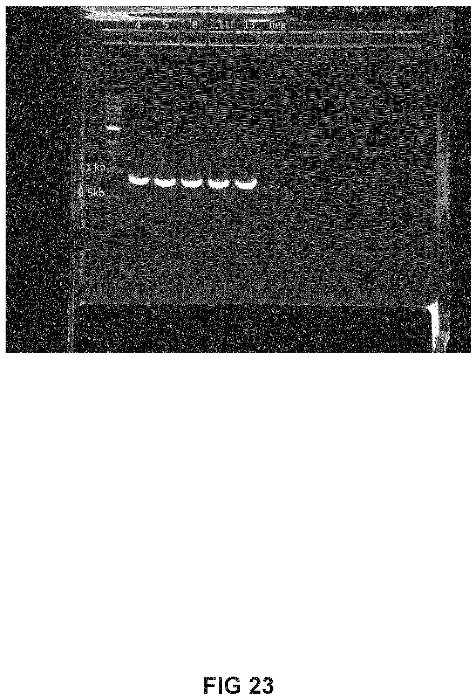

RNA was isolated and cDNA synthesis was performed using First Strand cDNA Synthesis (Invitrogen). PCR using Hot Start Taq DNA Polymerase (Qiagen) was performed with primers (described in Table 7 above) that were designed to cover the structural proteins of the LFV with 4 fragments overlapping each other. cDNA samples from fish numbers 4, 5, 8, 11 and 13 were used for analysis. The PCR products were visualized on an ethidium bromide stained 1.2% agarose gel, which confirmed the correct approximately 750 bp sized fragments. Figures 20-23 show the PCR product using the LFV1-4 forward and reverse primers.

The PCR fragments were isolated and sequenced. The sequence data was analyzed and fragments 1-4 from each fish was assembled and the five different virus strains were aligned.

### Results

The variation at nucleotide level in the 2577 nt coding for the structural proteins of LFV is generally low. Table 9 summarizes the number of nucleotide differences between the different strains.

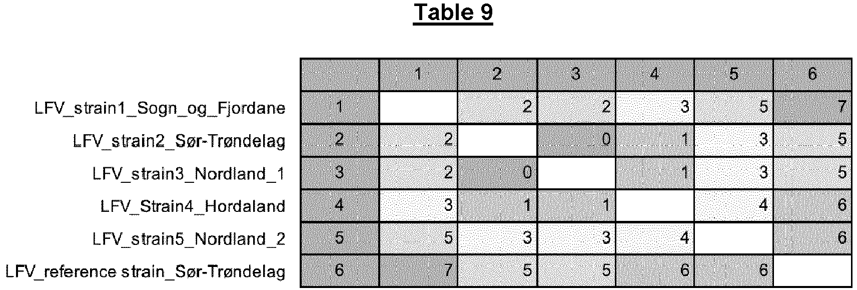

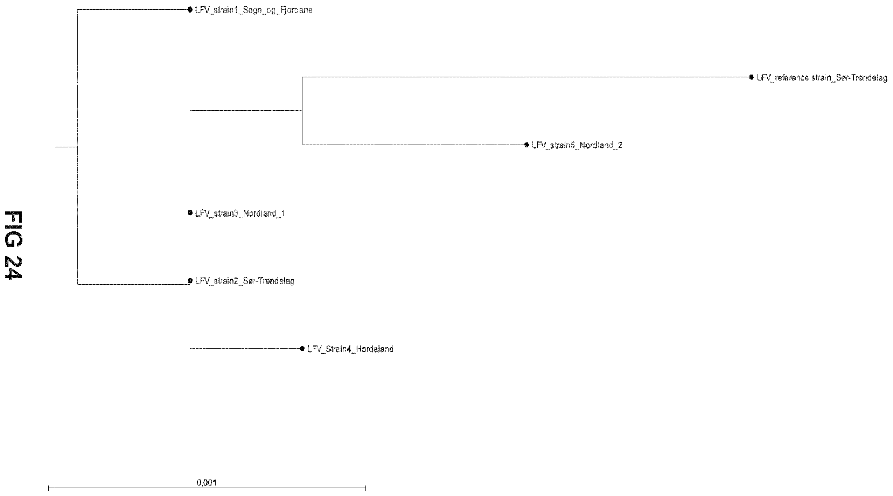

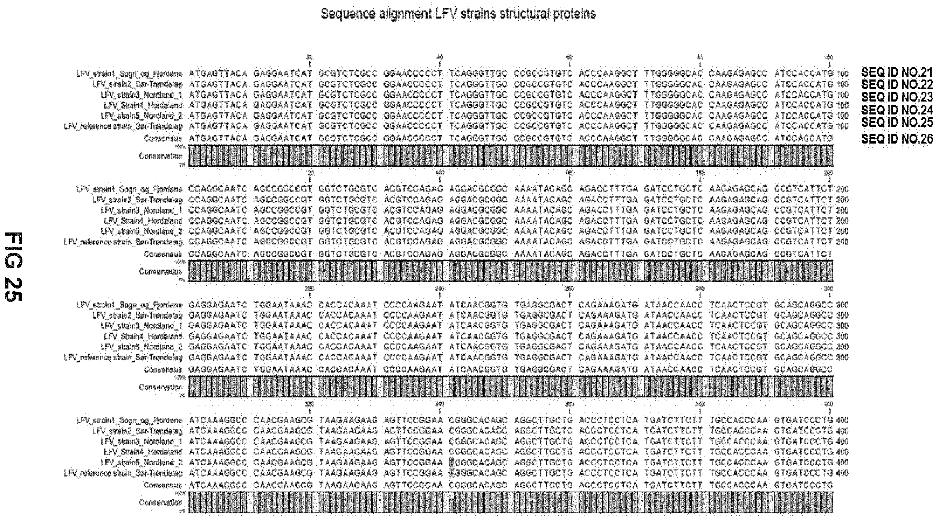

Figure 24 describes a phylogenetic tree constructed from the 2557 nucleotide sequences of the five strains studied in addition to the reference strain. Figure 25 describes the sequence alignment and the consensus sequence of the putative structural proteins of the five LFV strains studied.

The geographically spread field strains of LFV show low nucleotide variation in the region coding for the structural proteins. Although low variation, the reference strain from Sør-Trøndelag is the most distant with 5-7 nt differences compared to the other strains. It should be emphasized that a predominant part of the Lumpfish eggs in Norway are produced at one single localization in Norway (Averøya), where wild Lumpfish ready for spawning are caught locally and the eggs distributed to farms in Norway.

## Claims

1. A primer comprising a sequence of at least about 10 nucleotides, wherein said primer hybridizes to a ribonucleic acid sequence having at least 70% sequence identity to SEQ ID NO. 20, the primer having a DNA sequence selected from the group consisting of; SEQ ID NO. 1, SEQ ID NO. 2, SEQ ID NO. 4, SEQ ID NO. 6, and variants or fragments thereof which have at least 70 % sequence identity to any of the sequences SEQ ID NO. 1, SEQ ID NO. 2, SEQ ID NO. 4, or SEQ I D NO. 6.

2. A method for detection of a virus comprising a ribonucleic acid genome sequence having at least 70% sequence identity to SEQ ID NO: 20, and variants or fragments thereof in a biological sample, the method comprising the following steps:
a) isolating nucleic acids from a biological sample suspected to comprise Lumpfish flavivirus (LFV) or a variant thereof;
b) contacting the nucleic acids with at least one set of primers, each of said primers comprising a sequence of at least about 10 nucleotides, wherein said primers hybridize to a ribonucleic acid sequence having at least 70% sequence identity to SEQ ID NO. 20, the primer having a DNA sequence selected from the group consisting of; SEQ ID NO. 1, SEQ ID NO. 2, SEQ ID NO. 4, SEQ ID NO. 6, and variants or fragments thereof which have at least 70 % sequence identity to any of the sequences SEQ ID NO. 1, SEQ ID NO. 2, SEQ ID NO. 4, or SEQ I D NO. 6;
c) subjecting the mixture of b) to stringent hybridization conditions; and, d) determining whether hybridization between the primer and the nucleic acids has occurred, wherein hybridization indicates the presence of the LFV in the biological sample.

3. A diagnostic kit comprising at least one primer, said at least one primer a sequence of at least about 10 nucleotides, wherein said primer hybridizes to a ribonucleic acid sequence having at least 70% sequence identity to SEQ ID NO. 20, the primer having a DNA sequence selected from the group consisting of; SEQ ID NO. 1, SEQ ID NO. 2, SEQ ID NO. 4, SEQ ID NO. 6, and variants or fragments thereof which have at least 70 % sequence identity to any of the sequences SEQ ID NO. 1, SEQ ID NO. 2, SEQ ID NO. 4, or SEQ I D NO. 6.

4. The primer, method or diagnostic kit according to claims 1 to 3, wherein said primer comprises at least 15 nucleotides.

5. The primer, method or diagnostic kit according to any of claims 1 to 3, wherein said primer is selected from the group consisting of: SEQ ID NO. 8, SEQ ID NO. 9, SEQ ID NO. 10, and SEQ ID NO. 11, SEQ ID NO. 12; SEQ ID NO. 13; SEQ ID NO, 14; SEQ ID NO. 15; SEQ ID NO. 16; SEQ ID NO. 17; SEQ ID NO. 18; and SEQ ID NO. 19; and variants thereof.

6. The primer, method or diagnostic kit according to claim 1 to 3, wherein, said primer is about 20 nucleotides long.

7. The primer, method or diagnostic kit according to any of claims 1 to 3, wherein the primer or primers hybridize(s) to a ribonucleic acid sequence having at least 70% sequence identity to SEQ ID NO. 20.

8. The primer, method or diagnostic kit according to any of claims 1 to 3, wherein the primer or primers hybridize(s) to a ribonucleic acid sequence having at least 80% sequence identity to SEQ ID NO. 20.

9. The primer, method or diagnostic kit according to any of claims 1 to 3, wherein the primer or primers hybridize(s) to a ribonucleic acid sequence having at least 90% sequence identity to SEQ ID NO. 20.

10. The primer, method or diagnostic kit according to any of claims 1 to 3, wherein the primer or primers hybridize(s) to a ribonucleic acid sequence having at least 95% sequence identity to SEQ ID NO. 20.

11. The primer, method or diagnostic kit according to any of claims 1 to 3, wherein the primer or primers have a DNA sequence selected from the group consisting of; SEQ ID NO. 1, SEQ ID NO. 2, SEQ ID NO. 4, SEQ ID NO. 6, and variants or fragments thereof which have at least 80% sequence identity to any of the sequences SEQ ID NO. 1, SEQ ID NO. 2, SEQ ID NO. 4, or SEQ I D NO. 6.

12. The primer, method or diagnostic kit according to any of claims 1 to 3, wherein the primer or primers have a DNA sequence selected from the group consisting of; SEQ ID NO. 1, SEQ ID NO. 2, SEQ ID NO. 4, SEQ ID NO. 6, and variants or fragments thereof which have at least 90% sequence identity to any of the sequences SEQ ID NO. 1, SEQ ID NO. 2, SEQ ID NO. 4, or SEQ I D NO. 6.

13. The primer, method or diagnostic kit according to any of claims 1 to 3, wherein the primer or primers have a DNA sequence selected from the group consisting of; SEQ ID NO. 1, SEQ ID NO. 2, SEQ ID NO. 4, SEQ ID NO. 6, and variants or fragments thereof which have at least 95% sequence identity to any of the sequences SEQ ID NO. 1, SEQ ID NO. 2, SEQ ID NO. 4, or SEQ I D NO. 6.


SEQUENCE LISTING

<110>  Pharmaq AS
       Johansen, Renate
       Brudal, Espen
       Vestvik, Nils Fredrik
       Haugland, Oyvind

<120>  Fish Virus

<130>  ZP000160A

<150>  GB1611512.3
<151>  2016-06-30

<160>  26

<170>  PatentIn version 3.5

<210>  1
<211>  11336
<212>  DNA
<213>  Unknown

<220>
<223>  Flaviviridae

<400>  1
aatttacatt ctgacacgcc aatttagtcg ctaccaacat ttaggtcgct acacagttta      60

accactggtt caacgtttca gaatattgtt aaattacacc aattcagaat tgaacaacac     120

ccaatattga atggtattat aaaacaaaca caatataaca taatataata aattataata     180

aacaaaaaac acacacataa aacaaacaaa caaaaaacaa aacactcaaa caacatactt     240

agaagaataa cccggctggg tccacttctc aagacaatga gttacagagg aatcatgcgt     300

ctcgccggaa cccccttcag ggttgcccgc cgtgtcaccc aaggctttgg gggcaccaag     360

agagccatcc accatgccag gcaatcagcc ggccgtggtc tgcgtcacgt ccagagagga     420

cgcggcaaaa tacagcagac ctttgagatc ctgctcaaga gagcagccgt cattctgagg     480

agaatctgga ataaaccacc acaaatcccc aagaatatca acggtgtgag gcgactcaga     540

aagatgataa ccaacctcaa ctccgtgcag caggccatca aaggcccaac gaagcgtaag     600

aagaagagtt ccggaatggg cacagcaggc ttgctgaccc tcctcatgat cttctttgcc     660

acccaagtga tccctggagg gacctccaca tcaccaacac cttcacccac caccccaaca     720

gacatctcac caatggtgat agcatgtgtc cgcaaggaca agacttcctc tatggatcta     780

ctactggtga atgaggaagg actttttgca aagatggaca acaccaaaga tcctgaactt     840

ttcatacgca acaaccaaac ctatggcatt gatctcacag agtgttccac cggcttccag     900

ccggacacca actactcatc accagaattg aaatacgatg aacatgactg tgccctgatc     960

agagcaggag tgctaccaac ggcagcagac acatgtttcg gaactacatc tctgaggcag    1020

agacacatcc attcaaccct ctatcaagac ctttcaagga agactagaaa cagacgcgat    1080

ctgtcacgtg atactattca gaagtatgag atacacacca accagccatc agaaggctgg 1140

atgaagaccc tatcaacatg ggagaccaaa gtgctatcaa acacagacat aaccatagcc 1200

cttctggccc tggttatggc ctattttaaa gtacccactg tccacctgct gctgatcatc 1260

ctcttttcca aggtttacct cggggtagcc gattcgacct cttcaaccac tgaaatgagc 1320

ggaaaaacac tctgctctga cccggaagaa atgtcaacca tgactggagt agcacacacc 1380

tcagtcagcc tccgcatgga ctacaagcat tgctatcaga tccaaccggt aggcatggac 1440

acaatcacgg ccagaatgag ctacccctat atagacaatg cactcacagg atacaaaata 1500

tcctggttgg acattagttc caactcagtt tcatccgaca gatgcccagg agatggagaa 1560

cccgcctgcc caaaatcagg accatattcc atctgcgcta gaggatacca tgatcgtggc 1620

tggaccactg gctgcttcct cttcggacaa ggaccggtat gtacttgtgc tgaactagcc 1680

ctcaaacatc cgatacaggt ctatacaatc gacccaacaa agatgggagc ccgcttaacg 1740

gtctcctttg gacgcacaga aaatcagaac tattacaaca tggacacaaa cacaggaagt 1800

ttcgacatca cagacaaagc cgactcgtcc tcacgagtca gagtaacatg ccagctacca 1860

cacgcagaac ttgccaccaa gtacctgata aagatggcac caaatgatgg cgaatcatat 1920

ctcgtagata gattggcctt tgaatcaatg aaactcccct ggtccgtatg gaccacgtct 1980

atcccaacca gccttagcaa gattgaagat ggcaacgtcc atgatgccga gacctatata 2040

acttggagag acaccaccct ccattacgtg agattttcag aagcccataa cgtcgaatcc 2100

atggtcaaga atttcctgga caagaacggg aagataatca tagccgagat ggccaaagga 2160

tcaggaagag cctccccagg caccaagatg cccttagaca ttagccccac agactgcagt 2220

ttcatgctta attcccttaa aatacacacc accagtgtga aggactgtgc agacacaacc 2280

aactacgcat tcaaactgat cgaaggcagc agaatactca ccactcacga tggagttgtc 2340

acctttaact tagatatggc agttcctagc cagtgcaggt tagtcgtcaa gggacgtagc 2400

cgcagccaag ttggctctgg atacacctcc aactactgca cccttttcga tcaggcacac 2460

atagtcactt caaccaaaga agtactgaaa atgcactgcc cacccggaga gcatgaaatt 2520

aaactaggca acaacaagca tcaagtgaat gtcccccaaa tgggtatcac aggatggctt 2580

agcagatgga ccactaacgt tctggaagtc aaagacagaa taaccataga tggagcagtt 2640

aagagaccag tactagacat gttcaagaac ttctttgcca aggagcaggc cttcctagga 2700

agccttttcg gaatgacatc aacaatcatg atggcagtga cgggaggagc catagtatgg 2760

ctcggaaccc taatgacagg taacatgaag aatctcacag tcatcatagg acttatcctc 2820

atggccccct tagtaataac ggaggtagaa gcagagaagt atggttgcgt ggctgacaca 2880

gatgaatggt caatggaatg tggcagtatc aaggacgtca tgttcgattc atcaatcaag 2940

ctgtatgtcc ctcaattagg agacagctac aacatagact tagacctcat ggacggctta 3000

acaatcctta ggatgccaaa cagagaacaa atccagaaag ccgccaaagc cttaggctcc 3060

gtactccaca aagtctacac agatgaagga gaagaatgcg ccactgatgg tacagaccat 3120

gacgtgaatg acaccacctc ttgggatcaa acctcccgta aactcaaaga tgatgggaaa 3180

caaggagcac taatcacctg ccataattca gaaaaacact gcaagaactg ccgagaaatc 3240

tttttggaag tcaatgaggt agacgatgtc gatggatgga tgtatgacac caaagagtta 3300

gaaaccacca cctccacacc accagtctac tataaaggcg aagaaagacc agaatcttac 3360

ctcaaagacc tcacagtgga ctgccgtagg gcagggaaac attataaatg ttcggcccac 3420

ggagttacct ggtttggaac catccacact gatgaagaga aggaatactg cctgacacac 3480

aaagtcgaag gcactgtcac ccaaatattt ggagcaaaaa ggagaagttg gaggagctgc 3540

ggaatcagct gctcctctct ctgcgagaga gtcaacacca ccacaaaagg acacatctgg 3600

cccaagagat ttggaggacc agaatgcaac ttcaactgct ggccaatcac caaaaaacag 3660

aacgaagaag atgagaagaa agaaatgtca gcagccgaga acgcctatag aggcggtatc 3720

atcctacgca aggaagctag cgagagactc ctcaagagat ctctctataa agacagagcc 3780

ctgaccttta actccacctg gtgcaaacag aagaaatgca acaccactgt aatcaaagcc 3840

aacgatggcg acgaggaaat agatgaaggt tttacccccg tagacaagga agaagaacac 3900

gaccatgatg aagatttcat atccggaaaa actagcctag ccacagccat gcaagagaac 3960

ggccacaacc aagccacaat tgatgcagcc actggtttct acaagatgca gagggccacc 4020

ggcaccaaag ccgttaacca gctcatcaat gagctagtca aagcctcagg aggtgagaga 4080

acagccagca atgacaattt tatcaattct ccagcaacat tgaagagtga gatggagttg 4140

ctttcatact cccccacccg aattgaaaaa gccctcagga gctacaagaa aggaacagaa 4200

gcgaaaggca ccaaacagat taggaactta attagagaac tactgacaga tggagaacaa 4260

ccactcacta acggtgcaag cgaactaacc accacagcca ctgttgttct gggttttcct 4320

tagcttcatt cgcctttgag cccgcgactg aagcctttga atggctcctt aagacttttt 4380

tgacactaac aaccctcata tttaaccatg ggtttcgtgt aagaccggtt ggagctaaga 4440

cccagatcac catcttcgca acagcactac tccaccacct aacaccatta tcaatggaac 4500

caatcagtca atttggttgg tatgcagtac agactgccat tgatagtccg actaggttta 4560

tcatcagctt gctcatcaca ctcgaagtca tacttatctt catgttccct aaaccgattt 4620

acaacatgac acgatggtat tggtcttcct acaaaaaaga gagcattatg cagatggtgg 4680

caggaggagc gatctgtctg gccttagcac aagcacccaa gaacctttcc tacatcctgt 4740

tcccagtggc ggccctctac atcctcatga ggaggggcgc tactgttcga acacggcacc 4800

tcacatccca actgttagta cccatagaca aagacgatga tccaccgaca ggagtcggta 4860

ccataaccct taaggaaggc aagttacaat atgatatgag tagcctccac gaccacaggg 4920

tcgtgacgga ttccactagg gttttcgtgg tggtattaat tgccattttt ggcctcctag 4980

actacagggc tggaatagcc gccctcatcc tggcctttgg ttactttaag aatgccaaag 5040

ccgacctgtt cgacaccttc aacattgccg gacccgagag agtggagaaa gatactttct 5100

ctggccccga aggcatatat gaggtataca ataggacacc attaggagat gaacatcttg 5160

gttatggtta caacaagaac aacatttttc acaccatgta ccacattaca aaaggactgc 5220

ccctagaatt cgaagatgaa gtgaccaccc cctacgatta tagtgatgaa ctggattgga 5280

tcacctacgg tggcccctgg gctttcgcca aacgggaagg cagggacctc ttcgtctatc 5340

tccacaggga gcatagactt agaccactaa aaatcagttc agaagacgat aaagtctcac 5400

tgggaacaac gaggaccata gcatttggcg actctggctc accagtacta gctagagacg 5460

ccgacggaac actgacaccc gtctcattag ctggacacac ggtaccacta caggatccaa 5520

catatgaagc catgatttta cccaccccta ctacaggcat ggacctttca aaatttcaga 5580

ggaaattcga cacaagaggc ccaggatgga atccaatagt gttaagatgt ggagcaggaa 5640

aaacaagaca cgttctgaga agagccgtgg ttagatctac ggaaacagga cgtaactgtc 5700

tacttttagc acccaccagg acggtagcag gagaaatgta tgaagcatta aaagatttag 5760

atgtagggat tgatatcaca ggcaaggtcg aaagaagaaa caagagaaac cttatcatgt 5820

gccactcaac agcagtaaac cgcctcctcc accatagcct caacatccac aactacaaga 5880

acgtctttgt ggacgaagca cacatgcaag accccatgac tatagcattg ctaggttttc 5940

tcgaagaaag aaccagagac accagcaatg acatccaggt ctttccgatg acggccacct 6000

ggtgggacca ctatgaaact gaaactaatt ttccagtgat tgacaccaaa cttaacgatg 6060

aaaaaatgat cgttgatgca ataaaagact cacttagtaa gaatagaaga agcattacat 6120

tcgtccccac catcaagaaa gctgaagaac tctacaccag tgtaggtgaa ggagaaagaa 6180

tcatcctcaa tagatatacc tacagaagtg aaatcagcaa aatcaaaaca gataaggaca 6240

agcccatagc catctttgca accaatatag ccgaggtcgg cctaaacgcc aatttagacg 6300

acgtttttga cctccagcaa cagatagttt ttaaggagag tgtggccggt gtaatcacta 6360

gagaaacatc cggcgcctcc aaagcatcca ctacacagag aaggggaaga gttggaagag 6420

tcagatcagg cacttatcag tacttagacc aagccgcaac acacaacatc atgcatgact 6480

cagatatcat ccgtaaggag gctgccatag tattaaaaat gttaggagtc gacagtgaga 6540

gcacccgatt ttgccaactc ccccagatcc cacccatctc cctatcaaaa aagaaaggca 6600

taatgaggtt gaccaacgat aatgataaca taacaatcta cttagcccac tacgtcactg 6660

acgattcagg aatagaagta gattggacga ccggagtaca cccttgctca tgtaacgatt 6720

gcaaaacagc caataataag ttcatgatag tagaccccag atcacactca tacatgatgc 6780

acaagcagaa tggagagcca ctcatcaaag gcgacacccg catttacgaa ctccgtggat 6840

cctccactat cacaggtagc atacttcgct acattaaagc gtctgccaac ccaacatcca 6900

gactaatgat gtcagtattc gcggtggagg tggattggac ctgggctgac ccttacgtga 6960

ctagtttcaa ccaaggctac atagccatca ttaaacaact ccccatatgg ataggagtcc 7020

acctaaaaat agaaagggta gcagctgcta tcctgtggct cctgatttgg aagatcataa 7080

catacatttt tgggggaaga agcaatgaca gtaaagtcgt cataaacaat ggaaacatga 7140

gttcactacc cggcttagcc atattagcaa caggcattgc cctacacttc gttgacaaat 7200

tcaactcagg tcatttacca gtagccctag tatcggtggg gtgcatctac atcttaggtg 7260

tcctctacta ctcacagaga caggttagct ataagaccac agcgagtgag gctgcttttt 7320

acctctttgc cgtctttgcc acgctagtag gactattcat ctatatactt agaagccaca 7380

gggacgtttt tgatgatttt acatcaccca ggaaacgcta ctaccacacg gaagaagaca 7440

agaaagaaca tgagcccata actgaggctc cacctcttag cattagcaag ggatttacct 7500

ttttcctggg gttagtgccc ctcatcaacc tcctggcgga gacaatgaca cagagcaaag 7560

gcctacaaaa catgaatgtc aacactctac gtcattcact tagcggtggt gcagaagtca 7620

aaccccccgt acttatcacc atcttcaccg tctttgccat gttccagggt tcctcgctca 7680

cagactgcat cgtcacactt tgcctctctg tcctttatta ccttttctat ttcggacact 7740

cagcacacgt taacaccaaa agtgcagtca cagccagccc gggaaacacc accggagatt 7800

cactaggttt ctccaatgca ccccttaaag aagatggggt gattaaggtg tttaccggtc 7860

tggtactact gacagacgcc ctacttagac aggaaatgac ggtggctcta gcacttaatg 7920

cttttttcac actgataccc ctcttcctct ctaaaggaga ggaacccagc tacgcattct 7980

acatgataat ccaagccggt ttagaacaag aacttctcct ggtcataggc tccgtagcaa 8040

cagccctctt ttgcagatcc ataagccgga ccagaaaaac agggggagcc gtgctatcca 8100

tgagttttaa ctcagtcgtt ggacgacata aaaatctcga accaggtttc caagactaca 8160

agacgagaat gaacggactt ggcaagaaca ccttctacaa cctgagaaaa cacatggtcc 8220

ccgtacacga caaggggaca gtatgctcca gaggctacca caaaatgaat gaactcctca 8280

gataccacga tttagaaatt agcggaaaaa tcttagaatt aggatgtggt tctggtggct 8340

ttacccaaag aatggtatta gagaaaaaag tcgatagaat tgacgccatc tcttggggac 8400

cagataagaa agaccacaat ctgtttgaac tattcaaaaa atcaacacca ggccatggaa 8460

aagttaggta ctcagtagga gaagcattca ccaacaggga ttttgagaac tacgactggg 8520

tcgtgatgga cataggagaa caaagtccag acatggcccg ggagaggacc agagacctgt 8580

ggaggttaga ctggatcacc aagaaagtca tcccccatgc taaagtcatc atgaagatct 8640

tgtcccctac agatatagac gtcctaagag ccataccacc aggttacagg atcgttagac 8700

tatatcattc ttggaactca aattttgaaa tgtacatgat cccgggcgac ccctcagata 8760

acatcactag gatgaaccag ctcctcaaaa accttaacat caggctccag caaacactgg 8820

aaggagaaca tgtggagtta ggggcagaac aagtaaaact gccttcagtc gtggagaaaa 8880

taggcactaa ccttggaaga gaatatgagg attggtgcct ggcagagaga ttagaacaac 8940

tccctgccct cacacaggtc gaaaggccta aattggacca ctttcagtat ctggccagtt 9000

tcttcactga ggcccgcaag ctacctggca catgctctaa ccccattatc atgaccttct 9060

ggaatggact caccaaactt attcctgggg ctgggggatt caccatgaca gacaccacac 9120

acgatggtag ttacaagatg ttccaaaaga agatggataa cccaccaagc accgatcaaa 9180

cctacatccc tgaactccat agagtttttg acaagatgag tgactttctc tttaaaagaa 9240

acaaaccaag aagactcacc agagaggaaa tcatccaaat agttaggaac gacgcagccg 9300

tcggaaacat acatccagac atcatttggc caacagcact ggaagcccta gaatcagaaa 9360

gtttctggaa agtagttgag gaagaaacac acctccattc ccaaggaaaa tgccgctatg 9420

gcatattcaa caccatgggc aagagggaaa agaaggacac agacggtgaa agaagtggct 9480

cacgcataat atgctaccta ccacttgtgg aaagagtcat tgagatggac gtcctaggtt 9540

tcctcaataa agatcacatc gcaggaccgg aaagtctgcc atgtggggtg tcaggagtta 9600

gtccctatca ttacccagac atattttcga agaaagcagg actagatcca gaaacaggag 9660

acatgaccaa agcagtcatc caagatgaca cagcagcctg ggacaccaga gtaagaaaag 9720

atgtcctgca catggaacgc gacttcatcc ttaaacacac cacagacccc taccacgtca 9780

acctcataaa aacacagtac aaaatctacg gcgaaccgat ccttacgatc accagaccct 9840

tcaccgttga caggtcggta gttgacgtcc tccaaggcta cggacagaga tgttctggca 9900

cagtagtcac ctacgccatg aacaccatca caaatgcatg tgtccagaca ctcaggttct 9960

gggaggccat ggacgtagac ttggaggaag tcctaaatga catgccccac atcctcaaca 10020

gtatgatgat cagtggagac gacatgctct tcatggtaga accatggcta gccaaaaaac 10080

tcagttcatc tctcaaagtc atcaattcac tcggcttccc tcgcaaagga ctcacaacat 10140

ggcaggaatc acccataatc acccagtttg ataaagtcta cttctgcagt cataagttca 10200

ccatcgccag agtggccggt gaaagacgag cattcttaga caaagacctt ttcgagatca 10260

tcgggaaaac ccaactcgtt ctgggaggac tacacagccc acacgagata gccggacacg 10320

caaaaagcgt agccatctac atgttcgtca ccttcttcca caacagagcc gtgagaaagg 10380

tggcattggc catcctatca gcactgcccg acgaactggt gcccatgggg aatgtcgcca 10440

accccttctt tctggatcac tcctggatca gcaatgaaga cctaatcact atcttcaaca 10500

atgtccacgg cacacatgcc aggagctaca ctgacattgg ttacacgtac aaaacgttgg 10560

ccaagatcag aggagaccca ttggatcatg aagaacggac cacatggaac aaagacctca 10620

agagaaaaat cgagtcgatc tccacccact acaacagtgg tataacaaat gggagtttct 10680

ggcacgagca actccgaaca aacgttagtt accacagcct attctaggac tgtagcacca 10740

cttagtttag ataagaaaac aaacaaaaaa ccttactata aaaaataaaa aataagaaaa 10800

tacttgctat gaaataactt agatttagaa aatgtattaa cttagattta gagaatgtat 10860

tcatttactg tttagttagg accaagagga ctgctactct aagacgtgag gtcgttaaat 10920

aacatcgacc aagtacgaac acttacaact agtggtcgga agataacaaa aagccgtaac 10980

ctatctccgg tttttcaata aatagagtag aaagaaaaga accctgaatc ccccgcgtga 11040

tcaggtttaa ataaaagcaa cgtggaaaaa ttattagctt aatgaaaatg tttattaaaa 11100

ttacgcttat attatgtaaa tattattgtt ttataaaatg ttgtactaaa tgtaaatgac 11160

atgtatatag tcaccttacc actccagtat tacacaaaca ccattacagt atcgggcgag 11220

ttgttacatt cttatgttca tggaactaga attggtgtat tcctaccaag ctgtaggttt 11280

aacggaaaca ctaattttgg aactacttaa tatacagaag tagaaacaag cccccc 11336

<210> 2
<211> 4047
<212> DNA
<213> Unknown

<220>
<223> Flaviviridae

<400> 2
atgagttaca gaggaatcat gcgtctcgcc ggaaccccct tcagggttgc ccgccgtgtc 60

acccaaggct ttgggggcac caagagagcc atccaccatg ccaggcaatc agccggccgt 120

ggtctgcgtc acgtccagag aggacgcggc aaaatacagc agacctttga gatcctgctc 180

aagagagcag ccgtcattct gaggagaatc tggaataaac caccacaaat ccccaagaat 240

atcaacggtg tgaggcgact cagaaagatg ataaccaacc tcaactccgt gcagcaggcc 300

atcaaaggcc caacgaagcg taagaagaag agttccggaa tgggcacagc aggcttgctg 360

accctcctca tgatcttctt tgccacccaa gtgatccctg gagggacctc cacatcacca 420

acaccttcac ccaccacccc aacagacatc tcaccaatgg tgatagcatg tgtccgcaag 480

gacaagactt cctctatgga tctactactg gtgaatgagg aaggactttt tgcaaagatg 540

gacaacacca aagatcctga acttttcata cgcaacaacc aaacctatgg cattgatctc 600

acagagtgtt ccaccggctt ccagccggac accaactact catcaccaga attgaaatac 660

gatgaacatg actgtgccct gatcagagca ggagtgctac caacggcagc agacacatgt 720

ttcggaacta catctctgag gcagagacac atccattcaa ccctctatca agacctttca 780

aggaagacta gaaacagacg cgatctgtca cgtgatacta ttcagaagta tgagatacac 840

accaaccagc catcagaagg ctggatgaag accctatcaa catgggagac caaagtgcta 900

tcaaacacag acataaccat agcccttctg gccctggtta tggcctattt taaagtaccc 960

actgtccacc tgctgctgat catcctcttt tccaaggttt acctcggggt agccgattcg 1020

acctcttcaa ccactgaaat gagcggaaaa acactctgct ctgacccgga agaaatgtca 1080

accatgactg gagtagcaca cacctcagtc agcctccgca tggactacaa gcattgctat 1140

cagatccaac cggtaggcat ggacacaatc acggccagaa tgagctaccc ctatatagac 1200

aatgcactca caggatacaa aatatcctgg ttggacatta gttccaactc agtttcatcc 1260

gacagatgcc caggagatgg agaacccgcc tgcccaaaat caggaccata ttccatctgc 1320

gctagaggat accatgatcg tggctggacc actggctgct tcctcttcgg acaaggaccg 1380

gtatgtactt gtgctgaact agccctcaaa catccgatac aggtctatac aatcgaccca 1440

acaaagatgg gagcccgctt aacggtctcc tttggacgca cagaaaatca gaactattac 1500

aacatggaca caaacacagg aagtttcgac atcacagaca aagccgactc gtcctcacga 1560

gtcagagtaa catgccagct accacacgca gaacttgcca ccaagtacct gataaagatg 1620

gcaccaaatg atggcgaatc atatctcgta gatagattgg cctttgaatc aatgaaactc 1680

ccctggtccg tatggaccac gtctatccca accagcctta gcaagattga agatggcaac 1740

gtccatgatg ccgagaccta tataacttgg agagacacca ccctccatta cgtgagattt 1800

tcagaagccc ataacgtcga atccatggtc aagaatttcc tggacaagaa cgggaagata 1860

atcatagccg agatggccaa aggatcagga agagcctccc caggcaccaa gatgccctta 1920

gacattagcc ccacagactg cagtttcatg cttaattccc ttaaaataca caccaccagt 1980

gtgaaggact gtgcagacac aaccaactac gcattcaaac tgatcgaagg cagcagaata 2040

ctcaccactc acgatggagt tgtcaccttt aacttagata tggcagttcc tagccagtgc 2100

aggttagtcg tcaaggacgg tagccgcagc caagttggct ctggatacac ctccaactac 2160

tgcacccttt tcgatcaggc acacatagtc acttcaacca aagaagtact gaaaatgcac 2220

tgcccacccg gagagcatga aattaaacta ggcaacaaca agcatcaagt gaatgtcccc 2280

caaatgggta tcacaggatg gcttagcaga tggaccacta acgttctgga agtcaaagac 2340

agaataacca tagatggagc agttaagaga ccagtactag acatgttcaa gaacttcttt 2400

gccaaaggag caggcttcct aggaagcctt ttcggaatga catcaacaat catgatggca 2460

gtgacgggag gagccatagt atggctcgga accctaatga caggtaacat gaagaatctc 2520

acagtcatca taggacttat cctcatggcc cccttagtaa taacggaggt agaagcagag 2580

aagtatggtt gcgtggctga cacagatgaa tggtcaatgg aatgtggcag tatcaaggac 2640

gtcatgttcg attcatcaat caagctgtat gtccctcaat taggagacag ctacaacata 2700

gacttagacc tcatggacgg cttaacaatc cttaggatgc caaacagaga acaaatccag 2760

aaagccgcca aagccttagg ctccgtactc cacaaagtct acacagatga aggagaagaa 2820

tgcgccactg atggtacaga ccatgacgtg aatgacacca cctcttggga tcaaacctcc 2880

cgtaaactca aagatgatgg gaaacaagga gcactaatca cctgccataa ttcagaaaaa 2940

cactgcaaga actgccgaga aatctttttg gaagtcaatg aggtagacga tgtcgatgga 3000

tggatgtatg acaccaaaga gttagaaacc accacctcca caccaccagt ctactataaa 3060

ggcgaagaaa gaccagaatc ttacctcaaa gacctcacag tggactgccg tagggcaggg 3120

aaacattata aatgttcggc ccacggagtt acctggtttg gaaccatcca cactgatgaa 3180

gagaaggaat actgcctgac acacaaagtc gaaggcactg tcacccaaat atttggagca 3240

aaaaggagaa gttggaggag ctgcggaatc agctgctcct ctctctgcga gagagtcaac 3300

accaccacaa aaggacacat ctggcccaag agatttggag gaccagaatg caacttcaac 3360

tgctggccaa tcaccaaaaa acagaacgaa gaagatgaga agaaagaaat gtcagcagcc 3420

gagaacgcct atagaggcgg tatcatccta cgcaaggaag ctagcgagag actcctcaag 3480

agatctctct ataaagacag agccctgacc tttaactcca cctggtgcaa acagaagaaa 3540

tgcaacacca ctgtaatcaa agccaacgat ggcgacgagg aaatagatga aggttttacc 3600

cccgtagaca aggaagaaga acacgaccat gatgaagatt tcatatccgg aaaaactagc 3660

ctagccacag ccatgcaaga gaacggccac aaccaagcca caattgatgc agccactggt 3720

ttctacaaga tgcagagggc caccggcacc aaagccgtta accagctcat caatgagcta 3780

gtcaaagcct caggaggtga gagaacagcc agcaatgaca attttatcaa ttctccagca 3840

acattgaaga gtgagatgga gttgctttca tactccccca cccgaattga aaaagccctc 3900

aggagctaca agaaaggaac agaagcgaaa ggcaccaaac agattaggaa cttaattaga 3960

gaactactga cagatggaga acaaccactc actaacggtg caagcgaact aaccaccaca 4020

gccactgttg ttctgggttt tccttag 4047

<210> 3
<211> 1348
<212> PRT
<213> Unknown

<220>
<223> Flaviviridae

<400> 3

Met Ser Tyr Arg Gly Ile Met Arg Leu Ala Gly Thr Pro Phe Arg Val
1               5                   10                  15

Ala Arg Arg Val Thr Gln Gly Phe Gly Gly Thr Lys Arg Ala Ile His
            20                  25                  30

His Ala Arg Gln Ser Ala Gly Arg Gly Leu Arg His Val Gln Arg Gly
        35                  40                  45

Arg Gly Lys Ile Gln Gln Thr Phe Glu Ile Leu Leu Lys Arg Ala Ala
    50                  55                  60

Val Ile Leu Arg Arg Ile Trp Asn Lys Pro Pro Gln Ile Pro Lys Asn
65                  70                  75                  80

Ile Asn Gly Val Arg Arg Leu Arg Lys Met Ile Thr Asn Leu Asn Ser
                85                  90                  95

Val Gln Gln Ala Ile Lys Gly Pro Thr Lys Arg Lys Lys Lys Ser Ser
            100                 105                 110

Gly Met Gly Thr Ala Gly Leu Leu Thr Leu Leu Met Ile Phe Phe Ala
        115                 120                 125

Thr Gln Val Ile Pro Gly Gly Thr Ser Thr Ser Pro Thr Pro Ser Pro
    130                 135                 140

Thr Thr Pro Thr Asp Ile Ser Pro Met Val Ile Ala Cys Val Arg Lys
145                 150                 155                 160

Asp Lys Thr Ser Ser Met Asp Leu Leu Leu Val Asn Glu Glu Gly Leu
                165                 170                 175

Phe Ala Lys Met Asp Asn Thr Lys Asp Pro Glu Leu Phe Ile Arg Asn
            180                 185                 190

Asn Gln Thr Tyr Gly Ile Asp Leu Thr Glu Cys Ser Thr Gly Phe Gln
        195                 200                 205

Pro Asp Thr Asn Tyr Ser Ser Pro Glu Leu Lys Tyr Asp Glu His Asp
    210                 215                 220

Cys Ala Leu Ile Arg Ala Gly Val Leu Pro Thr Ala Ala Asp Thr Cys
225                 230                 235                 240

Phe Gly Thr Thr Ser Leu Arg Gln Arg His Ile His Ser Thr Leu Tyr
                245                 250                 255

Gln Asp Leu Ser Arg Lys Thr Arg Asn Arg Arg Asp Leu Ser Arg Asp
            260                 265                 270

Thr Ile Gln Lys Tyr Glu Ile His Thr Asn Gln Pro Ser Glu Gly Trp
        275                 280                 285

Met Lys Thr Leu Ser Thr Trp Glu Thr Lys Val Leu Ser Asn Thr Asp
    290                 295                 300

Ile Thr Ile Ala Leu Leu Ala Leu Val Met Ala Tyr Phe Lys Val Pro
305                 310                 315                 320

Thr Val His Leu Leu Leu Ile Ile Leu Phe Ser Lys Val Tyr Leu Gly
                325                 330                 335

Val Ala Asp Ser Thr Ser Ser Thr Thr Glu Met Ser Gly Lys Thr Leu
            340                 345                 350

Cys Ser Asp Pro Glu Glu Met Ser Thr Met Thr Gly Val Ala His Thr
        355                 360                 365

Ser Val Ser Leu Arg Met Asp Tyr Lys His Cys Tyr Gln Ile Gln Pro
    370                 375                 380

Val Gly Met Asp Thr Ile Thr Ala Arg Met Ser Tyr Pro Tyr Ile Asp
385                 390                 395                 400

Asn Ala Leu Thr Gly Tyr Lys Ile Ser Trp Leu Asp Ile Ser Ser Asn
                405                 410                 415

Ser Val Ser Ser Asp Arg Cys Pro Gly Asp Gly Glu Pro Ala Cys Pro
            420                 425                 430

Lys Ser Gly Pro Tyr Ser Ile Cys Ala Arg Gly Tyr His Asp Arg Gly
        435                 440                 445

Trp Thr Thr Gly Cys Phe Leu Phe Gly Gln Gly Pro Val Cys Thr Cys
    450                 455                 460

Ala Glu Leu Ala Leu Lys His Pro Ile Gln Val Tyr Thr Ile Asp Pro
465                 470                 475                 480

Thr Lys Met Gly Ala Arg Leu Thr Val Ser Phe Gly Arg Thr Glu Asn
                485                 490                 495

Gln Asn Tyr Tyr Asn Met Asp Thr Asn Thr Gly Ser Phe Asp Ile Thr
            500                 505                 510

Asp Lys Ala Asp Ser Ser Ser Arg Val Arg Val Thr Cys Gln Leu Pro
        515                 520                 525

His Ala Glu Leu Ala Thr Lys Tyr Leu Ile Lys Met Ala Pro Asn Asp
    530                 535                 540

Gly Glu Ser Tyr Leu Val Asp Arg Leu Ala Phe Glu Ser Met Lys Leu
545                 550                 555                 560

Pro Trp Ser Val Trp Thr Thr Ser Ile Pro Thr Ser Leu Ser Lys Ile
                565                 570                 575

Glu Asp Gly Asn Val His Asp Ala Glu Thr Tyr Ile Thr Trp Arg Asp
            580                 585                 590

Thr Thr Leu His Tyr Val Arg Phe Ser Glu Ala His Asn Val Glu Ser
        595                 600                 605

Met Val Lys Asn Phe Leu Asp Lys Asn Gly Lys Ile Ile Ile Ala Glu
    610                 615                 620

Met Ala Lys Gly Ser Gly Arg Ala Ser Pro Gly Thr Lys Met Pro Leu
625                 630                 635                 640

Asp Ile Ser Pro Thr Asp Cys Ser Phe Met Leu Asn Ser Leu Lys Ile
                645                 650                 655

His Thr Thr Ser Val Lys Asp Cys Ala Asp Thr Thr Asn Tyr Ala Phe
            660                 665                 670

Lys Leu Ile Glu Gly Ser Arg Ile Leu Thr Thr His Asp Gly Val Val
        675                 680                 685

Thr Phe Asn Leu Asp Met Ala Val Pro Ser Gln Cys Arg Leu Val Val
    690                 695                 700

Lys Gly Arg Ser Arg Ser Gln Val Gly Ser Gly Tyr Thr Ser Asn Tyr
705                 710                 715                 720

Cys Thr Leu Phe Asp Gln Ala His Ile Val Thr Ser Thr Lys Glu Val
                725                 730                 735

Leu Lys Met His Cys Pro Pro Gly Glu His Glu Ile Lys Leu Gly Asn
            740                 745                 750

Asn Lys His Gln Val Asn Val Pro Gln Met Gly Ile Thr Gly Trp Leu
        755                 760                 765

Ser Arg Trp Thr Thr Asn Val Leu Glu Val Lys Asp Arg Ile Thr Ile

770                 775                 780

Asp Gly Ala Val Lys Arg Pro Val Leu Asp Met Phe Lys Asn Phe Phe
785                 790                 795                 800

Ala Lys Gly Ala Gly Phe Leu Gly Ser Leu Phe Gly Met Thr Ser Thr
                805                 810                 815

Ile Met Met Ala Val Thr Gly Gly Ala Ile Val Trp Leu Gly Thr Leu
            820                 825                 830

Met Thr Gly Asn Met Lys Asn Leu Thr Val Ile Ile Gly Leu Ile Leu
        835                 840                 845

Met Ala Pro Leu Val Ile Thr Glu Val Glu Ala Glu Lys Tyr Gly Cys
    850                 855                 860

Val Ala Asp Thr Asp Glu Trp Ser Met Glu Cys Gly Ser Ile Lys Asp
865                 870                 875                 880

Val Met Phe Asp Ser Ser Ile Lys Leu Tyr Val Pro Gln Leu Gly Asp
                885                 890                 895

Ser Tyr Asn Ile Asp Leu Asp Leu Met Asp Gly Leu Thr Ile Leu Arg
            900                 905                 910

Met Pro Asn Arg Glu Gln Ile Gln Lys Ala Ala Lys Ala Leu Gly Ser
        915                 920                 925

Val Leu His Lys Val Tyr Thr Asp Glu Gly Glu Glu Cys Ala Thr Asp
    930                 935                 940

Gly Thr Asp His Asp Val Asn Asp Thr Thr Ser Trp Asp Gln Thr Ser
945                 950                 955                 960

Arg Lys Leu Lys Asp Asp Gly Lys Gln Gly Ala Leu Ile Thr Cys His
                965                 970                 975

Asn Ser Glu Lys His Cys Lys Asn Cys Arg Glu Ile Phe Leu Glu Val
            980                 985                 990

Asn Glu Val Asp Asp Val Asp Gly  Trp Met Tyr Asp Thr  Lys Glu Leu
        995                 1000                1005

Glu Thr  Thr Thr Ser Thr Pro  Pro Val Tyr Tyr Lys  Gly Glu Glu
    1010                1015                1020

Arg Pro Glu Ser Tyr Leu Lys Asp Leu Thr Val Asp Cys Arg Arg
    1025                1030                1035

Ala Gly Lys His Tyr Lys Cys Ser Ala His Gly Val Thr Trp Phe
    1040                1045                1050

Gly Thr Ile His Thr Asp Glu Glu Lys Glu Tyr Cys Leu Thr His
    1055                1060                1065

Lys Val Glu Gly Thr Val Thr Gln Ile Phe Gly Ala Lys Arg Arg
    1070                1075                1080

Ser Trp Arg Ser Cys Gly Ile Ser Cys Ser Ser Leu Cys Glu Arg
    1085                1090                1095

Val Asn Thr Thr Thr Lys Gly His Ile Trp Pro Lys Arg Phe Gly
    1100                1105                1110

Gly Pro Glu Cys Asn Phe Asn Cys Trp Pro Ile Thr Lys Lys Gln
    1115                1120                1125

Asn Glu Glu Asp Glu Lys Lys Glu Met Ser Ala Ala Glu Asn Ala
    1130                1135                1140

Tyr Arg Gly Gly Ile Ile Leu Arg Lys Glu Ala Ser Glu Arg Leu
    1145                1150                1155

Leu Lys Arg Ser Leu Tyr Lys Asp Arg Ala Leu Thr Phe Asn Ser
    1160                1165                1170

Thr Trp Cys Lys Gln Lys Lys Cys Asn Thr Thr Val Ile Lys Ala
    1175                1180                1185

Asn Asp Gly Asp Glu Glu Ile Asp Glu Gly Phe Thr Pro Val Asp
    1190                1195                1200

Lys Glu Glu Glu His Asp His Asp Glu Asp Phe Ile Ser Gly Lys
    1205                1210                1215

Thr Ser Leu Ala Thr Ala Met Gln Glu Asn Gly His Asn Gln Ala
    1220                1225                1230

Thr Ile Asp Ala Ala Thr Gly Phe Tyr Lys Met Gln Arg Ala Thr
    1235                1240                1245

Gly Thr Lys Ala Val Asn Gln Leu Ile Asn Glu Leu Val Lys Ala
    1250                1255                1260

Ser Gly Gly Glu Arg Thr Ala Ser Asn Asp Asn Phe Ile Asn Ser
    1265                1270                1275

Pro Ala Thr Leu Lys Ser Glu Met Glu Leu Leu Ser Tyr Ser Pro
    1280                1285                1290

Thr Arg Ile Glu Lys Ala Leu Arg Ser Tyr Lys Lys Gly Thr Glu
    1295                1300                1305

Ala Lys Gly Thr Lys Gln Ile Arg Asn Leu Ile Arg Glu Leu Leu
    1310                1315                1320

Thr Asp Gly Glu Gln Pro Leu Thr Asn Gly Ala Ser Glu Leu Thr
    1325                1330                1335

Thr Thr Ala Thr Val Val Leu Gly Phe Pro
    1340                1345

<210> 4
<211> 6408
<212> DNA
<213> Unknown

<220>
<223> Flaviviridae

<400> 4
ttagcttcat tcgcctttga gcccgcgact gaagcctttg aatggctcct taagactttt      60

ttgacactaa caaccctcat atttaaccat gggtttcgtg taagaccggt tggagctaag     120

acccagatca ccatcttcgc aacagcacta ctccaccacc taacaccatt atcaatggaa     180

ccaatcagtc aatttggttg gtatgcagta cagactgcca ttgatagtcc gactaggttt     240

atcatcagct tgctcatcac actcgaagtc atacttatct tcatgttccc taaaccgatt     300

tacaacatga cacgatggta ttggtcttcc tacaaaaaag agagcattat gcagatggtg     360

gcaggaggag cgatctgtct ggccttagca caagcaccca agaacctttc ctacatcctg     420

ttcccagtgg cggccctcta catcctcatg aggaggggcg ctactgttcg aacacggcac     480

ctcacatccc aactgttagt acccatagac aaagacgatg atccaccgac aggagtcggt     540

accataaccc ttaaggaagg caagttacaa tatgatatga gtagcctcca cgaccacagg     600

gtcgtgacgg attccactag ggttttcgtg gtggtattaa ttgccatttt tggcctccta     660

gactacaggg ctggaatagc cgccctcatc ctggcctttg gttactttaa gaatgccaaa     720

gccgacctgt tcgacacctt caacattgcc ggacccgaga gagtggagaa agatactttc     780

tctggccccg aaggcatata tgaggtatac aataggacac cattaggaga tgaacatctt     840

ggttatggtt acaacaagaa caacattttt cacaccatgt accacattac aaaaggactg     900

cccctagaat tcgaagatga agtgaccacc ccctacgatt atagtgatga actggattgg 960

atcacctacg gtggcccctg ggctttcgcc aaacgggaag gcagggacct cttcgtctat 1020

ctccacaggg agcatagact tagaccacta aaaatcagtt cagaagacga taaagtctca 1080

ctgggaacaa cgaggaccat agcatttggc gactctggct caccagtact agctagagac 1140

gccgacggaa cactgacacc cgtctcatta gctggacaca cggtaccact acaggatcca 1200

acatatgaag ccatgatttt acccacccct actacaggca tggacctttc aaaatttcag 1260

aggaaattcg acacaagagg cccaggatgg aatccaatag tgttaagatg tggagcagga 1320

aaaacaagac acgttctgag aagagccgtg gttagatcta cggaaacagg acgtaactgt 1380

ctacttttag cacccaccag gacggtagca ggagaaatgt atgaagcatt aaaagattta 1440

gatgtaggga ttgatatcac aggcaaggtc gaaagaagaa acaagagaaa ccttatcatg 1500

tgccactcaa cagcagtaaa ccgcctcctc caccatagcc tcaacatcca caactacaag 1560

aacgtctttg tggacgaagc acacatgcaa gaccccatga ctatagcatt gctaggtttt 1620

ctcgaagaaa gaaccagaga caccagcaat gacatccagg tctttccgat gacggccacc 1680

tggtgggacc actatgaaac tgaaactaat tttccagtga ttgacaccaa acttaacgat 1740

gaaaaaatga tcgttgatgc aataaaagac tcacttagta agaatagaag aagcattaca 1800

ttcgtcccca ccatcaagaa agctgaagaa ctctacacca gtgtaggtga aggagaaaga 1860

atcatcctca atagatatac ctacagaagt gaaatcagca aaatcaaaac agataaggac 1920

aagcccatag ccatctttgc aaccaatata gccgaggtcg gcctaaacgc caatttagac 1980

gacgtttttg acctccagca acagatagtt tttaaggaga gtgtggccgg tgtaatcact 2040

agagaaacat ccggcgcctc caaagcatcc actacacaga gaaggggaag agttggaaga 2100

gtcagatcag gcacttatca gtacttagac caagccgcaa cacacaacat catgcatgac 2160

tcagatatca tccgtaagga ggctgccata gtattaaaaa tgttaggagt cgacagtgag 2220

agcacccgat tttgccaact cccccagatc ccacccatct ccctatcaaa aaagaaaggc 2280

ataatgaggt tgaccaacga taatgataac ataacaatct acttagccca ctacgtcact 2340

gacgattcag gaatagaagt agattggacg accggagtac acccttgctc atgtaacgat 2400

tgcaaaacag ccaataataa gttcatgata gtagacccca gatcacactc atacatgatg 2460

cacaagcaga atggagagcc actcatcaaa ggcgacaccc gcatttacga actccgtgga 2520

tcctccacta tcacaggtag catacttcgc tacattaaag cgtctgccaa cccaacatcc 2580

agactaatga tgtcagtatt cgcggtggag gtggattgga cctgggctga cccttacgtg 2640

actagtttca accaaggcta catagccatc attaaacaac tccccatatg gataggagtc 2700

cacctaaaaa tagaaagggt agcagctgct atcctgtggc tcctgatttg gaagatcata 2760

acatacattt ttgggggaag aagcaatgac agtaaagtcg tcataaacaa tggaaacatg 2820

agttcactac ccggcttagc catattagca acaggcattg ccctacactt cgttgacaaa 2880

ttcaactcag gtcatttacc agtagcccta gtatcggtgg ggtgcatcta catcttaggt 2940

gtcctctact actcacagag acaggttagc tataagacca cagcgagtga ggctgctttt 3000

tacctctttg ccgtctttgc cacgctagta ggactattca tctatatact tagaagccac 3060

agggacgttt ttgatgattt tacatcaccc aggaaacgct actaccacac ggaagaagac 3120

aagaaagaac atgagcccat aactgaggct ccacctctta gcattagcaa gggatttacc 3180

tttttcctgg ggttagtgcc cctcatcaac ctcctggcgg agacaatgac acagagcaaa 3240

ggcctacaaa acatgaatgt caacactcta cgtcattcac ttagcggtgg tgcagaagtc 3300

aaaccccccg tacttatcac catcttcacc gtctttgcca tgttccaggg ttcctcgctc 3360

acagactgca tcgtcacact ttgcctctct gtcctttatt accttttcta tttcggacac 3420

tcagcacacg ttaacaccaa aagtgcagtc acagccagcc cgggaaacac caccggagat 3480

tcactaggtt tctccaatgc accccttaaa gaagatgggg tgattaaggt gtttaccggt 3540

ctggtactac tgacagacgc cctacttaga caggaaatga cggtggctct agcacttaat 3600

gcttttttca cactgatacc cctcttcctc tctaaaggag aggaacccag ctacgcattc 3660

tacatgataa tccaagccgg tttagaacaa gaacttctcc tggtcatagg ctccgtagca 3720

acagccctct tttgcagatc cataagccgg accagaaaaa cagggggagc cgtgctatcc 3780

atgagtttta actcagtcgt tggacgacat aaaaatctcg aaccaggttt ccaagactac 3840

aagacgagaa tgaacggact tggcaagaac accttctaca acctgagaaa acacatggtc 3900

cccgtacacg acaaggggac agtatgctcc agaggctacc acaaaatgaa tgaactcctc 3960

agataccacg atttagaaat tagcggaaaa atcttagaat taggatgtgg ttctggtggc 4020

tttacccaaa gaatggtatt agagaaaaaa gtcgatagaa ttgacgccat ctcttgggga 4080

ccagataaga aagaccacaa tctgtttgaa ctattcaaaa aatcaacacc aggccatgga 4140

aaagttaggt actcagtagg agaagcattc accaacaggg attttgagaa ctacgactgg 4200

gtcgtgatgg acataggaga acaaagtcca gacatggccc gggagaggac cagagacctg 4260

tggaggttag actggatcac caagaaagtc atcccccatg ctaaagtcat catgaagatc 4320

ttgtccccta cagatataga cgtcctaaga gccataccac caggttacag gatcgttaga 4380

ctatatcatt cttggaactc aaattttgaa atgtacatga tcccgggcga cccctcagat 4440

aacatcacta ggatgaacca gctcctcaaa aaccttaaca tcaggctcca gcaaacactg 4500

gaaggagaac atgtggagtt aggggcagaa caagtaaaac tgccttcagt cgtggagaaa 4560

ataggcacta accttggaag agaatatgag gattggtgcc tggcagagag attagaacaa 4620

ctccctgccc tcacacaggt cgaaaggcct aaattggacc actttcagta tctggccagt 4680

ttcttcactg aggcccgcaa gctacctggc acatgctcta accccattat catgaccttc 4740

tggaatggac tcaccaaact tattcctggg gctgggggat tcaccatgac agacaccaca 4800

cacgatggta gttacaagat gttccaaaag aagatggata acccaccaag caccgatcaa 4860

acctacatcc ctgaactcca tagagttttt gacaagatga gtgactttct ctttaaaaga 4920

aacaaaccaa gaagactcac cagagaggaa atcatccaaa tagttaggaa cgacgcagcc 4980

gtcggaaaca tacatccaga catcatttgg ccaacagcac tggaagccct agaatcagaa 5040

agtttctgga aagtagttga ggaagaaaca cacctccatt cccaaggaaa atgccgctat 5100

ggcatattca acaccatggg caagagggaa aagaaggaca cagacggtga aagaagtggc 5160

tcacgcataa tatgctacct accacttgtg gaaagagtca ttgagatgga cgtcctaggt 5220

ttcctcaata aagatcacat cgcaggaccg gaaagtctgc catgtggggt gtcaggagtt 5280

agtccctatc attacccaga catattttcg aagaaagcag gactagatcc agaaacagga 5340

gacatgacca aagcagtcat ccaagatgac acagcagcct gggacaccag agtaagaaaa 5400

gatgtcctgc acatggaacg cgacttcatc cttaaacaca ccacagaccc ctaccacgtc 5460

aacctcataa aaacacagta caaaatctac ggcgaaccga tccttacgat caccagaccc 5520

ttcaccgttg acaggtcggt agttgacgtc ctccaaggct acggacagag atgttctggc 5580

acagtagtca cctacgccat gaacaccatc acaaatgcat gtgtccagac actcaggttc 5640

tgggaggcca tggacgtaga cttggaggaa gtcctaaatg acatgcccca catcctcaac 5700

agtatgatga tcagtggaga cgacatgctc ttcatggtag aaccatggct agccaaaaaa 5760

ctcagttcat ctctcaaagt catcaattca ctcggcttcc ctcgcaaagg actcacaaca 5820

tggcaggaat cacccataat cacccagttt gataaagtct acttctgcag tcataagttc 5880

accatcgcca gagtggccgg tgaaagacga gcattcttag acaaagacct tttcgagatc 5940

atcgggaaaa cccaactcgt tctgggagga ctacacagcc cacacgagat agccggacac 6000

gcaaaaagcg tagccatcta catgttcgtc accttcttcc acaacagagc cgtgagaaag 6060

gtggcattgg ccatcctatc agcactgccc gacgaactgg tgcccatggg gaatgtcgcc 6120

aaccccttct ttctggatca ctcctggatc agcaatgaag acctaatcac tatcttcaac 6180

aatgtccacg gcacacatgc caggagctac actgacattg gttacacgta caaaacgttg 6240

gccaagatca gaggagaccc attggatcat gaagaacgga ccacatggaa caaagacctc 6300

aagagaaaaa tcgagtcgat ctccacccac tacaacagtg gtataacaaa tgggagtttc 6360

tggcacgagc aactccgaac aaacgttagt taccacagcc tattctag 6408

<210> 5
<211> 2135
<212> PRT

<213>  Unknown

<220>
<223>  Flaviviridae

<400>  5

Leu Ala Ser Phe Ala Phe Glu Pro Ala Thr Glu Ala Phe Glu Trp Leu
1               5                   10                  15

Leu Lys Thr Phe Leu Thr Leu Thr Thr Leu Ile Phe Asn His Gly Phe
            20                  25                  30

Arg Val Arg Pro Val Gly Ala Lys Thr Gln Ile Thr Ile Phe Ala Thr
        35                  40                  45

Ala Leu Leu His His Leu Thr Pro Leu Ser Met Glu Pro Ile Ser Gln
    50                  55                  60

Phe Gly Trp Tyr Ala Val Gln Thr Ala Ile Asp Ser Pro Thr Arg Phe
65                  70                  75                  80

Ile Ile Ser Leu Leu Ile Thr Leu Glu Val Ile Leu Ile Phe Met Phe
                85                  90                  95

Pro Lys Pro Ile Tyr Asn Met Thr Arg Trp Tyr Trp Ser Ser Tyr Lys
            100                 105                 110

Lys Glu Ser Ile Met Gln Met Val Ala Gly Gly Ala Ile Cys Leu Ala
        115                 120                 125

Leu Ala Gln Ala Pro Lys Asn Leu Ser Tyr Ile Leu Phe Pro Val Ala
    130                 135                 140

Ala Leu Tyr Ile Leu Met Arg Arg Gly Ala Thr Val Arg Thr Arg His
145                 150                 155                 160

Leu Thr Ser Gln Leu Leu Val Pro Ile Asp Lys Asp Asp Asp Pro Pro
                165                 170                 175

Thr Gly Val Gly Thr Ile Thr Leu Lys Glu Gly Lys Leu Gln Tyr Asp
            180                 185                 190

Met Ser Ser Leu His Asp His Arg Val Val Thr Asp Ser Thr Arg Val
        195                 200                 205

Phe Val Val Val Leu Ile Ala Ile Phe Gly Leu Leu Asp Tyr Arg Ala
    210                 215                 220

Gly Ile Ala Ala Leu Ile Leu Ala Phe Gly Tyr Phe Lys Asn Ala Lys
225                 230                 235                 240

Ala Asp Leu Phe Asp Thr Phe Asn Ile Ala Gly Pro Glu Arg Val Glu
                245                 250                 255

Lys Asp Thr Phe Ser Gly Pro Glu Gly Ile Tyr Glu Val Tyr Asn Arg
            260                 265                 270

Thr Pro Leu Gly Asp Glu His Leu Gly Tyr Gly Tyr Asn Lys Asn Asn
        275                 280                 285

Ile Phe His Thr Met Tyr His Ile Thr Lys Gly Leu Pro Leu Glu Phe
    290                 295                 300

Glu Asp Glu Val Thr Thr Pro Tyr Asp Tyr Ser Asp Glu Leu Asp Trp
305                 310                 315                 320

Ile Thr Tyr Gly Gly Pro Trp Ala Phe Ala Lys Arg Glu Gly Arg Asp
                325                 330                 335

Leu Phe Val Tyr Leu His Arg Glu His Arg Leu Arg Pro Leu Lys Ile
            340                 345                 350

Ser Ser Glu Asp Asp Lys Val Ser Leu Gly Thr Thr Arg Thr Ile Ala
        355                 360                 365

Phe Gly Asp Ser Gly Ser Pro Val Leu Ala Arg Asp Ala Asp Gly Thr
    370                 375                 380

Leu Thr Pro Val Ser Leu Ala Gly His Thr Val Pro Leu Gln Asp Pro
385                 390                 395                 400

Thr Tyr Glu Ala Met Ile Leu Pro Thr Pro Thr Thr Gly Met Asp Leu
                405                 410                 415

Ser Lys Phe Gln Arg Lys Phe Asp Thr Arg Gly Pro Gly Trp Asn Pro
            420                 425                 430

Ile Val Leu Arg Cys Gly Ala Gly Lys Thr Arg His Val Leu Arg Arg
        435                 440                 445

Ala Val Val Arg Ser Thr Glu Thr Gly Arg Asn Cys Leu Leu Leu Ala
    450                 455                 460

Pro Thr Arg Thr Val Ala Gly Glu Met Tyr Glu Ala Leu Lys Asp Leu
465                 470                 475                 480

Asp Val Gly Ile Asp Ile Thr Gly Lys Val Glu Arg Arg Asn Lys Arg
                485                 490                 495

Asn Leu Ile Met Cys His Ser Thr Ala Val Asn Arg Leu Leu His His
            500                 505                 510

Ser Leu Asn Ile His Asn Tyr Lys Asn Val Phe Val Asp Glu Ala His
        515                 520                 525

Met Gln Asp Pro Met Thr Ile Ala Leu Leu Gly Phe Leu Glu Glu Arg
    530                 535                 540

Thr Arg Asp Thr Ser Asn Asp Ile Gln Val Phe Pro Met Thr Ala Thr
545                 550                 555                 560

Trp Trp Asp His Tyr Glu Thr Glu Thr Asn Phe Pro Val Ile Asp Thr
                565                 570                 575

Lys Leu Asn Asp Glu Lys Met Ile Val Asp Ala Ile Lys Asp Ser Leu
            580                 585                 590

Ser Lys Asn Arg Arg Ser Ile Thr Phe Val Pro Thr Ile Lys Lys Ala
        595                 600                 605

Glu Glu Leu Tyr Thr Ser Val Gly Glu Gly Glu Arg Ile Ile Leu Asn
    610                 615                 620

Arg Tyr Thr Tyr Arg Ser Glu Ile Ser Lys Ile Lys Thr Asp Lys Asp
625                 630                 635                 640

Lys Pro Ile Ala Ile Phe Ala Thr Asn Ile Ala Glu Val Gly Leu Asn
                645                 650                 655

Ala Asn Leu Asp Asp Val Phe Asp Leu Gln Gln Gln Ile Val Phe Lys
            660                 665                 670

Glu Ser Val Ala Gly Val Ile Thr Arg Glu Thr Ser Gly Ala Ser Lys
        675                 680                 685

Ala Ser Thr Thr Gln Arg Arg Gly Arg Val Gly Arg Val Arg Ser Gly
    690                 695                 700

Thr Tyr Gln Tyr Leu Asp Gln Ala Ala Thr His Asn Ile Met His Asp
705                 710                 715                 720

Ser Asp Ile Ile Arg Lys Glu Ala Ala Ile Val Leu Lys Met Leu Gly
                725                 730                 735

Val Asp Ser Glu Ser Thr Arg Phe Cys Gln Leu Pro Gln Ile Pro Pro
            740                 745                 750

Ile Ser Leu Ser Lys Lys Lys Gly Ile Met Arg Leu Thr Asn Asp Asn
        755                 760                 765

Asp Asn Ile Thr Ile Tyr Leu Ala His Tyr Val Thr Asp Asp Ser Gly
    770                 775                 780

Ile Glu Val Asp Trp Thr Thr Gly Val His Pro Cys Ser Cys Asn Asp
785                 790                 795                 800

Cys Lys Thr Ala Asn Asn Lys Phe Met Ile Val Asp Pro Arg Ser His
                805                 810                 815

Ser Tyr Met Met His Lys Gln Asn Gly Glu Pro Leu Ile Lys Gly Asp
            820                 825                 830

Thr Arg Ile Tyr Glu Leu Arg Gly Ser Ser Thr Ile Thr Gly Ser Ile
        835                 840                 845

Leu Arg Tyr Ile Lys Ala Ser Ala Asn Pro Thr Ser Arg Leu Met Met
    850                 855                 860

Ser Val Phe Ala Val Glu Val Asp Trp Thr Trp Ala Asp Pro Tyr Val
865                 870                 875                 880

Thr Ser Phe Asn Gln Gly Tyr Ile Ala Ile Ile Lys Gln Leu Pro Ile
                885                 890                 895

Trp Ile Gly Val His Leu Lys Ile Glu Arg Val Ala Ala Ala Ile Leu
            900                 905                 910

Trp Leu Leu Ile Trp Lys Ile Ile Thr Tyr Ile Phe Gly Gly Arg Ser
        915                 920                 925

Asn Asp Ser Lys Val Val Ile Asn Asn Gly Asn Met Ser Ser Leu Pro
    930                 935                 940

Gly Leu Ala Ile Leu Ala Thr Gly Ile Ala Leu His Phe Val Asp Lys
945                 950                 955                 960

Phe Asn Ser Gly His Leu Pro Val Ala Leu Val Ser Val Gly Cys Ile
                965                 970                 975

Tyr Ile Leu Gly Val Leu Tyr Tyr Ser Gln Arg Gln Val Ser Tyr Lys

            980                 985                 990

Thr Thr Ala Ser Glu Ala Ala Phe Tyr Leu Phe Ala Val Phe Ala Thr
        995                 1000                1005

Leu Val Gly Leu Phe Ile Tyr Ile Leu Arg Ser His Arg Asp Val
    1010                1015                1020

Phe Asp Asp Phe Thr Ser Pro Arg Lys Arg Tyr Tyr His Thr Glu
    1025                1030                1035

Glu Asp Lys Lys Glu His Glu Pro Ile Thr Glu Ala Pro Pro Leu
    1040                1045                1050

Ser Ile Ser Lys Gly Phe Thr Phe Phe Leu Gly Leu Val Pro Leu
    1055                1060                1065

Ile Asn Leu Leu Ala Glu Thr Met Thr Gln Ser Lys Gly Leu Gln
    1070                1075                1080

Asn Met Asn Val Asn Thr Leu Arg His Ser Leu Ser Gly Gly Ala
    1085                1090                1095

Glu Val Lys Pro Pro Val Leu Ile Thr Ile Phe Thr Val Phe Ala
    1100                1105                1110

Met Phe Gln Gly Ser Ser Leu Thr Asp Cys Ile Val Thr Leu Cys
    1115                1120                1125

Leu Ser Val Leu Tyr Tyr Leu Phe Tyr Phe Gly His Ser Ala His
    1130                1135                1140

Val Asn Thr Lys Ser Ala Val Thr Ala Ser Pro Gly Asn Thr Thr
    1145                1150                1155

Gly Asp Ser Leu Gly Phe Ser Asn Ala Pro Leu Lys Glu Asp Gly
    1160                1165                1170

Val Ile Lys Val Phe Thr Gly Leu Val Leu Leu Thr Asp Ala Leu
    1175                1180                1185

Leu Arg Gln Glu Met Thr Val Ala Leu Ala Leu Asn Ala Phe Phe
    1190                1195                1200

Thr Leu Ile Pro Leu Phe Leu Ser Lys Gly Glu Glu Pro Ser Tyr
    1205                1210                1215

Ala Phe Tyr Met Ile Ile Gln Ala Gly Leu Glu Gln Glu Leu Leu
    1220                1225                1230

Leu Val Ile Gly Ser Val Ala Thr Ala Leu Phe Cys Arg Ser Ile
    1235                1240                1245

Ser Arg Thr Arg Lys Thr Gly Gly Ala Val Leu Ser Met Ser Phe
    1250                1255                1260

Asn Ser Val Val Gly Arg His Lys Asn Leu Glu Pro Gly Phe Gln
    1265                1270                1275

Asp Tyr Lys Thr Arg Met Asn Gly Leu Gly Lys Asn Thr Phe Tyr
    1280                1285                1290

Asn Leu Arg Lys His Met Val Pro Val His Asp Lys Gly Thr Val
    1295                1300                1305

Cys Ser Arg Gly Tyr His Lys Met Asn Glu Leu Leu Arg Tyr His
    1310                1315                1320

Asp Leu Glu Ile Ser Gly Lys Ile Leu Glu Leu Gly Cys Gly Ser
    1325                1330                1335

Gly Gly Phe Thr Gln Arg Met Val Leu Glu Lys Lys Val Asp Arg
    1340                1345                1350

Ile Asp Ala Ile Ser Trp Gly Pro Asp Lys Lys Asp His Asn Leu
    1355                1360                1365

Phe Glu Leu Phe Lys Lys Ser Thr Pro Gly His Gly Lys Val Arg
    1370                1375                1380

Tyr Ser Val Gly Glu Ala Phe Thr Asn Arg Asp Phe Glu Asn Tyr
    1385                1390                1395

Asp Trp Val Val Met Asp Ile Gly Glu Gln Ser Pro Asp Met Ala
    1400                1405                1410

Arg Glu Arg Thr Arg Asp Leu Trp Arg Leu Asp Trp Ile Thr Lys
    1415                1420                1425

Lys Val Ile Pro His Ala Lys Val Ile Met Lys Ile Leu Ser Pro
    1430                1435                1440

Thr Asp Ile Asp Val Leu Arg Ala Ile Pro Pro Gly Tyr Arg Ile
    1445                1450                1455

Val Arg Leu Tyr His Ser Trp Asn Ser Asn Phe Glu Met Tyr Met
    1460                1465                1470

Ile Pro Gly Asp Pro Ser Asp Asn Ile Thr Arg Met Asn Gln Leu
    1475                1480                1485

Leu Lys Asn Leu Asn Ile Arg Leu Gln Gln Thr Leu Glu Gly Glu
    1490                1495                1500

His Val Glu Leu Gly Ala Glu Gln Val Lys Leu Pro Ser Val Val
    1505                1510                1515

Glu Lys Ile Gly Thr Asn Leu Gly Arg Glu Tyr Glu Asp Trp Cys
    1520                1525                1530

Leu Ala Glu Arg Leu Glu Gln Leu Pro Ala Leu Thr Gln Val Glu
    1535                1540                1545

Arg Pro Lys Leu Asp His Phe Gln Tyr Leu Ala Ser Phe Phe Thr
    1550                1555                1560

Glu Ala Arg Lys Leu Pro Gly Thr Cys Ser Asn Pro Ile Ile Met
    1565                1570                1575

Thr Phe Trp Asn Gly Leu Thr Lys Leu Ile Pro Gly Ala Gly Gly
    1580                1585                1590

Phe Thr Met Thr Asp Thr Thr His Asp Gly Ser Tyr Lys Met Phe
    1595                1600                1605

Gln Lys Lys Met Asp Asn Pro Pro Ser Thr Asp Gln Thr Tyr Ile
    1610                1615                1620

Pro Glu Leu His Arg Val Phe Asp Lys Met Ser Asp Phe Leu Phe
    1625                1630                1635

Lys Arg Asn Lys Pro Arg Arg Leu Thr Arg Glu Glu Ile Ile Gln
    1640                1645                1650

Ile Val Arg Asn Asp Ala Ala Val Gly Asn Ile His Pro Asp Ile
    1655                1660                1665

Ile Trp Pro Thr Ala Leu Glu Ala Leu Glu Ser Glu Ser Phe Trp
    1670                1675                1680

Lys Val Val Glu Glu Glu Thr His Leu His Ser Gln Gly Lys Cys
    1685                1690                1695

Arg Tyr Gly Ile Phe Asn Thr Met Gly Lys Arg Glu Lys Lys Asp
    1700                1705                1710

Thr Asp Gly Glu Arg Ser Gly Ser Arg Ile Ile Cys Tyr Leu Pro
    1715                1720                1725

Leu Val Glu Arg Val Ile Glu Met Asp Val Leu Gly Phe Leu Asn
    1730                1735                1740

Lys Asp His Ile Ala Gly Pro Glu Ser Leu Pro Cys Gly Val Ser
    1745                1750                1755

Gly Val Ser Pro Tyr His Tyr Pro Asp Ile Phe Ser Lys Lys Ala
    1760                1765                1770

Gly Leu Asp Pro Glu Thr Gly Asp Met Thr Lys Ala Val Ile Gln
    1775                1780                1785

Asp Asp Thr Ala Ala Trp Asp Thr Arg Val Arg Lys Asp Val Leu
    1790                1795                1800

His Met Glu Arg Asp Phe Ile Leu Lys His Thr Thr Asp Pro Tyr
    1805                1810                1815

His Val Asn Leu Ile Lys Thr Gln Tyr Lys Ile Tyr Gly Glu Pro
    1820                1825                1830

Ile Leu Thr Ile Thr Arg Pro Phe Thr Val Asp Arg Ser Val Val
    1835                1840                1845

Asp Val Leu Gln Gly Tyr Gly Gln Arg Cys Ser Gly Thr Val Val
    1850                1855                1860

Thr Tyr Ala Met Asn Thr Ile Thr Asn Ala Cys Val Gln Thr Leu
    1865                1870                1875

Arg Phe Trp Glu Ala Met Asp Val Asp Leu Glu Glu Val Leu Asn
    1880                1885                1890

Asp Met Pro His Ile Leu Asn Ser Met Met Ile Ser Gly Asp Asp
    1895                1900                1905

Met Leu Phe Met Val Glu Pro Trp Leu Ala Lys Lys Leu Ser Ser
    1910                1915                1920

Ser Leu Lys Val Ile Asn Ser Leu Gly Phe Pro Arg Lys Gly Leu

1925                    1930                    1935

Thr Thr Trp Gln Glu Ser Pro Ile Ile Thr Gln Phe Asp Lys Val
    1940                    1945                    1950

Tyr Phe Cys Ser His Lys Phe Thr Ile Ala Arg Val Ala Gly Glu
    1955                    1960                    1965

Arg Arg Ala Phe Leu Asp Lys Asp Leu Phe Glu Ile Ile Gly Lys
    1970                    1975                    1980

Thr Gln Leu Val Leu Gly Gly Leu His Ser Pro His Glu Ile Ala
    1985                    1990                    1995

Gly His Ala Lys Ser Val Ala Ile Tyr Met Phe Val Thr Phe Phe
    2000                    2005                    2010

His Asn Arg Ala Val Arg Lys Val Ala Leu Ala Ile Leu Ser Ala
    2015                    2020                    2025

Leu Pro Asp Glu Leu Val Pro Met Gly Asn Val Ala Asn Pro Phe
    2030                    2035                    2040

Phe Leu Asp His Ser Trp Ile Ser Asn Glu Asp Leu Ile Thr Ile
    2045                    2050                    2055

Phe Asn Asn Val His Gly Thr His Ala Arg Ser Tyr Thr Asp Ile
    2060                    2065                    2070

Gly Tyr Thr Tyr Lys Thr Leu Ala Lys Ile Arg Gly Asp Pro Leu
    2075                    2080                    2085

Asp His Glu Glu Arg Thr Thr Trp Asn Lys Asp Leu Lys Arg Lys
    2090                    2095                    2100

Ile Glu Ser Ile Ser Thr His Tyr Asn Ser Gly Ile Thr Asn Gly
    2105                    2110                    2115

Ser Phe Trp His Glu Gln Leu Arg Thr Asn Val Ser Tyr His Ser
    2120                    2125                    2130

Leu Phe
    2135

<210>  6
<211>  1557
<212>  DNA
<213>  Unknown

<220>
<223>  Flaviviridae

<400>  6
acctcttcaa ccactgaaat gagcggaaaa acactctgct ctgacccgga agaaatgtca      60

accatgactg gagtagcaca cacctcagtc agcctccgca tggactacaa gcattgctat     120

cagatccaac cggtaggcat ggacacaatc acggccagaa tgagctaccc ctatatagac     180

aatgcactca caggatacaa aatatcctgg ttggacatta gttccaactc agtttcatcc     240

gacagatgcc caggagatgg agaacccgcc tgcccaaaat caggaccata ttccatctgc     300

gctagaggat accatgatcg tggctggacc actggctgct tcctcttcgg acaaggaccg     360

gtatgtactt gtgctgaact agccctcaaa catccgatac aggtctatac aatcgaccca     420

acaaagatgg gagcccgctt aacggtctcc tttggacgca cagaaaatca gaactattac     480

aacatggaca caaacacagg aagtttcgac atcacagaca aagccgactc gtcctcacga     540

gtcagagtaa catgccagct accacacgca gaacttgcca ccaagtacct gataaagatg     600

gcaccaaatg atggcgaatc atatctcgta gatagattgg cctttgaatc aatgaaactc     660

ccctggtccg tatggaccac gtctatccca accagcctta gcaagattga agatggcaac     720

gtccatgatg ccgagaccta tataacttgg agagacacca ccctccatta cgtgagattt     780

tcagaagccc ataacgtcga atccatggtc aagaatttcc tggacaagaa cgggaagata     840

atcatagccg agatggccaa aggatcagga agagcctccc caggcaccaa gatgccctta     900

gacattagcc ccacagactg cagtttcatg cttaattccc ttaaaataca caccaccagt     960

gtgaaggact gtgcagacac aaccaactac gcattcaaac tgatcgaagg cagcagaata    1020

ctcaccactc acgatggagt tgtcaccttt aacttagata tggcagttcc tagccagtgc    1080

aggttagtcg tcaagggacg tagccgcagc caagttggct ctggatacac ctccaactac    1140

tgcacccttt tcgatcaggc acacatagtc acttcaacca aagaagtact gaaaatgcac    1200

tgcccacccg gagagcatga aattaaacta ggcaacaaca agcatcaagt gaatgtcccc    1260

caaatgggta tcacaggatg gcttagcaga tggaccacta acgttctgga agtcaaagac    1320

agaataacca tagatggagc agttaagaga ccagtactag acatgttcaa gaacttcttt    1380

gccaaaggag caggcttcct aggaagcctt ttcggaatga catcaacaat catgatggca    1440

gtgacgggag gagccatagt atggctcgga accctaatga caggtaacat gaagaatctc    1500

acagtcatca taggacttat cctcatggcc cccttagtaa taacggaggt agaagca       1557

<210>  7
<211>  519
<212>  PRT
<213>  Unknown

<220>
<223>  Flaviviridae

<400>  7

Thr Ser Ser Thr Thr Glu Met Ser Gly Lys Thr Leu Cys Ser Asp Pro
1               5                   10                  15

Glu Glu Met Ser Thr Met Thr Gly Val Ala His Thr Ser Val Ser Leu
            20                  25                  30

Arg Met Asp Tyr Lys His Cys Tyr Gln Ile Gln Pro Val Gly Met Asp
        35                  40                  45

Thr Ile Thr Ala Arg Met Ser Tyr Pro Tyr Ile Asp Asn Ala Leu Thr
    50                  55                  60

Gly Tyr Lys Ile Ser Trp Leu Asp Ile Ser Ser Asn Ser Val Ser Ser
65                  70                  75                  80

Asp Arg Cys Pro Gly Asp Gly Glu Pro Ala Cys Pro Lys Ser Gly Pro
                85                  90                  95

Tyr Ser Ile Cys Ala Arg Gly Tyr His Asp Arg Gly Trp Thr Thr Gly
            100                 105                 110

Cys Phe Leu Phe Gly Gln Gly Pro Val Cys Thr Cys Ala Glu Leu Ala
        115                 120                 125

Leu Lys His Pro Ile Gln Val Tyr Thr Ile Asp Pro Thr Lys Met Gly
    130                 135                 140

Ala Arg Leu Thr Val Ser Phe Gly Arg Thr Glu Asn Gln Asn Tyr Tyr
145                 150                 155                 160

Asn Met Asp Thr Asn Thr Gly Ser Phe Asp Ile Thr Asp Lys Ala Asp
                165                 170                 175

Ser Ser Ser Arg Val Arg Val Thr Cys Gln Leu Pro His Ala Glu Leu
            180                 185                 190

Ala Thr Lys Tyr Leu Ile Lys Met Ala Pro Asn Asp Gly Glu Ser Tyr
        195                 200                 205

Leu Val Asp Arg Leu Ala Phe Glu Ser Met Lys Leu Pro Trp Ser Val
    210                 215                 220

Trp Thr Thr Ser Ile Pro Thr Ser Leu Ser Lys Ile Glu Asp Gly Asn
225                 230                 235                 240

Val His Asp Ala Glu Thr Tyr Ile Thr Trp Arg Asp Thr Thr Leu His
                245                 250                 255

Tyr Val Arg Phe Ser Glu Ala His Asn Val Glu Ser Met Val Lys Asn
            260                 265                 270

Phe Leu Asp Lys Asn Gly Lys Ile Ile Ile Ala Glu Met Ala Lys Gly
        275                 280                 285

Ser Gly Arg Ala Ser Pro Gly Thr Lys Met Pro Leu Asp Ile Ser Pro
    290                 295                 300

Thr Asp Cys Ser Phe Met Leu Asn Ser Leu Lys Ile His Thr Thr Ser
305                 310                 315                 320

Val Lys Asp Cys Ala Asp Thr Thr Asn Tyr Ala Phe Lys Leu Ile Glu
                325                 330                 335

Gly Ser Arg Ile Leu Thr Thr His Asp Gly Val Val Thr Phe Asn Leu
            340                 345                 350

Asp Met Ala Val Pro Ser Gln Cys Arg Leu Val Val Lys Gly Arg Ser
        355                 360                 365

Arg Ser Gln Val Gly Ser Gly Tyr Thr Ser Asn Tyr Cys Thr Leu Phe
    370                 375                 380

Asp Gln Ala His Ile Val Thr Ser Thr Lys Glu Val Leu Lys Met His
385                 390                 395                 400

Cys Pro Pro Gly Glu His Glu Ile Lys Leu Gly Asn Asn Lys His Gln
                405                 410                 415

Val Asn Val Pro Gln Met Gly Ile Thr Gly Trp Leu Ser Arg Trp Thr
            420                 425                 430

Thr Asn Val Leu Glu Val Lys Asp Arg Ile Thr Ile Asp Gly Ala Val
        435                 440                 445

Lys Arg Pro Val Leu Asp Met Phe Lys Asn Phe Phe Ala Lys Gly Ala
    450                 455                 460

Gly Phe Leu Gly Ser Leu Phe Gly Met Thr Ser Thr Ile Met Met Ala
465                 470                 475                 480

Val Thr Gly Gly Ala Ile Val Trp Leu Gly Thr Leu Met Thr Gly Asn

485                 490                 495

Met Lys Asn Leu Thr Val Ile Ile Gly Leu Ile Leu Met Ala Pro Leu
            500                 505                 510

Val Ile Thr Glu Val Glu Ala
        515

<210>  8
<211>  20
<212>  DNA
<213>  Unknown

<220>
<223>  Flaviviridae

<400>  8
cctccattcc caaggaaaat                                                    20

<210>  9
<211>  20
<212>  DNA
<213>  Unknown

<220>
<223>  Flaviviridae

<400>  9
ggtcctgcga tgtgatcttt                                                    20

<210>  10
<211>  20
<212>  DNA
<213>  Unknown

<220>
<223>  Flaviviridae

<400>  10
gagaacagcc agcaatgaca                                                    20

<210>  11
<211>  20
<212>  DNA
<213>  Unknown

<220>
<223>  Flaviviridae

<400>  11
ttcgagtgtg atgagcaagc                                                    20

<210>  12
<211>  23
<212>  DNA
<213>  Unknown

<220>
<223>  Flaviviridae

<400>  12
acatacttag aagaataacc cgg                                               23


<210>  13
<211>  19
<212>  DNA
<213>  Unknown

<220>
<223>  Flaviviridae

<400>  13
tgccgttggt agcactcct                                                    19


<210>  14
<211>  22
<212>  DNA
<213>  Unknown

<220>
<223>  Flaviviridae

<400>  14
tacgcaacaa ccaaacctat gg                                                22


<210>  15
<211>  20
<212>  DNA
<213>  Unknown

<220>
<223>  Flaviviridae

<400>  15
gcagatggaa tatggtcctg                                                   20


<210>  16
<211>  21
<212>  DNA
<213>  Unknown

<220>
<223>  Flaviviridae

<400>  16
gacaatgcac tcacaggata c                                                 21


<210>  17
<211>  20
<212>  DNA
<213>  Unknown

<220>
<223>  Flaviviridae

<400>  17
gcatgaaact gcagtctgtg                                                    20


<210>  18
<211>  20
<212>  DNA
<213>  Unknown

<220>
<223>  Flaviviridae

<400>  18
tcagaagccc ataacgtcga                                                    20


<210>  19
<211>  21
<212>  DNA
<213>  Unknown

<220>
<223>  Flaviviridae

<400>  19
gatgaatcga acatgacgtc c                                                  21


<210>  20
<211>  11336
<212>  RNA
<213>  Unknown

<220>
<223>  Flaviviridae

<400>  20
   ggggggcuug uuucuacuuc uguauauuaa guaguuccaa aauuaguguu uccguuaaac      60
   cuacagcuug guaggaauac accaauucua guuccaugaa cauaagaaug uaacaacucg     120
   cccgauacug uaaugguguu uguguaauac uggaguggua aggugacuau auacauguca     180
   uuuacauuua guacaacauu uuauaaaaca auaauauuua cauaauauaa gcguaauuuu     240
   aauaaacauu uucauuaagc uaauaauuuu uccacguugc uuuuauuuaa accugaucac     300
   gcgggggauu caggguucuu uucuuucuac ucuauuuauu gaaaaaccgg agauagguua     360
   cggcuuuuug uuaucuuccg accacuaguu guaaguguuc guacuugguc gauguuauuu     420
   aacgaccuca cgucuuagag uagcaguccu cuugguccua acuaaacagu aaaugaauac     480
   auucucuaaa ucuaaguuaa uacauuuucu aaaucuaagu uauuucauag caaguauuuu     540
   cuuauuuuuu auuuuuuaua guaagguuuu uuguuuguuu ucuuaucuaa acuaaguggu     600
   gcuacagucc uagaauaggc ugugguaacu aacguuuguu cggaguugcu cgugccagaa     660
   acucccauuu guuauaccac uguuguagug gguggagauc gacucgauuu uucucuugag     720
   gucuuuguuc cauguggucc guucuucaug auccaauggg ucuccucuga ucuuggccaa     780
   cguuuuguac guguaaccaa ugucagugua gcuccuggca ugugugccgu ggacauuguu 840
   gaagauagug auuaggucuu cauugcugau ccaggaguga uccagaaaga aggggguugc 900
   gacauucccc augggcacca guucgucggg cagugcugau aggauggcca augccaccuu 960
   ucucacggcu cuguugugga agaaggugac gaacauguag auggcuacgc uuuuugcgug 1020
   uccggcuauc ucgugugggc uguguagucc ucccagaacg aguugguuuu ucccgaugau 1080
   cucgaaaagg ucuuugucua agaaugcucg ucuuucaccg gccacucugg cgauggugaa 1140
   cuuaugacug cagaaguaga cuuuaucaaa cugggugauu augggugauu ccugccaugu 1200
   ugugaguccu uugcgaggga agccgaguga auugaugacu uugagagaug aacugaguuu 1260
   uuuggcuagc caugguucua ccaugaagag caugucgucu ccacugauca ucauacuguu 1320
   gaggaugugg ggcaugucau uuaggacuuc cuccaagucu acguccaugg ccucccagaa 1380
   ccugaguguc uggacacaug cauuugugau gguguucaug gcguagguga cuacugugcc 1440
   agaacaucuc uguccguagc cuuggaggac gucaacuacc gaccugucaa cggugaaggg 1500
   ucuggugauc guaaggaucg guucgccgua gauuuuguac uguguuuuua ugagguugac 1560
   gugguagggg ucuguggguu guuuaaggau gaagucgcgu uccaugugca ggacaucuuu 1620
   ucuuacucug gugucccagg cugcuguguc aucuuggaug acugcuuugg ucaugucucc 1680
   uguuucugga ucuaguccug cuuucuucga aaauaugucu ggguaaugau agggacuaac 1740
   uccugacacc ccacauggca gacuuuccgg uccugcgaug ugaucuuuau ugaggaaacc 1800
   uaggacgucc aucucaauga cucuuuccac aagugguagg uagcauauua ugcgugagcc 1860
   acuucuuuca ccgucugugu ccuucuuuuc ccucuugccc augguguuga auaugccaua 1920
   gcggcauuuu ccuugggaau ggaggugugu uucuuccuca acuacuuucc agaaacuuuc 1980
   ugauucuagg gcuuccagug cuguuggcca aaugaugucu ggauguaugu uuccgacggc 2040
   ugcgucguuc cuaacuauuu ggaugauuuc cucucuggug agucuucuug guuuguuucu 2100
   uuuaaagaga aagucacuca ucuugucaaa aacucuaugg aguucaggga uguagguuug 2160
   aucggugcuu gguggguuau ccaucuucuu uuggaacauc uuguaacuac caucgugugu 2220
   ggugucuguc auggugaauc ccccagcccc aggaauaagu uuggugaguc cauuccagaa 2280
   ggucaugaua augggguuag agcaugugcc agguagcuug cgggccucag ugaagaaacu 2340
   ggccagauac ugaaaguggu ccaauuuagg ccuuucgacc ugugugaggg cagggaguug 2400
   uucuaaucuc ucugccaggc accaauccuc auauucucuu ccaagguuag ugccuauuuu 2460
   cuccacgacu gaaggcaguu uuacuuguuc ugccccuaac uccacauguu cuccuuccag 2520
   uguuugcugg agccugaugu uaagguuuuu gaggagcugg uucauccuag ugauguuauc 2580
   ugaggggucg cccgggauca uguacauuuc aaaauuugag uuccaagaau gauauagucu 2640
   aacgauccug uaaccuggug guauggcucu uaggacgucu auaucuguag gggacaagau 2700
   cuucaugaug acuuuagcau gggggaugac uuucuuggug auccagucua accuccacag 2760
   gucucugguc cucucccggg ccaugucugg acuuuguucu ccuaugucca ucacgaccca 2820
   gucguaguuc ucaaaauccc uguuggugaa ugcuucuccu acugaguacc uaacuuuucc 2880
   auggccuggu guugauuuuu ugaauaguuc aaacagauug uggucuuucu uaucuggucc 2940
   ccaagagaug gcgucaauuc uaucgacuuu uuucucuaau accauucuuu ggguaaagcc 3000
   accagaacca cauccuaauu cuaagauuuu uccgcuaauu ucuaaaucgu gguaucugag 3060
   gaguucauuc auuuuguggu agccucugga gcauacuguc cccuugucgu guacggggac 3120
   cauguguuuu cucagguugu agaagguguu cuugccaagu ccguucauuc ucgucuugua 3180
   gucuuggaaa ccugguucga gauuuuuaug ucguccaacg acugaguuaa aacucaugga 3240
   uagcacggcu cccccuguuu uucugguccg gcuuauggau cugcaaaaga gggcuguugc 3300
   uacggagccu augaccagga gaaguucuug uucuaaaccg gcuuggauua ucauguagaa 3360
   ugcguagcug gguuccucuc cuuuagagag gaagaggggu aucaguguga aaaaagcauu 3420
   aagugcuaga gccaccguca uuuccugucu aaguagggcg ucugucagua guaccagacc 3480
   gguaaacacc uuaaucaccc caucuucuuu aaggggugca uuggagaaac cuagugaauc 3540
   uccgguggug uuucccgggc uggcugugac ugcacuuuug guguuaacgu gugcugagug 3600
   uccgaaauag aaaagguaau aaaggacaga gaggcaaagu gugacgaugc agucugugag 3660
   cgaggaaccc uggaacaugg caaagacggu gaagauggug auaaguacgg gggguuugac 3720
   uucugcacca ccgcuaagug aaugacguag aguguugaca uucauguuuu guaggccuuu 3780
   gcucuguguc auugucuccg ccaggagguu gaugaggggc acuaacccca ggaaaaaggu 3840
   aaaucccuug cuaaugcuaa gagguggagc cucaguuaug ggcucauguu cuuucuuguc 3900
   uucuuccgug ugguaguagc guuuccuggg ugauguaaaa ucaucaaaaa cgucccugug 3960
   gcuucuaagu auauagauga auaguccuac uagcguggca aagacggcaa agagguaaaa 4020
   agcagccuca cucgcugugg ucuuauagcu aaccugucuc ugugaguagu agaggacacc 4080
   uaagauguag augcacccca ccgauacuag ggcuacuggu aaaugaccug aguugaauuu 4140
   gucaacgaag uguagggcaa ugccuguugc uaauauggcu aagccgggua gugaacucau 4200
   guuuccauug uuuaugacga cuuuacuguc auugcuucuu cccccaaaaa uguauguuau 4260
   gaucuuccaa aucaggagcc acaggauagc agcugcuacc cuuucuauuu uuagguggac 4320
   uccuauccau auggggaguu guuuaaugau ggcuauguag ccuugguuga aacuagucac 4380
   guaaggguca gcccaggucc aauccaccuc caccgcgaau acugacauca uuagucugga 4440
   uguuggguug gcagacgcuu uaauguagcg aaguaugcua ccugugauag uggaggaucc 4500
   acggaguucg uaaaugcggg ugucgccuuu gaugaguggc ucuccauucu gcuugugcau 4560
   cauguaugag ugugaucugg ggucuacuau caugaacuua uuauuggcug uuuugcaauc 4620
   guuacaugag caagggugua cuccggucgu ccaaucuacu ucuauuccug aaucgucagu 4680
   gacguagugg gcuaaguaga uuguuauguu aucauuaucg uuggucaacc ucauuaugcc 4740
   uuucuuuuuu gauagggaga ugggugggau cugggggagu uggcaaaauc gggugcucuc 4800
   acugucgacu ccuaacauuu uuaauacuau ggcagccucc uuacggauga uaucugaguc 4860
   augcaugaug uuguguguug cggcuugguc uaaguacuga uaagugccug aucugacucu 4920
   uccaacucuu ccccuucucu guguagugga ugcuuuggag gcgccggaug uuucucuagu 4980
   gauuacaccg gccacacucu ccuuaaaaac uaucuguugc uggaggucaa aaacgucguc 5040
   uaaauuggcg uuuaggccga ccucggcuau auugguugca aagauggcua ugggcuuguc 5100
   cuuaucuguu uugauuuugc ugauuucacu ucuguaggua uaucuauuga ggaugauucu 5160
   uucuccuuca ccuacacugg uguagaguuc uucagcuuuc uugauggugg ggacgaaugu 5220
   aaugcuucuu cuauucuuac uaagugaguc uuuuauugca ucaacgauca uuuuuucauc 5280
   guuaaguuug gugucaauca cuggaaaauu aguuucaguu ucauaguggu cccaccaggu 5340
   ggccgucauc ggaaagaccu ggaugucauu gcuggugucu cugguucuuu cuucgagaaa 5400
   accuagcaau gcuauaguca uggggucuug caugugugcu ucguccacaa agacguucuu 5460
   guaguugugg auguugaggc uaugguggag gaggcgguuu acugcuguug aguggcacau 5520
   gauaagguuu cucuuguuuc uucuuucgac cuugccugug auaucaaucc cuacaucuaa 5580
   aucuuuuaau gcuucauaca uuucuccugc uaccguccug gugggugcua aaaguagaca 5640
   guuacguccu guuuccguag aucuaaccac ggcucuucuc agaacguguc uuguuuuucc 5700
   ugcuccacau cuuaacacua uuggauucca uccugggccu cuugugucga auuuccucug 5760
   aaauuuugaa agguccaugc cuguaguagg gguggguaaa aucauggcuu cauauguugg 5820
   auccuguagu gguaccgugu guccagcuaa ugagacgggu gucaguguuc cgucggcguc 5880
   ucuagcuagu acuggugagc cagagucgcc aaaugcuaug guccucguug uucccaguga 5940
   gacuuuaucg ucuucugaac ugauuuuuag uggucuaagu cuaugcuccc uguggagaua 6000
   gacgaagagg ucccugccuu cccguuuggc gaaagcccag gggccaccgu aggugaucca 6060
   auccaguuca ucacuauaau cguagggggu ggucacuuca ucuucgaauu cuaggggcag 6120
   uccuuuugua augugguaca uggugugaaa aauguuguuc uuguuguaac cauaaccaag 6180
   auguucaucu ccuaauggug uccuauugua uaccucauau augccuucgg ggccagagaa 6240
   aguaucuuuc uccacucucu cggguccggc aauguugaag gugucgaaca ggucggcuuu 6300
   ggcauucuua aaguaaccaa aggccaggau gagggcggcu auuccagccc uguagucuag 6360
   gaggccaaaa auggcaauua auaccaccac gaaaacccua guggaauccg ucacgacccu 6420
   guggucgugg aggcuacuca uaucauauug uaacuugccu uccuuaaggg uuaugguacc 6480
   gacuccuguc gguggaucau cgucuuuguc uauggguacu aacaguuggg augugaggug 6540
   ccguguucga acaguagcgc cccuccucau gaggauguag agggccgcca cugggaacag 6600
   gauguaggaa agguucuugg gugcuugugc uaaggccaga cagaucgcuc cuccugccac 6660
   caucugcaua augcucucuu uuuuguagga agaccaauac caucguguca uguuguaaau 6720
   cgguuuaggg aacaugaaga uaaguaugac uucgagugug augagcaagc ugaugauaaa 6780
   ccuagucgga cuaucaaugg cagucuguac ugcauaccaa ccaaauugac ugauugguuc 6840
   cauugauaau gguguuaggu gguggaguag ugcuguugcg aagaugguga ucugggucuu 6900
   agcuccaacc ggucuuacac gaaacccaug guuaaauaug aggguuguua gugucaaaaa 6960
   agucuuaagg agccauucaa aggcuucagu cgcgggcuca aaggcgaaug aagcuaagga 7020
   aaacccagaa caacaguggc uguggugguu aguucgcuug caccguuagu gagugguugu 7080
   ucuccaucug ucaguaguuc ucuaauuaag uuccuaaucu guuuggugcc uuucgcuucu 7140
   guuccuuucu uguagcuccu gagggcuuuu ucaauucggg uggggaguaa ugaaagcaac 7200
   uccaucucac ucuucaaugu ugcuggagaa uugauaaaau ugucauugcu ggcuguucuc 7260
   ucaccuccug aggcuuugac uagcucauug augagcuggu uaacggcuuu ggugccggug 7320
   gcccucugca ucuuguagaa accaguggcu gcaucaauug uggcuugguu guggccguuc 7380
   ucuugcaugg cuguggcuag gcuaguuuuu ccggauauga aaucuucauc auggucgugu 7440
   ucuucuuccu ugucuacggg gguaaaaccu ucaucuauuu ccucgucgcc aucguuggcu 7500
   uugauuacag ugguguugca uuucuucugu uugcaccagg uggaguuaaa ggucagggcu 7560
   cugucuuuau agagagaucu cuugaggagu cucucgcuag cuuccuugcg uaggaugaua 7620
   ccgccucuau aggcguucuc ggcugcugac auuucuuucu ucucaucuuc uucguucugu 7680
   uuuuugguga uuggccagca guugaaguug cauucugguc cuccaaaucu cuugggccag 7740
   auguguccuu uugugguggu guugacucuc ucgcagagag aggagcagcu gauuccgcag 7800
   cuccuccaac uucuccuuuu ugcuccaaau auuuggguga cagugccuuc gacuuugugu 7860
   gucaggcagu auuccuucuc uucaucagug uggaugguuc caaaccaggu aacuccgugg 7920
   gccgaacauu uauaauguuu cccugcccua cggcagucca cugugagguc uuugagguaa 7980
   gauucugguc uuucuucgcc uuuauaguag acugguggug uggagguggu gguuucuaac 8040
   ucuuuggugu cauacaucca uccaucgaca ucgucuaccu cauugacuuc caaaaagauu 8100
   ucucggcagu ucuugcagug uuuuucugaa uuauggcagg ugauuagugc uccuuguuuc 8160
   ccaucaucuu ugaguuuacg ggagguuuga ucccaagagg uggugucauu cacgucaugg 8220
   ucuguaccau caguggcgca uucuucuccu ucaucugugu agacuuugug gaguacggag 8280
   ccuaaggcuu uggcggcuuu cuggauuugu ucucuguuug gcauccuaag gauuguuaag 8340
   ccguccauga ggucuaaguc uauguuguag cugucuccua auugagggac auacagcuug 8400
   auugaugaau cgaacaugac guccuugaua cugccacauu ccauugacca uucaucugug 8460
   ucagccacgc aaccauacuu cucugcuucu accuccguua uuacuaaggg ggccaugagg 8520
   auaaguccua ugaugacugu gagauucuuc auguuaccug ucauuagggu uccgagccau 8580
   acuauggcuc cucccgucac ugccaucaug auuguugaug ucauuccgaa aaggcuuccu 8640
   aggaagccug cuccuuuggc aaagaaguuc uugaacaugu cuaguacugg ucucuuaacu 8700
   gcuccaucua ugguuauucu gucuuugacu uccagaacgu uaguggucca ucugcuaagc 8760
   cauccuguga uacccauuug ggggacauuc acuugaugcu uguuguugcc uaguuuaauu 8820
   ucaugcucuc cgggugggca gugcauuuuc aguacuucuu ugguugaagu gacuaugugu 8880
   gccugaucga aaagggugca guaguuggag guguauccag agccaacuug gcugcggcua 8940
   cgucccuuga cgacuaaccu gcacuggcua ggaacugcca uaucuaaguu aaaggugaca 9000
   acuccaucgu gaguggugag uauucugcug ccuucgauca guuugaaugc guaguugguu 9060
   gugucugcac aguccuucac acugguggug uguauuuuaa gggaauuaag caugaaacug 9120
   cagucugugg ggcuaauguc uaagggcauc uuggugccug gggaggcucu uccugauccu 9180
   uuggccaucu cggcuaugau uaucuucccg uucuugucca ggaaauucuu gaccauggau 9240
   ucgacguuau gggcuucuga aaaucucacg uaauggaggg uggugucucu ccaaguuaua 9300
   uaggucucgg caucauggac guugccaucu ucaaucuugc uaaggcuggu ugggauagac 9360
   gugguccaua cggaccaggg gaguuucauu gauucaaagg ccaaucuauc uacgagauau 9420
   gauucgccau cauuuggugc caucuuuauc agguacuugg uggcaaguuc ugcguguggu 9480
   agcuggcaug uuacucugac ucgugaggac gagucggcuu ugucugugau gucgaaacuu 9540
   ccuguguuug uguccauguu guaauaguuc ugauuuucug ugcguccaaa ggagaccguu 9600
   aagcgggcuc ccaucuuugu ugggucgauu guauagaccu guaucggaug uuugagggcu 9660
   aguucagcac aaguacauac cgguccuugu ccgaagagga agcagccagu gguccagcca 9720
   cgaucauggu auccucuagc gcagauggaa uaugguccug auuuugggca ggcggguucu 9780
   ccaucuccug ggcaucuguc ggaugaaacu gaguuggaac uaauguccaa ccaggauauu 9840
   uuguauccug ugagugcauu gucuauauag ggguagcuca uucuggccgu gauugugucc 9900
   augccuaccg guuggaucug auagcaaugc uuguagucca ugcggaggcu gacugaggug 9960
   ugugcuacuc cagucauggu ugacauuucu uccgggucag agcagagugu uuuuccgcuc 10020
   auuucagugg uugaagaggu cgaaucggcu accccgaggu aaaccuugga aaagaggaug 10080
   aucagcagca gguggacagu ggguacuuua aaauaggcca uaaccagggc cagaagggcu 10140
   augguuaugu cuguguuuga uagcacuuug gucucccaug uugauagggu cuucauccag 10200
   ccuucugaug gcugguuggu guguaucuca uacuucugaa uaguaucacg ugacagaucg 10260
   cgucuguuuc uagucuuccu ugaaaggucu ugauagaggg uugaauggau gugucucugc 10320
   cucagagaug uaguuccgaa acaugugucu gcugccguug guagcacucc ugcucugauc 10380
   agggcacagu cauguucauc guauuucaau ucuggugaug aguaguuggu guccggcugg 10440
   aagccggugg aacacucugu gagaucaaug ccauagguuu gguuguugcg uaugaaaagu 10500
   ucaggaucuu ugguguuguc caucuuugca aaaaguccuu ccucauucac caguaguaga 10560
   uccauagagg aagucuuguc cuugcggaca caugcuauca ccauugguga gaugucuguu 10620
   gggguggugg gugaaggugu uggugaugug gaggucccuc cagggaucac uuggguggca 10680
   aagaagauca ugaggagggu cagcaagccu gcugugccca uuccggaacu cuucuucuua 10740
   cgcuucguug ggccuuugau ggccugcugc acggaguuga gguugguuau caucuuucug 10800
   agucgccuca caccguugau auucuugggg auuuguggug guuuauucca gauucuccuc 10860
   agaaugacgg cugcucucuu gagcaggauc ucaaaggucu gcuguauuuu gccgcguccu 10920
   cucuggacgu gacgcagacc acggccggcu gauugccugg caugguggau ggcucucuug 10980
   gugcccccaa agccuugggu gacacggcgg gcaacccuga agggguuucc ggcgagacgc 11040
   augauuccuc uguaacucau ugucuugaga aguggaccca gccggguuau ucuucuaagu 11100
   auguuguuug aguguuuugu uuuuuguuug uuuguuuuau guguguguuu uuuguuuauu 11160
   auaauuuauu auauuauguu auauuguguu uguuuuauaa uaccauucaa uauugggugu 11220
   uguucaauuc ugaauuggug uaauuuaaca auauucugaa acguugaacc agugguuaaa 11280
   cuguguagcg accuaaaugu ugguagcgac uaaauuggcg ugucagaaug uaaauu 11336

<210> 21
<211> 2659
<212> DNA
<213> Unknown

<220>
<223> Flaviviridae

<400> 21
ggtccacttc tcaagacaat gagttacaga ggaatcatgc gtctcgccgg aacccccttc 60

agggttgccc gccgtgtcac ccaaggcttt gggggcacca agagagccat ccaccatgcc 120

aggcaatcag ccggccgtgg tctgcgtcac gtccagagag gacgcggcaa aatacagcag 180

acctttgaga tcctgctcaa gagagcagcc gtcattctga ggagaatctg gaataaacca 240

ccacaaatcc ccaagaatat caacggtgtg aggcgactca gaaagatgat aaccaacctc 300

aactccgtgc agcaggccat caaaggccca acgaagcgta agaagaagag ttccggaacg 360

ggcacagcag gcttgctgac cctcctcatg atcttctttg ccacccaagt gatccctgga 420

gggacctcca catcaccaac accttcaccc accaccccaa cagacatctc accaatggtg 480

atagcatgtg tccgcaagga caagacttcc tctatggatc tactgctggt gaatgaggaa 540

ggactttttg caaagatgga caacaccaaa gatcctgaac ttttcatacg caacaaccaa 600

acctatggca ttgatctcac agagtgttcc accggcttcc agccggacac caactactca 660

tcaccagaat tgaaatacga tgaacatgac tgtgccctga tcagagcagg agtgctacca 720

acggcagcag acacatgttt cggaactaca tctctgaggc agagacacat ccattcaacc 780

ctctatcaag acctttcaag gaagactaga aacagacgcg atctgtcacg tgatactatt 840

cagaagtatg agatacacac caaccagcca tcagaaggct ggatgaagac cctatcaaca 900

tgggagacca aagtgctatc aaacacagac ataaccatag cccttctggc cctggttatg 960

gcctatttta aagtacccac tgtccacctg ctgctgatca tcctcttttc caaggtttac 1020

ctcggggtag ccgattcgac ctcttcaacc actgaaatga gcggaaaaac actctgctct 1080

gacccggaag aaatgtcaac catgactgga gtagcacaca cctcagtcag cctacgcatg 1140

gactacaagc attgctatca gatccaaccg gtaggcatgg acacaatcac ggccagaatg 1200

agctacccct acatagacaa tgcactcaca ggatacaaaa tatcctggtt ggacattagt 1260

tccaactcag tttcatccga cagatgccca ggagatggag aacccgcctg cccaaaatca 1320

ggaccatatt ccatctgcgc tagaggatac catgatcgtg gctggaccac tggctgcttc 1380

ctcttcggac aaggaccggt atgtacttgt gctgaactaa ccctcaaaca tccgatacag 1440

gtctatacaa tcgacccaac aaagatggga gcccgcttaa cggtctcctt tggacgcaca 1500

gaaaatcaga actattacaa catggacaca aacacaggaa gtttcgacat cacagacaaa 1560

gccgactcgt cctcacgagt cagagtaaca tgccagctac cacacgcaga acttgccacc 1620

aagtacctga taaagatggc accaaatgat ggcgaatcat atctcgtaga tagattggcc 1680

tttgaatcaa tgaaactccc ctggtccgta tggaccacgt ctatcccaac cagccttagc 1740

aagattgaag atggcaacgt ccatgatgcc gagacctata taacttggag agacaccacc 1800

ctccattacg tgagattttc agaagcccat aacgtcgaat ccatggtcaa gaatttcctg 1860

gacaagaacg ggaagataat catagccgag atggccaaag gatcaggaag agcctcccca 1920

ggcaccaaga tgcccttaga cattagcccc acagactgca gtttcatgct taattccctt 1980

aaaatacaca ccaccagtgt gaaggactgt gcagacacaa ccaactacgc attcaaactg 2040

atcgaaggca gcagaatact caccactcac gatggagtcg tcacctttaa cttagatatg 2100

gcagttccta gccagtgcag gttagtcgtc aagggacgta gccgcagcca agttggctct 2160

ggatacacct ccaactactg cacccttttc gatcaggcac acatagtcac ttcaaccaaa 2220

gaagtactga aaatgcactg cccacccgga gagcatgaaa ttaaactagg caacaacaag 2280

tatcaagtga atgtccccca aatgggtatc acaggatggc ttagcagatg gaccactaac 2340

gttctggaag tcaaagacag aataaccata gatggagcag ttaagagacc agtactagac 2400

atgttcaaga acttctttgc caaaggagca ggcttcctag gaagcctttt cggaatgaca 2460

tcaacaatca tgatggcagt gacgggagga gccatagtat ggctcggaac cctaatgaca 2520

ggtaacatga agaatctcac agtcatcata ggacttatcc tcatggcccc cttagtaata 2580

acggaggtag aagcagagaa gtatggttgc gtggctgaca cagatgaatg gtcaatggaa 2640

tgtggcagta tcaaggacg 2659

<210> 22
<211> 2661
<212> DNA
<213> Unknown

<220>
<223> Flaviviridae

<400> 22
   ggtccacttc tcaagacaat gagttacaga ggaatcatgc gtctcgccgg aacccccttc 60
   agggttgccc gccgtgtcac ccaaggcttt gggggcacca agagagccat ccaccatgcc 120
   aggcaatcag ccggccgtgg tctgcgtcac gtccagagag gacgcggcaa aatacagcag 180
   acctttgaga tcctgctcaa gagagcagcc gtcattctga ggagaatctg gaataaacca 240
   ccacaaatcc ccaagaatat caacggtgtg aggcgactca gaaagatgat aaccaacctc 300
   aactccgtgc agcaggccat caaaggccca acgaagcgta agaagaagag ttccggaacg 360
   ggcacagcag gcttgctgac cctcctcatg atcttctttg ccacccaagt gatccctgga 420
   gggacctcca catcaccaac accttcaccc accaccccaa cagacatctc accaatggtg 480
   atagcatgtg tccgcaagga caagacttcc tctatggatc tactactggt gaatgaggaa 540
   ggactttttg caaagatgga caacaccaaa gatcctgaac ttttcatacg caacaaccaa 600
   acctatggca ttgatctcac agagtgttcc accggcttcc agccggacac caactactca 660
   tcaccagaat tgaaatacga tgaacatgac tgtgccctga tcagagcagg agtgctacca 720
   acggcagcag acacatgttt cggaactaca tctctgaggc agagacacat ccattcaacc 780
   ctctatcaag acctttcaag gaagactaga aacagacgcg atctgtcacg tgatactatt 840
   cagaagtatg agatacacac caaccagcca tcagaaggct ggatgaagac cctatcaaca 900
   tgggagacca aagtgctatc aaacacagac ataaccatag cccttctggc cctggttatg 960
   gcctatttta aagtacccac tgtccacctg ctgctgatca tcctcttttc caaggtttac 1020
   ctcggggtag ccgattcgac ctcttcaacc actgaaatga gcggaaaaac actctgctct 1080
   gacccggaag aaatgtcaac catgactgga gtagcacaca cctcagtcag cctccgcatg 1140
   gactacaagc attgctatca gatccaaccg gtaggcatgg acacaatcac ggccagaatg 1200
   agctacccct acatagacaa tgcactcaca ggatacaaaa tatcctggtt ggacattagt 1260
   tccaactcag tttcatccga cagatgccca ggagatggag aacccgcctg cccaaaatca 1320
   ggaccatatt ccatctgcgc tagaggatac catgatcgtg gctggaccac tggctgcttc 1380
   ctcttcggac aaggaccggt atgtacttgt gctgaactaa ccctcaaaca tccgatacag 1440
   gtctatacaa tcgacccaac aaagatggga gcccgcttaa cggtctcctt tggacgcaca 1500
   gaaaatcaga actattacaa catggacaca aacacaggaa gtttcgacat cacagacaaa 1560
   gccgactcgt cctcacgagt cagagtaaca tgccagctac cacacgcaga acttgccacc 1620
   aagtacctga taaagatggc accaaatgat ggcgaatcat atctcgtaga tagattggcc 1680
   tttgaatcaa tgaaactccc ctggtccgta tggaccacgt ctatcccaac cagccttagc 1740
   aagattgaag atggcaacgt ccatgatgcc gagacctata taacttggag agacaccacc 1800
   ctccattacg tgagattttc agaagcccat aacgtcgaat ccatggtcaa gaatttcctg 1860
   gacaagaacg ggaagataat catagccgag atggccaaag gatcaggaag agcctcccca 1920
   ggcaccaaga tgcccttaga cattagcccc acagactgca gtttcatgct taattccctt 1980
   aaaatacaca ccaccagtgt gaaggactgt gcagacacaa ccaactacgc attcaaactg 2040
   atcgaaggca gcagaatact caccactcac gatggagtcg tcacctttaa cttagatatg 2100
   gcagttccta gccagtgcag gttagtcgtc aagggacgta gccgcagcca agttggctct 2160
   ggatacacct ccaactactg cacccttttc gatcaggcac acatagtcac ttcaaccaaa 2220
   gaagtactga aaatgcactg cccacccgga gagcatgaaa ttaaactagg caacaacaag 2280
   tatcaagtga atgtccccca aatgggtatc acaggatggc ttagcagatg gaccactaac 2340
   gttctggaag tcaaagacag aataaccata gatggagcag ttaagagacc agtactagac 2400
   atgttcaaga acttctttgc caaaggagca ggcttcctag gaagcctttt cggaatgaca 2460
   tcaacaatca tgatggcagt gacgggagga gccatagtat ggctcggaac cctaatgaca 2520
   ggtaacatga agaatctcac agtcatcata ggacttatcc tcatggcccc cttagtaata 2580
   acggaggtag aagcagagaa gtatggttgc gtggctgaca cagatgaatg gtcaatggaa 2640
   tgtggcagta tcaaggacgt c 2661

<210> 23
<211> 2644
<212> DNA
<213> Unknown

<220>
<223> Flaviviridae

<400> 23
ctcaagacaa tgagttacag aggaatcatg cgtctcgccg gaaccccctt cagggttgcc 60

cgccgtgtca cccaaggctt tgggggcacc aagagagcca tccaccatgc caggcaatca 120

gccggccgtg gtctgcgtca cgtccagaga ggacgcggca aaatacagca gacctttgag 180

atcctgctca agagagcagc cgtcattctg aggagaatct ggaataaacc accacaaatc 240

cccaagaata tcaacggtgt gaggcgactc agaaagatga taaccaacct caactccgtg 300

cagcaggcca tcaaaggccc aacgaagcgt aagaagaaga gttccggaac gggcacagca 360

ggcttgctga ccctcctcat gatcttcttt gccacccaag tgatccctgg agggacctcc 420

acatcaccaa caccttcacc caccacccca acagacatct caccaatggt gatagcatgt 480

gtccgcaagg acaagacttc ctctatggat ctactactgg tgaatgagga aggacttttt 540

gcaaagatgg acaacaccaa agatcctgaa cttttcatac gcaacaacca aacctatggc 600

attgatctca cagagtgttc caccggcttc cagccggaca ccaactactc atcaccagaa 660

ttgaaatacg atgaacatga ctgtgccctg atcagagcag gagtgctacc aacggcagca 720

gacacatgtt tcggaactac atctctgagg cagagacaca tccattcaac cctctatcaa 780

gacctttcaa ggaagactag aaacagacgc gatctgtcac gtgatactat tcagaagtat 840

gagatacaca ccaaccagcc atcagaaggc tggatgaaga ccctatcaac atgggagacc 900

aaagtgctat caaacacaga cataaccata gcccttctgg ccctggttat ggcctatttt 960

aaagtaccca ctgtccacct gctgctgatc atcctctttt ccaaggttta cctcggggta 1020

gccgattcga cctcttcaac cactgaaatg agcggaaaaa cactctgctc tgacccggaa 1080

gaaatgtcaa ccatgactgg agtagcacac acctcagtca gcctccgcat ggactacaag 1140

cattgctatc agatccaacc ggtaggcatg gacacaatca cggccagaat gagctacccc 1200

tacatagaca atgcactcac aggatacaaa atatcctggt tggacattag ttccaactca 1260

gtttcatccg acagatgccc aggagatgga gaacccgcct gcccaaaatc aggaccatat 1320

tccatctgcg ctagaggata ccatgatcgt ggctggacca ctggctgctt cctcttcgga 1380

caaggaccgg tatgtacttg tgctgaacta accctcaaac atccgataca ggtctataca 1440

atcgacccaa caaagatggg agcccgctta acggtctcct ttggacgcac agaaaatcag 1500

aactattaca acatggacac aaacacagga agtttcgaca tcacagacaa agccgactcg 1560

tcctcacgag tcagagtaac atgccagcta ccacacgcag aacttgccac caagtacctg 1620

ataaagatgg caccaaatga tggcgaatca tatctcgtag atagattggc ctttgaatca 1680

atgaaactcc cctggtccgt atggaccacg tctatcccaa ccagccttag caagattgaa 1740

gatggcaacg tccatgatgc cgagacctat ataacttgga gagacaccac cctccattac 1800

gtgagatttt cagaagccca taacgtcgaa tccatggtca agaatttcct ggacaagaac 1860

gggaagataa tcatagccga gatggccaaa ggatcaggaa gagcctcccc aggcaccaag 1920

atgcccttag acattagccc cacagactgc agtttcatgc ttaattccct taaaatacac 1980

accaccagtg tgaaggactg tgcagacaca accaactacg cattcaaact gatcgaaggc 2040

agcagaatac tcaccactca cgatggagtc gtcaccttta acttagatat ggcagttcct 2100

agccagtgca ggttagtcgt caagggacgt agccgcagcc aagttggctc tggatacacc 2160

tccaactact gcaccctttt cgatcaggca cacatagtca cttcaaccaa agaagtactg 2220

aaaatgcact gcccacccgg agagcatgaa attaaactag gcaacaacaa gtatcaagtg 2280

aatgtccccc aaatgggtat cacaggatgg cttagcagat ggaccactaa cgttctggaa 2340

gtcaaagaca gaataaccat agatggagca gttaagagac cagtactaga catgttcaag 2400

aacttctttg ccaaaggagc aggcttccta ggaagccttt tcggaatgac atcaacaatc 2460

atgatggcag tgacgggagg agccatagta tggctcggaa ccctaatgac aggtaacatg 2520

aagaatctca cagtcatcat aggacttatc ctcatggccc ccttagtaat aacggaggta 2580

gaagcagaga agtatggttg cgtggctgac acagatgaat ggtcaatgga atgtggcagt 2640

atca 2644

<210> 24
<211> 2673
<212> DNA
<213> Unknown

<220>
<223> Flaviviridae

<400> 24
agaataaccc ggctgggtcc acttctcaag acaatgagtt acagaggaat catgcgtctc 60

gccggaaccc ccttcagggt tgcccgccgt gtcacccaag gctttggggg caccaagaga 120

gccatccacc atgccaggca atcagccggc cgtggtctgc gtcacgtcca gagaggacgc 180

ggcaaaatac agcagacctt tgagatcctg ctcaagagag cagccgtcat tctgaggaga 240

atctggaata aaccaccaca aatccccaag aatatcaacg gtgtgaggcg actcagaaag 300

atgataacca acctcaactc cgtgcagcag gccatcaaag gcccaacgaa gcgtaagaag 360

aagagttccg gaacgggcac agcaggcttg ctgaccctcc tcatgatctt ctttgccacc 420

caagtgatcc ctggagggac ctccacatca ccaacacctt cacccaccac cccaacagac 480

atctcaccaa tggtgatagc atgtgtccgc aaggacaaga cttcctctat ggatctacta 540

ctggtgaatg aggaaggact ttttgcaaag atggacaaca ccaaagatcc tgaacttttc 600

atacgcaaca accaaaccta tggcattgat ctcacagagt gttccaccgg cttccagccg 660

gacaccaact actcatcacc agaattgaaa tacgatgaac atgactgtgc cctgatcaga 720

gcaggagtgc taccaacggc agcagacaca tgtttcggaa ctacatctct gaggcagaga 780

cacatccatt caaccctcta tcaagacctt tcaaggaaga ctagaaacag acgcgatctg 840

tcacgtgata ctattcagaa gtatgagata cacaccaacc agccatcaga aggctggatg 900

aagaccctat caacatggga gaccaaagtg ctatcaaaca cagacataac catagccctt 960

ctggccctgg ttatggccta ttttaaagta cccactgtcc acctgctgct gatcatcctc 1020

ttttccaagg tttacctcgg ggtagccgat tcgacctctt caaccactga aatgagcgga 1080

aaaacactct gctctgaccc ggaagaaatg tcaaccatga ctggagtagc acacacctca 1140

gtcagcctcc gcatggacta caagcattgc tatcagatcc aaccggtagg catggacaca 1200

atcacggcca gaatgagcta cccctacata gacaatgcac tcacaggata caaaatatcc 1260

tggttggaca ttagttccaa ctcagtttca tccgacagat gcccaggaga tggagaaccc 1320

gcctgcccaa aatcaggacc atattccatc tgcgctagag gataccatga tcgtggctgg 1380

accactggct gcttcctctt cggacaagga ccggtatgta cttgtgctga actaaccctc 1440

aaacatccga tacaggtcta tacaatcgac ccaacaaaga tgggagcccg cctaacggtc 1500

tcctttggac gcacagaaaa tcagaactat tacaacatgg acacaaacac aggaagtttc 1560

gacatcacag acaaagccga ctcgtcctca cgagtcagag taacatgcca gctaccacac 1620

gcagaacttg ccaccaagta cctgataaag atggcaccaa atgatggcga atcatatctc 1680

gtagatagat tggcctttga atcaatgaaa ctcccctggt ccgtatggac cacgtctatc 1740

ccaaccagcc ttagcaagat tgaagatggc aacgtccatg atgccgagac ctatataact 1800

tggagagaca ccaccctcca ttacgtgaga ttttcagaag cccataacgt cgaatccatg 1860

gtcaagaatt tcctggacaa gaacgggaag ataatcatag ccgagatggc caaaggatca 1920

ggaagagcct ccccaggcac caagatgccc ttagacatta gccccacaga ctgcagtttc 1980

atgcttaatt ccccttaaat acacaccacc agtgtgaagg actgtgcaga cacaaccaac 2040

tacgcattca aactgatcga aggcagcaga atactcacca ctcacgatgg agtcgtcacc 2100

tttaacttag atatggcagt tcctagccag tgcaggttag tcgtcaaggg acgtagccgc 2160

agccaagttg gctctggata cacctccaac tactgcaccc ttttcgatca ggcacacata 2220

gtcacttcaa ccaaagaagt actgaaaatg cactgcccac ccggagagca tgaaattaaa 2280

ctaggcaaca acaagtatca agtgaatgtc ccccaaatgg gtatcacagg atggcttagc 2340

agatggacca ctaacgttct ggaagtcaaa gacagaataa ccatagatgg agcagttaag 2400

agaccagtac tagacatgtt caagaacttc tttgccaaag gagcaggctt cctaggaagc 2460

cttttcggaa tgacatcaac aatcatgatg gcagtgacgg gaggagccat agtatggctc 2520

ggaaccctaa tgacaggtaa catgaagaat ctcacagtca tcataggact tatcctcatg 2580

gcccccttag taataacgga ggtagaagca gagaagtatg gttgcgtggc tgacacagat 2640

gaatggtcaa tggaatgtgg cagtatcaag gac 2673

<210> 25
<211> 2645

<212>  DNA
<213>  Unknown

<220>
<223>  Flaviviridae

<400>  25
tcaagacaat gagttacaga ggaatcatgc gtctcgccgg aacccccttc agggttgccc      60

gccgtgtcac ccaaggcttt gggggcacca agagagccat ccaccatgcc aggcaatcag     120

ccggccgtgg tctgcgtcac gtccagagag gacgcggcaa aatacagcag acctttgaga     180

tcctgctcaa gagagcagcc gtcattctga ggagaatctg gaataaacca ccacaaatcc     240

ccaagaatat caacggtgtg aggcgactca gaaagatgat aaccaacctc aactccgtgc     300

agcaggccat caaaggccca acgaagcgta agaagaagag ttccggaatg ggcacagcag     360

gcttgctgac cctcctcatg atcttctttg ccacccaagt gatccctgga gggacctcca     420

catcaccaac accttcaccc accaccccaa cagacatctc accaatggtg atagcatgtg     480

tccgcaagga caagacttcc tctatggatc tactactggt gaatgaggaa ggactttttg     540

caaagatgga caacaccaaa gatcctgaac ttttcatacg caacaaccaa acctatggca     600

ttgatctcac agagtgttcc accggcttcc agccggacac caactactca tcaccagaat     660

tgaaatacga tgaacatgac tgtgccctga tcagagcagg agtgctacca acggcagcag     720

acacatgttt cggaactaca tctctgaggc agagacacat ccattcaacc ctctatcaag     780

acctttcaag gaagactaga aacagacgcg atctgtcacg tgatactatt cagaagtatg     840

agatacacac caaccagcca tcagaaggct ggatgaagac cctatcaaca tgggagacca     900

aagtgctatc aaacacagac ataaccatag cccttctggc cctggttatg gcctatttta     960

aagtacccac tgtccacctg ctgctgatca tcctcttttc caaggtttac ctcggggtag    1020

ccgattcgac ctcttcaacc actgaaatga gcggaaaaac actctgctct gacccggaag    1080

aaatgtcaac catgactgga gtagcacaca cctcagtcag cctccgcatg gactacaagc    1140

attgctatca gatccaaccg gtaggcatgg acacaatcac ggccagaatg agctacccct    1200

acatagacaa tgcactcaca ggatacaaaa tatcctggtt ggacattagt tccaactcag    1260

tttcatccga cagatgccca ggagatggag aacccgcctg cccaaaatca ggaccatatt    1320

ccatctgcgc tagaggatac catgatcgtg gctggaccac cggctgcttc ctcttcggac    1380

aaggaccggt atgtacttgt gctgaactaa ccctcaaaca tccgatacag gtctatacaa    1440

tcgacccaac aaagatggga gcccgcttaa cggtctcctt tggacgcaca gaaaatcaga    1500

actattacaa catggacaca aacacaggaa gtttcgacat cacagacaaa gccgactcgt    1560

cctcacgagt cagagtaaca tgccagctac cacacgcaga acttgccacc aagtacctga    1620

taaagatggc accaaatgat ggcgaatcat atctcgtaga tagattggcc tttgaatcaa    1680

tgaaactccc ctggtccgta tggaccacgt ctatcccaac cagccttagc aagattgaag 1740

atggcaacgt ccatgatgcc gagacctata taacttggag agacaccacc ctccattacg 1800

tgagattttc agaagcccat aacgtcgaat ccatggtcaa gaatttcctg gacaagaacg 1860

ggaagataat catagccgag atggccaaag gatcaggaag agcctcccca ggcaccaaga 1920

tgcccttaga cattagcccc acagactgca gtttcatgct taattccctt aaaatacaca 1980

ccaccagtgt gaaggactgt gcagatacaa ccaactacgc attcaaactg atcgaaggca 2040

gcagaatact caccactcac gatggagtcg tcacctttaa cttagatatg gcagttccta 2100

gccagtgcag gttagtcgtc aagggacgta gccgcagcca agttggctct ggatacacct 2160

ccaactactg cacccttttc gatcaggcac acatagtcac ttcaaccaaa gaagtactga 2220

aaatgcactg cccacccgga gagcatgaaa ttaaactagg caacaacaag tatcaagtga 2280

atgtccccca aatgggtatc acaggatggc ttagcagatg gaccactaac gttctggaag 2340

tcaaagacag aataaccata gatggagcag ttaagagacc agtactagac atgttcaaga 2400

acttctttgc caaaggagca ggcttcctag gaagcctttt cggaatgaca tcaacaatca 2460

tgatggcagt gacgggagga gccatagtat ggctcggaac cctaatgaca ggtaacatga 2520

agaatctcac agtcatcata ggacttatcc tcatggcccc cttagtaata acggaggtag 2580

aagcagagaa gtatggttgc gtggctgaca cagatgaatg gtcaatggaa tgtggcagta 2640

tcaag 2645

<210> 26
<211> 350
<212> DNA
<213> Unknown

<220>
<223> Flaviviridae

<400> 26
atgagttaca gaggaatcat gcgtctcgcc ggaaccccct tcagggttgc ccgccgtgtc 60

acccaaggct ttgggggcac caagagagcc atccaccatg ccaggcaatc agccggccgt 120

ggtctgcgtc acgtccagag aggacgcggc aaaatacagc agacctttga gatcctgctc 180

aagagagcag ccgtcattct gaggagaatc tggaataaac caccacaaat ccccaagaat 240

atcaacggtg tgaggcgact cagaaagatg ataaccaacc tcaactccgt gcagcaggcc 300

atcaaaggcc caacgaagcg taagaagaag agttccggaa cgggcacagc 350